# FASCICULE DE BREVET EUROPEEN

(11) **EP 2 504 338 B1**
(45) Date de publication et mention de la délivrance du brevet: **18.03.2015**
(21) Numéro de dépôt: 10799090.5
(22) Date de dépôt: 22.11.2010
(51) Int. Cl.: C07D 471/04, A61K 31/4375, A61P 35/00

(54) **DÉRIVÉS DE PYRIDINO-PYRIDINONES, LEUR PRÉPARATION ET LEUR APPLICATION EN THÉRAPEUTIQUE**
PYRIDIN-PYRIDINON-DERIVATE, IHRE HERSTELLUNG UND THERAPEUTISCHE VERWENDUNG
PYRIDINE-PYRIDINONE DERIVATIVES, PREPARATION AND THERAPEUTIC USE THEREOF

(30) Priorité: 23.11.2009 FR 0905602
(43) Date de publication de la demande: 03.10.2012
(73) Titulaire: SANOFI, 75008 Paris (FR)
(72) Inventeur: LASSALLE, Gilbert, F-75013 Paris (FR); MARTIN, Valérie, F-75013 Paris (FR); MCCORT, Gary, F-75013 Paris (FR); VOLLE-CHALLIER, Cécile, F-75013 Paris (FR)
(74) Mandataire: Catillon, Marie
(86) Numéro de dépôt international: PCT/FR2010/052480
(87) Numéro de publication internationale: WO 2011/061458

(56) Documents cités:
- WO-A2-2008/121687

## Description

La présente invention se rapporte à des dérivés de pyridino-pyridinones substitués (i) en position 3 par un imidazole, lui-même substitué par un groupement R1 et substitués (ii) en position 7 par un aryle ou hétéroaryle, lui-même substitué de manière optimum par un motif de type -[C(R3)(R4)]ₘ-CO-N(R5)(R6), à leur préparation et à leur application en thérapeutique en tant qu'inhibiteurs de l'activité kinase des récepteurs aux ligands PDGFs (abréviation de Platelet derived growth factors) et éventuellement des récepteurs au ligand FLT3 (abréviation de fms-like tyrosine kinase receptor).

Les récepteurs FLT3 et PDGF-R sont des membres de classe III de la famille des récepteurs à tyrosine kinases (RTK), dont font également partie le récepteur du Stem cell factor (c-kit) et du M-CSF (c-fms). Ils sont caractérisés pas un domaine extracellulaire composé de 5 domaines immunoglobuline-like contenant la région de liaison au ligand, un domaine transmembranaire, et une partie intracellulaire composée d'un domaine juxtamembranaire, d'un domaine kinase séparé en deux par un domaine insert (split domain) (Ullrich & Schlessinger, 1990).

La fixation des ligands sur les RTK induit la dimérisation des récepteurs, l'activation de leur partie tyrosine kinase qui conduit à la transphosphorylation des résidus tyrosine (Weiss & Schlessinger, 1998). Ces résidus phosphorylés servent ainsi de point d'ancrage aux protéines intracellulaires de la signalisation qui *in fine* provoquent différentes réponses cellulaires : la maintenance, la division, la prolifération, la différentiation, ou bien encore la migration cellulaire. (Claesson-Welsh, 1994).

Le gène codant pour FLT3 est situé sur le chromosome 13q12 (Rosnet et al, 1992) et code pour la protéine FLT3 (antigène CD135) exprimée spécifiquement par les cellules hématopoïétiques et plus particulièrement les cellules immatures comme les cellules souches hématopoïétiques et les progéniteurs multipotents myéloïdes et lymphoïdes et son expression disparaît au cours de la différentiation hématopoïétique. Son ligand, le FLT3 Ligand induit la dimérisation du récepteur, suivi de l'autophophosphorylation de la partie intracellulaire du récepteur qui conduit à l'activation de la cascade de signalisation. Les effets de l'activation du récepteur par son ligand sont la survie et l'expansion des progéniteurs multipotents.

Deux isoformes de récepteurs aux PDGFs ont été mis en évidence, la chaine PDGF-Ralpha et la chaine PDGF-Rbeta, qui suite à la fixation de leurs ligands s'homo- ou s'hétérodimérisent et induisent la signalisation intracellulaire. Les récepteurs aux PDGF sont essentiellement exprimés par les cellules d'origine mésenchymateuse et sont notamment retrouvés sur les fibroblastes, les cellules musculaires lisses, les péricytes et les cellules gliales (Ross et coll. 1986, Heldin, 1992).

Le « Platelet Derived Growth Factor », PDGF, protéine de poids moléculaire d'environ 30.000 daltons, est sécrété essentiellement par les plaquettes, accessoirement par l'endothélium, les muscles lisses vasculaires et les monocytes. Il est formé de deux chaînes polypeptidiques reliées entre elles par des ponts disulfures formant soit des homodimères, soit des hétérodimères. Quatre gènes (7p22, 22q13, 4q31 et 11q22) ont été décrits comme codant pour 4 différentes chaines polypeptiques (A, B C et D), qui une fois dimérisées donnent cinq ligands biologiquement actifs PDGF-AA, BB, CC, DD et AB (pour revue, Yu et al, 2003). Une spécificité de liaison existe, dont notamment le PDGF-AA pour l'isoforme alpha du récepteur, le PDGF-D pour la forme BB, et le PDGF-C pour la forme alpha et alpha/beta. Les PDGFs ligands sont de puissants mitogènes, mais sont également impliqués dans les phénomènes de migration, survie, apoptose et transformation cellulaire.

Les inhibiteurs de la fonction de PDGF-R alpha, beta et FLT3 interviennent dans différents domaines thérapeutiques. Parmi les phénomènes physiopathologiques où ces récepteurs peuvent être impliqués on retrouve, les cancers liquides ou leucémies, les cancers solides avec ou sans métastases ciblant les cellules tumorales et/ou les cellules de l'environnement tumoral (vasculaires, fibroblastiques), les fibroses et les maladies vasculaires :

### A. Cancer liquides

Les leucémies sont de différents types et touchent soit le compartiment myéloide soit le compartiment lymphoïde.

L'expression de FLT3 dans les cellules leucémiques issues de leucémies aigues myéloïdes (LAM) est de l'ordre de 100% des cas, et FLT3 contribue ainsi à stimuler la survie et la prolifération des cellules leucémiques (Carow et al, 1996 ; Drexler et al, 1996, Stacchini et al, 1996).

De plus, FLT3 est le siège de mutations activatrices dans 22 à 30% des LAM adultes et 11 % de l'enfant. Il s'agit le plus fréquemment de duplications en tandem (ITD) dans la région transmembranaire du récepteur (plus particulièrement les exons 14 et 15). Ces mutations conservent le cadre de lecture et leur taille peuvent varier entre 18 et 111 paires de bases. Plus rarement dans environ 7% des LAM, une mutation ponctuelle sur le résidu D835 situé dans le domaine kinase est retrouvée. Dans la majorité des cas, les formes FLT3 ITD ont un plus grand risque de rechute et sont des marqueurs de pronostic de survie faible. Ces deux types de mutations conduisent à une activité constitutive du domaine kinase indépendante de la stimulation par le ligand et ont été montrées comme transformant les cellules hématopoïétiques *in vitro* et *in vivo* (Mizuki et al, 2000; Tse et al ; 2000). De façon élégante, Kelly et coll. (2002), ont montré dans un modèle de reconstitution de moelle osseuse chez la souris que FLT3 ITD provoquait un syndrome myéloprolifératif.

L'intérêt d'utiliser des inhibiteurs de l'activité tyrosine kinase a été reporté à la fois *in vitro* et *in vivo* par plusieurs équipes, et récemment dans le modèle de reconstitution de moelle osseuse FLT3 ITD, un tel inhibiteur a été montré comme capable d'induire la régression de la tumeur et d'augmenter la survie des animaux (Ofarrel, 2003).

De plus, des données récentes mettent en évidence l'intérêt de combiner de tels inhibiteurs avec des cytotoxiques tel que la daunorubicine (Levis et al, 2004).

De façon intéressante, les cellules blastiques de type LAM peuvent également surexprimer d'autres récepteurs à activité kinase comme c-kit ou bien encore PDGF-R.

### Les syndromes myéloprolifératifs/dysplasiques

De façon assez fréquente, des anormalités cytogénétiques suite à des translocations chromosomiques ont été reportées dans les syndromes myéloprolifératifs. Ces réarrangements génèrent des protéines de fusion à activité tyrosine kinase dérégulées impliquées dans la prolifération des cellules myéloides blastiques.

### -protéines de fusion à activité kinase PDGF-R beta

Les protéines de fusion à activité kinase PDGF-R beta sont constituées de la partie intracellulaire de PDGF-R-beta et d'autre part d'un domaine N-ter d'une autre protéine (en général un facteur de transcription). Ont été reportés notamment dans les leucémies myélomonocytaire chronique (LMMC): Rab5/PDGF-Rbeta, H4-PDGF-Rbeta, HIP1-PDGF-RB ou bien encore Tel/PDGF-R beta. Cette dernière est la plus représentée. Elle est issue de la translocation t(5;12)(q31;p12) et code pour une protéine de fusion constituée de la partie N-terminale du facteur de transcription Tel et de la partie C-terminal de PDGF-Rbeta. Un domaine d'oligomérisation présent dans la partie Tel, conduit à une forme dimérisée de la protéine de fusion et à l'activité constitutive du domaine kinase. Cette protéine a été montrée *in vitro* comme capable de transformer des cellules hématopoïétiques à plusieurs reprises et notamment de façon détaillée dans l'article de M. Carrol et coll. (PNAS, 1996, 93, 14845-14850). *In vivo,* cette protéine de fusion conduit à un syndrome d'hyperprolifération des cellules myéloïdes (Ritchie et coll., 1999).

De plus, chez l'animal comme en clinique chez l'homme, il a été montré que des inhibiteurs de l'activité tyrosine kinase inhibent la prolifération des cellules blastiques et permettent d'enrayer le processus de leucémogénèse.

### -protéines de fusion à activité kinase PDGF-R alpha

Deux protéines de fusion impliquant PDGF-R alpha ont été reportées: bcr-PDGF-Ralpha présente dans une leucémie myéloide chronique (CML) atypique et FIP1L1-PDGF-Ralpha retrouvée dans une sous-population de leucémies, les LEC « leucémies à éosinophiles », provenant d'un syndrome d'hyper-éosinophilie (Griffin et coll. 2003). Cette protéine de fusion porte une activité constitutive du domaine kinase de PDGF-R alpha et est responsable de la prolifération anarchique de ces cellules.

Des inhibiteurs de l'activité kinase de PDGF-R alpha ont montré l'efficacité sur la prolifération de cellules FIP1L1-PDGF-R alpha positives et récemment un composé inhibiteur a reçu l'indication pour les HES/CEL.

Ainsi, inhiber l'activité kinase de PDGF-Ralpha et beta et l'activité FLT3wt et FLT3ITD comme le font les composés de l'invention, s'avèrent d'intérêt thérapeutique pour les LAM.

Outre les LAM et les syndromes myéloprolifératifs, d'autres leucémies peuvent être intéressantes à cibler avec de tels inhibiteurs dont les B-LAL et les T-LAL (leucémies aigues lymphoïdes-B ou lymphoïdes-T), où FLT3 est également exprimé. De plus, de par l'expression normale de FLT3 sur les cellules souches hématopoïétiques et la mise en évidence de son expression sur les cellules souches leucémiques, des inhibiteurs de l'activité kinase de FLT3 pourraient s'avérer d'intérêt dans toutes les leucémies (dont les CML) où le rôle des cellules souches leucémiques dans la récidive où la résistance est impliquée.

### B. Cancers solides

Des inhibiteurs de l'activité tyrosine kinase des récepteurs PDGF-R alpha et beta peuvent être d'intérêt pour les cancers solides soit en ciblant directement la cellule tumorale qui par autocrinie ou paracrinie est sensible à l'activité d'inhibiteur TK de PDGF-R, soit en ciblant les cellules de l'environnement en déstabilisant le réseau pour favoriser l'association avec d'autres agents thérapeutiques.

### -Exemples de cancers solides dont la cible est la cellule tumorale

### * Cancer mou : le sarcome dEwing

Le sarcome d'Ewing est une forme de cancer des os qui touche principalement les enfants et les jeunes adultes (la moyenne d'âge est de 13 ans). Il concerne 10% des tumeurs osseuses primaires et le risque de métastases est important. C'est une tumeur rare touchant 2 à 3 personnes par million d'habitants par an. Les cellules tumorales sont caractérisées par une translocation chromosomique t(11;22) codant pour la protéine de fusion EWS/FLI1.

Les cellules responsables sont celles du mésenchyme, qui expriment le récepteur PDGF-R-beta qui induit la motilité et la croissance des cellules de sarcome d'Ewing sous stimulation par le PDGF-BB (Üren et al, 2003). De plus, Zwerner and May (2001) ont montré l'expression de PDGF-C par les cellules de sarcome d'Ewing.

Ces mêmes cellules expriment également le récepteur RTK c-kit et il a été montré qu'un inhibiteur de l'activité kinase de PDGF-R et c-kit est capable d'inhiber la croissance tumorale de lignées de sarcome d'Ewing dans un modèle de xénogreffe chez la souris (Merchant et coll., 2002).

### * Tumeur du tissu conjonctif (GIST, dermatofibrosarcome)

### - les GISTs (tumeurs stromales gastro-intestinales)

Le groupe de Fletcher (2004) s'est intéressé aux 15 % des GIST chez qui KIT n'est ni muté ni surexprimé (KIT-wt). Ces auteurs ont observé une forte surexpression du récepteur PDGF-R alpha. Cette situation est retrouvée dans environ un tiers de ces GIST KIT-wt. Quant aux mutations de PDGFRA, les auteurs en observent (35 %) dans les cas où KIT est normal. Les PDGFRA mutées ont une activité tyrosine kinase élevée et constitutives et touchent l'acide aspartique en position 842. De la même façon que pour les Sarcomes d'Ewing, deux inhibiteurs de l'activité kinase de c-kit et PDGF-R ont montré leur efficacité *in vitro* et *in vivo* sur la proliferation des cellules PDGF-Ralpha muté (Le Tourneau et coll. 2007; Corless et coll., 2005).

### - les dermatofibrosarcomes (de Darier et Ferrand ou protuberans ou DFSP)

Le dermatofibrosarcome de Darier et Ferrand (ou DFSP) est une tumeur dermique à cellules fusiformes de malignité intermédiaire caractérisée par une évolution lente avec un risque majeur de récidive en cas d'exérèse insuffisante. Une anomalie génétique présente dans 95% des cas a été découverte en 1990, avec la mise en évidence notamment de la translocation des chromosomes 17 et 22 t(17-22)(q22; q13) qui aboutit à la fusion des gènes COL1A1 et PDGF B et la grande quantité de PDGFB surexprime son récepteur tyrosine kinase, le PDGFR. Inhiber l'activité kinase de PDGF-R est une thérapie prometteuse puisque *in vitro,* cela conduit à l'inhibition de la prolifération et l'apoptose des cellules tumorales et *in vivo* cela permet la réduction de la croissance tumorale dans des modèles de greffe de tumeurs chez la souris immunodéficientes (Sjöblom T et coll., 2001). De plus, des études cliniques ont montré l'efficacité (rémission complète ou totale) d'une telle molécule dans les DFSP (pour revue voir Mc Arthur, 2007).

### * Gliomes et Glioblastomes :

Le glioblastome est la tumeur de cerveau la plus répandue et la plus agressive avec une survie médiane autour de 1 an. PDGFs et ses récepteurs (alpha et beta) sont fréquemment exprimés dans les gliomes. La possibilité existe qu'une boucle autocrine/paracrine puisse contribuer à la pathogénicité de ces tumeurs. Le recepteur PDGF-R-alpha est exprimé préférentiellement dans les cellules de la tumeur, alors que le récepteur PDGF-beta est exprimé préférentiellement dans les cellules endothéliales vasculaires de la tumeur. Bloquer l'activité kinase du PDGF-R a montré son efficacité 1) *in vitro* par la diminution du nombre de colonies en soft agar et l'inhibition de la prolifération de lignées cellulaires 2) sur la réduction de la croissance tumorale dans des modèles de greffe chez la souris nude 3) en association avec l'irradiation dans des modèles de greffe intracraniale de cellules de lignées de glioblastomes (Oerbel et al, 2006; Geng et coll., 2005, Strawn et coll., 1994 Chin et al, 1997).

Ainsi, les composés de l'invention sont d'intérêt pour les sarcomes d'Ewing, les GIST, les dermatofibrosarcomes mais aussi les tumeurs desmoides, les hémangiomes et autres fibrosarcomes pour lesquelles des données d'expression du PDGF-R existent.

### C. Cibler PDGF-R dans l'environnement tumoral

### Angiogénèse

Les cellules de l'environnement de la tumeur font partie intégrante du développement du cancer que ce soit dans le cas d'une tumeur primaire ou secondaire (métastases). Parmi les cellules de l'environnement qui expriment PDGF-R et pour lesquelles le rôle de ce récepteur a été mis en évidence, on retrouve les cellules murales des vaisseaux c'est à dire les péricytes et les cellules musculaires lisses mais aussi les fibroblastes activés. L'angiogénèse est un processus de génération de nouveaux vaisseaux capillaires à partir de vaisseaux préexistants ou par mobilisation et différentiation de cellules de la moelle osseuse. Ainsi, à la fois une prolifération incontrôlée des cellules endothéliales et une mobilisation d'angioblastes à partir de la moelle osseuse sont observées dans les processus de néovascularisation des tumeurs. Il a été montré *in vitro* et *in vivo* que plusieurs facteurs de croissance stimulent la prolifération endothéliale comme le VEGF et les FGF. Outre ces mécanismes, il a été également mis en évidence que les cellules murales comme les péricytes et les cellules musculaires lisses participent à la stabilisation des vaisseaux néoformés. L'invalidation de PDGF-R beta cause un déficit des péricytes chez la souris et conduit à la mort des animaux en fin de gestation due à des micro-hémorragies et des oedèmes (Hellström et al, 1999, Hellström et al, 2001). Dans une élégante étude de transplantation, l'expression de PDGF-R-beta par les péricytes a été montrée nécessaire pour leur recrutement au niveau des vaisseaux tumoraux via la rétention de PDGF-B par les cellules endothéliales mais aussi par le PDGF-B secrété par les cellules tumorales (Abramsson et al, 2003). Dans le modèle transgénique Rip1Tag2 de tumeur pancréatique, Song et coll. ont également montré l'expression de PDGF-R beta sur les progéniteurs périvasculaires dans la moelle issus de la moelle osseuse, progéniteurs qui se différenciént en péricytes matures autour de la tumeur.

L'intérêt de bloquer l'activité de PDGF-R sur les péricytes tumoraux a été démontré par l'utilisation d'inhibiteur de l'activité tyrosine kinase de PDGF-R dans des modèles animaux (modèle transgénique de tumeur du pancréas et implantation de tumeur de glioma),et l'effet sur la croissance tumorale s'avère profond en association avec un inhibiteur de l'activité kinase de VEGF-R (Bergers et coll., 2003). Des données de la littérature (Cao et al, 2002, Fons et coll., 2004) ont mis en évidence l'intervention de PDGF-R alpha et du PDGF-C en angiogénèse et dans la différenciation des progéniteurs endothéliaux vers les cellules de type péricytes et cellules musculaires lisses.

### Fibroblastes activés

PDGF-R est abondant dans le stroma tumoral et est retrouvé sur les fibroblastes activés (myofibroblastes). Il a été montré dans deux études que l'association d'inhibiteurs ou antagonistes de PDGF-R avec des agents cytotoxiques conduit à une diminution de la microdensité des vaisseaux des cancers de l'ovaire (Apte et coll.2004) et des cancers du pancréas (Hwang et coll., 2003). PDGF-R beta régule la pression du tissu interstitiel de la tumeur (Heuchel et coll., 1999) et la co-administration des inhibiteurs de PDGF-R et des agents de chimiothérapie améliore leur délivrance dans les cellules tumorales en diminuant la pression intra-tumorale (Griffon-Etienne, 1999). Enfin dans un modèle murin, l'administration d'un inhibiteur de l'activité kinase de PDGF-R améliore la consommation d'agents de chimiothérapie par la tumeur et ainsi augmente leur efficacité (Griffon-Etienne, 1999; Pietras et coll., 2002 ; Pietras et coll., 2003). Ces effets sont certainement l'effet des TAF (tumour associated fibroblast), encore appelés CAF (carcinoma associated fibroblastes), fibroblastes activés présents autour de la tumeur qui expriment PDGF-R, comme l'illustrent les travaux récents de Hwang et coll. (2008), Kain et al (2008), Pietras et al (2008) dans des modèles vivo de cancer pancréatique et de cancérogénèse cervical. La stimulation par le PDGF ligand produit par les cellules tumorales stimule les fibroblastes qui produisent la matrice extracellulaire et ainsi augmentent la tension interstitielle. Aussi, diminuer cette tension peut faciliter la délivrance des drogues au sein de la tumeur et ainsi augmenter leur efficacité. Les fibroblastes activés présents dans le stroma tumoral représentent donc une nouvelle cible thérapeutique en oncologie (pour revue voir Bouzin & Feron, 2007).

### Métastases

Plusieurs travaux indiquent que le couple PDGF-R et PDGF-ligand est impliqué dans le développement des métastases, certainement par leur action sur l'angiogénèse et la metastatisation par la circulation sanguine, mais aussi par un effet direct sur la lymphangiogénèse et donc les métastases disséminées par les vaisseaux lymphatiques. Une revue documente notamment le rôle direct du PDGF-BB dans la lymphangiogénèse et les métastases lymphatiques (Cao et coll., 2005). Mais la majorité des travaux impliquent l'expression de PDGF-R dans l'environnement des métastases qui favorisent la prise et le développement des tumeurs secondaires. L'exemple le plus fréquemment rapporté est le développement des métastases osseuses.

### * Exemple du cancer de la prostate :

L'os est fréquemment le siège de métastases. 85 à 100% des patients qui meurent de cancer de prostate ont des métastases osseuses. La chimiothérapie améliore la survie sans progression et la survie globale cependant en raison de l'extrême hétérogénéité des métastases osseuses chez un même patient, la chimiothérapie n'est pas curatrice. Il a été montré en utilisant un modèle de souris immunodéficientes que PDGF-BB joue un rôle important dans le développement des métastases osseuses ostéoblastiques *in vivo* (Yu et coll., 2003). Le PDGF-DD, quant à lui, accélère la croissance des cellules tumorales prostatiques et augmente leur interaction avec les cellules du stroma. L'expression du récepteur PDGF alpha et beta a été mis en évidence respectivement dans 62 et 75% des cancers de prostate. De plus, une étude immunohistochimique a montré que la tumeur prostatique et ses métastases exprimaient PDGF-R (Hwang et coll., 2003). Kim et coll. (2003) ont montré que PDGF-R est exprimé sur les métastases osseuses et les cellules endothéliales vasculaires dépendantes des métastases. Un inhibiteur de tyrosine kinase de PDGF-R associé à un agent cytotoxique, réduit substantiellement les métastases osseuses du cancer de la prostate dans un modèle murin (Uehara et coll., 2003). De plus, cette même association conduit à l'apoptose des cellules tumorales, des cellules vasculaires endothéliales et l'inhibition de la croissance des cellules tumorales dans l'os. Le blocage de ces récepteurs et de leurs voies de signalisation dans l'os constitue une approche thérapeutique nouvelle (Hwang et coll. 2003 ; Uehara et coll., 2003). Chez l'homme, des essais cliniques ont montré le gain que présente l'association d'inhibiteur de PDGF-R et de cytotoxique chez des patients atteints de cancers de prostate hormonorésistants avec des métastases osseuses. Une diminution du marqueur (antigène prostatique spécifique) PSA > 50% a été en effet observée chez 38% des patients. La durée moyenne de la réponse du PSA était de 8 mois et la durée de la survie sans progression était de 11 mois.

Au vu des ces différents travaux, il apparaît que les composés de l'invention sont d'intérêt pour le traitement des cancers solides par leur effet sur les cellules de l'environnement et ce en association avec d'autres agents thérapeutiques comme des cytotoxiques ou des inhibiteurs de l'angiogénèse.

### D. Fibroses

Les fibroses sont souvent la cause d'un événement primaire comme un cancer, le traitement par la radiothérapie, les hépatites, l'alcoolémie. L'implication de PDGF est clairement démontrée dans la fibrose pulmonaire (incluant asbestose), rénale (glomérulonéphrite), médullaire (souvent associé aux leucémies à mégacaryocytes), induite par la radiothérapie ainsi que la fibrose hépatique et pancréatique (liée à l'alcoolémie ou à des hépatites) (pour revue voir JC Bonner, 2004). Une surexpression de PDGF a été notamment clairement montrée et des résultats dans des modèles *vivo* avec des inhibiteurs de l'activité TK de PDGF-R ont été également rapportés. Parmi ces études, celle de Einter et coll. (2002) a montré que le PDGF-CC est un puissant inducteur de fibrose rénale. Les auteurs ont testé l'efficacité d'un anticorps neutralisant dans un modèle de ligature urétrale unilatérale, où la fibrose se développe particulièrement rapidement. Ils ont observé un effet antifibrosant très marqué avec une réduction de l'accumulation de myofibroblastes, une réduction de l'accumulation de matrice extracellulaire et une réduction des dépôts de collagène IV. Une autre étude conduite dans un modèle de fibrose pulmonaire induite par la Bléomycine chez la souris a montré l'efficacité d'un inhibiteur de l'activité TK de PDGF-R sur la prévention de la fibrose par inhibition de la prolifération des cellules mésenchymales (Aono et coll., 2005). Dans un modèle de fibrose induite par l'amiante, un inhibiteur de PDGF-R TK a réduit la progression de la fibrose dans le parenchyme pulmonaire et le dépot de collagène (Vuorinen K, Gao F, Oury TD, Kinnula VL, Myllärniemi M. Imatinib mesylate inhibits fibrogenesis in asbestos-induced interstitial pneumonia. Exp Lung Res. 2007 Sep;33(7):357-73). Plusieurs équipes ont montré l'implication de PDGF-R dans la fibrose hépatique. Il est clairement montré que PDGFBB et DD possèdent des caractéristiques pro-fibrogéniques sur les cellules stellates hépatiques (Rovida et coll., 2008; Borkham-Kamphorst et coll., 2007). In vivo, un inhibiteur de PDGF-R TK est capable de réduire la fibrogénèse précoce dans un modèle de ligature du canal biliaire chez le rat (Neef et coll., 2006).

Aussi, au vue des données de la littérature, les composés de l'invention semblent d'intérêt thérapeutique pour les différents types de fibrose.

### E. Maladies vasculaires : athérosclérose & resténose, artériosclérose

La prolifération et la migration de cellules musculaires lisses vasculaires contribuent à l'hypertrophie intimale des artères et joue ainsi un rôle prépondérant dans l'athérosclérose et dans la resténose après angioplastie et endoarterectomie. Il a été clairement démontré *in vitro* et *in vivo* dans des modèles animaux, que PDGF est impliqué dans ces phénomènes. In vivo, il a été montré notamment une augmentation de l'expression de PDGF dans un modèle « Vein graft » chez le cochon. De plus, il a été également montré qu'un inhibiteur de l'activité TK de PDGF-R diminuait de façon conséquente la taille des lésions de l'artère thoracique et abdominale de souris diabetiques ApoE-KO (animaux traités à la streptozotocin), Une autre étude a montré que l'inhibition de la signalisation induite par PDGF (TK or PDGF A antisens) conduit à une diminution de la formation de la neointima formation dans les modèles « balloon injury » et de « coronary artery restenosis ». (Deguchi J, 1999, Ferns et coll. 1991, Sirois et al, 1997, Lindner et coll. 1995)

Ainsi, des inhibiteurs de l'activité tyrosine kinase de PDGF-R, tels que les composés de la présente invention représentent une thérapie de choix, soit seul, soit en association avec des composés antagonistes d'autres facteurs de croissance impliqués dans ces pathologies comme le FGF, dans le traitement des pathologies liées à la prolifération des cellules musculaires lisses vasculaires telles que l'athérosclérose, la resténose post-angioplastie ou suite à la pose de prothèses endovasculaires (stents) ou lors de pontages aorto-coronariens.

Les composés de l'invention de par leur activité inhibitrice de l'activité TK de PDGF-R semblent d'intérêt pour traiter ces maladies vasculaires.

### F. Autres

D'autres pathologies semblent pouvoir être des indications pour les composés de l'invention dont l'hypertension artérielle pulmonaire (HTAP) idiopathique. L'HTAP caractérisée par une élévation importante et continue de la pression dans l'artère pulmonaire, conduit à l'insuffisance ventriculaire droite et, souvent, à la mort du patient. Elle est associée à l'accroissement de la prolifération et à la migration des cellules musculaires lisses des vaisseaux pulmonaires. Schermuly et coll. (2005) ont montré que l'inhibition de l'activité tyrosine kinase des récepteurs aux PDGF améliore considérablement l'évolution de la maladie. Pour cela, ils ont utilisé entre autre un modèle d'hypertension artérielle pulmonaire expérimentale chez le rat obtenu par administration de monocrotaline pendant 28 jours. Tous les rats traités ont survécus, alors que 50% dentre eux sont morts dans le groupe témoin non traité.

Les composés de l'invention pourraient également présenter un intérêt thérapeutique dans les pathologies de l'oeil. D'une part, ils pourraient participer à la prévention de la fibrose en post-opératoire dans le cas de lésions cicatricielles de la cornée et de kératocone. Ceci s'expliquerait par leur action sur la prolifération des myofibroblastes comme raporté récemment par Kaur et coll. (2009). De plus, ils pourraient également favoriser la régression néovasculaire pour des pathologies telle que la DMLA (dégénérescence maculaire liée à l'âge). Ceci a en effet été montré par plusieurs équipes dans des modèles expérimentaux, dont notamment Jo et coll.; Dell et coll. en 2006.

WO2008/121687 a pour objet des dérivés d'imidazo[1,2-A]pyridine utiles dans le traitement et la prévention de maladies liées aux récepteurs de classe 3 et 5 de la famille des récepteurs de tyrosine kinases.

La présente invention se rapporte à des dérivés de pyridino-pyridinones substitués (i) en position 3 par un imidazole lui-même substitué par un groupement R1 et substitués (ii) en position 7 par un aryle ou hétéroaryle, lui-même substitué de manière optimum par un motif de type -[C(R3)(R4)]ₘ-CO-N(R5)(R6). La présente invention concerne également la préparation de tels composés et leur application en thérapeutique en tant qu'inhibiteurs de l'activité kinase des récepteurs aux ligands PDGFs (Platelet derived growth factors) et éventuellement des récepteurs au ligand FLT3 (fms-like tyrosine kinase receptor).

La présente invention a pour objet des composés répondant à la formule (I) : 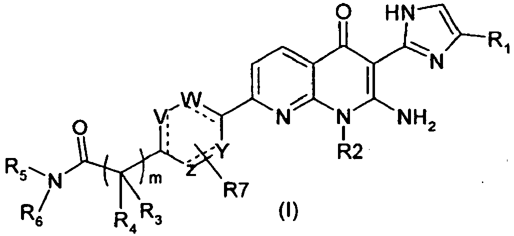 dans laquelle,
**R1** représente un atome d'hydrogène ou un groupe (C1-C4)alkyle ;
**R2** représente un groupement -(CH₂)_{n'}-B où :
   - n'=0,1,2,3 ou 4 ; et
   - B représente (i) un groupe (C3-C5)cycloalkyle ou un groupe (C1-C4)alkyle, ledit groupe étant éventuellement substitué par un ou plusieurs atomes de fluor, ou (ii) un groupe (C1-C4)alcoxy ;
**Y, Z, V** et **W** représentent, indépendamment les uns des autres :
   - un groupe -CH-,
   - un atome de carbone éventuellement substitué par un groupe R7, ledit groupe R7 représentant un groupe (C1-C4)alkyle ou un atome d'halogène,
   - un hétéroatome tel qu'un atome d'azote, un atome de soufre ou un atome d'oxygène, ou
   - pas d'atome,
   étant entendu que le cycle, dans lequel V, W, Y et Z sont compris, est un cycle comprenant 5 ou 6 chaînons, étant entendu que les pointillés dans ledit cycle indique que le cycle résultant est un cycle aromatique et étant entendu que ledit cycle comprend 0, 1 ou 2 hétéroatomes ;
**R3** et **R4** représentent, indépendamment l'un de l'autre, des groupes identiques ou différents, R3 et R4 étant choisis parmi :
   - un atome d'hydrogène ; et
   - un groupe (C1-C4)alkyle linéaire ;
ou **R3** et **R4** forment ensemble avec le carbone auxquels ils sont liés un groupe (C3-C5) cycloalkyle ;
**m** est un nombre entier égal à 1, 2 3 ou 4;
**R5** représente un atome d'hydrogène ou un groupe (C1-C4)alkyle ;
**R6** représente un groupement -(CH₂)ₙ-L dans lequel :
   - n= 0, 1, 2 ou 3, et
   - L est un groupe sélectionné parmi les groupes suivants :
      o un aryle comprenant 6 atomes de carbone ;
      o un hétéroaryle comprenant entre 5 et 6 chaînons et comprenant au moins un hétéroatome choisi parmi l'azote, l'oxygène et le soufre ;
      o un hétérocycle saturé comprenant 5, 6 ou 7 chaînons et comprenant au moins un hétéroatome choisi parmi l'azote et l'oxygène, ledit hétérocycle étant éventuellement un lactame ;
         ledit groupe aryle, hétéroaryle ou hétérocycle étant éventuellement substitué par au moins un substituant choisi parmi (i) les groupes (C1-C4)alkyle, linéaires ou ramifiés, (ii) les groupes (C3-C5)cycloalkyle, (iii) les atomes d'halogène, (iv) les aryles et (v) le benzyle ;
   étant entendu que, lorsque **L** est un hétéroaryle ou un hétérocycle, ledit hétéroaryle ou hétérocycle comprenant au moins un atome d'azote, ce dernier peut éventuellement être substitué par ledit substituant ;
ou **R5** et **R6** forment ensemble avec l'atome d'azote auxquels ils sont liés un groupe hétérocycle, éventuellement substitué par au moins
   - un hétéroaryle, ou
   - un groupe (C1-C3)alkyle, lui-même pouvant être substitué par un heterocycle comprenant 5 ou 6 atomes et comprenant au moins un hétéroatome choisi parmi l'azote et l'oxygène, étant entendu que lorsqu'il s'agit d'un hétérocycle comprenant au moins un atome d'azote, ce dernier peut éventuellement être substitué ;
ledit composé de formule (I), ses énantiomères et diastéréoisomères, y compris leurs mélanges, étant à l'état de base ou de sel d'addition à un acide, tel que par exemple l'acide trifluoroacétique (TFA) ou l'acide chlorhydrique et/ou à l'état de solvat.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention. Par exemple, quand **L** représente un hétérocycle, la configuration absolue d'un carbone substitué sur ledit hétérocycle peut être R ou S, ou lorsque **R3** est différent de **R4.**

Les composés de formule (I) peuvent exister à l'état de bases ou de sels d'addition à un ou des acides. De tels sels d'addition font partie de l'invention. Ces sels peuvent être préparés avec des acides pharmaceutiquement acceptables, mais les sels d'autres acides utiles, par exemple, pour la purification ou l'isolement des composés de formule (I) font également partie de l'invention.

Les composés de formule (I) peuvent également exister sous forme de solvats, à savoir sous forme d'associations ou de combinaisons avec une ou plusieurs molécules de solvant. De tels solvats font également partie de l'invention.

Dans le cadre de la présente invention, on entend par :
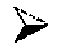 un groupe alkyle : un groupe aliphatique saturé comprenant de 1 à 7 atomes de carbone (avantageusement, un groupe aliphatique saturé comprenant de 1 à 4 atomes de carbone et abrégé (C1-C4)alkyle) et étant linéaire ou, quand la chaîne alkyle comprend au moins 3 atomes de carbone, pouvant être ramifié ou cyclique. A titre d'exemples, on peut citer les groupes méthyle, éthyle, propyle, isopropyle, butyle, iso-butyle, tertio-butyle, méthyl-cyclopropyle, pentyle, 2,2-diméthylpropyle, hexyle, heptyle, ainsi que les groupes cycloalkyles définis ci-dessous ;
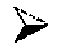 un groupe cycloalkyle : un groupe alkyle cyclique comprenant de 3 à 7 atomes de carbone (avantageusement de 3 à 5 atomes de carbone) et dont tous les atomes de carbones sont engagés dans le cycle. On peut citer les groupes cyclopropyle, cyclobutyle, cyclopentyle, cyclohexyle et cycloheptyle ;
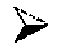 un groupe alcoxy : un groupe -O-alkyle, où le groupe alkyle est tel que défini ci-dessus ;
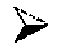 un atome d'halogène : un fluor, un chlore, un brome ou un iode ;
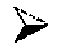 un groupe halogénoalkyle : un groupe comprenant un groupe alkyle tel que défini ci-dessus dont un ou plusieurs atomes d'hydrogène ont été substitués par un ou plusieurs atomes d'halogène tel que défini ci-dessus ; on parle ainsi de fluoroalkyle lorsque l'halogène en question est le fluor,
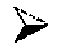 un hétéroatome : un atome d'azote, d'oxygène ou de soufre ;
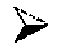 un groupe aryle : un groupe aromatique monocyclique comprenant 6 chaînons, par exemple un groupe phényle ;
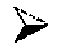 un groupe hétéroaryle : un groupe aromatique monocyclique comprenant entre 5 et 7 chaînons dont entre 1 et 3 hétéroatomes tels que précédemment définis. A titre d'exemples, on peut citer les groupes pyridine, pyrazine, pyrimidine, imidazole, pyrrole, pyrazole, thiophène, thiazole, isothiazole, thiadiazole, oxazole, isoxazole ;
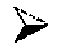 un groupe hétérocycle : un groupe alkyle cyclique comprenant entre 5 et 7 chaînons dont un ou plusieurs hétéroatomes tels que précédemment définis. A titre d'exemples, on peut citer les groupes pyrrolidine, morpholine, pipéridine, pipérazine, tetrahydrofuranne.

Les groupes mentionnés précédemment peuvent être substitués sachant en outre que dans le cas des groupes hétéroaryle ou hétérocycle comprenant au moins un atome d'azote, la substitution peut avoir lieu sur cet atome d'azote lorsqu'une telle substitution s'avère chimiquement possible.

Parmi les composés de formule (I) objets de l'invention, on peut citer les composés pour lesquels :
R5 représente un atome d'hydrogène ou un méthyle, ou R5 et R6 forment ensemble avec l'atome d'azote auxquels ils sont liés un groupe hétérocycle, éventuellement substitué par au moins
   - un hétéroaryle, avantageusement une pyridine ; ou
   - un groupe (C1-C3)alkyle, lui-même pouvant être substitué par un heterocycle comprenant 5 ou 6 atomes et comprenant au moins un hétéroatome choisi parmi l'azote et l'oxygène, avantageusement il s'agit d'un groupe C1alkyle, lui-même substitué par un hétérocycle comprenant 5 atomes dont un atome d'azote ;
   et/ou
**m** est égal à 0, 1 ou 3,
   et/ou
**R3 et R4** représentent, indépendamment l'un de l'autre, des groupes identiques ou différents, R3 et R4 étant choisis parmi :
   o Un atome d'hydrogène, et
   o Un méthyle,
   et/ou
**Y, Z, V et W** représentent, indépendamment les uns des autres :
   o un groupe -CH- ;
   o un atome de carbone substitué par un groupe R7, ledit groupe R7 représentant un groupe (C1-C4)alkyle ou un atome de fluor ; ou
   o un hétéroatome tel qu'un atome d'azote, un atome de soufre ou un atome d'oxygène, avantageusement un atome d'azote,
   et/ou
**R1** représente un atome d'hydrogène ou un méthyle,
   et/ou
**R2** représente un groupement -(CH₂)_{n'}-B où :
   o n'=0,1 ou 3 ; et/ou
   o B représente (i) un groupe (C3-C5)cycloalkyle, (ii) un groupe (C1-C4)alkyle ou (iii) un groupe (C1-C4)alcoxy,
   et/ou
les composés de formule (I) se présentant à l'état de base ou de sel d'addition à un acide tel que l'acide chlorhydrique ou l'acide trifluoroacétique.

Parmi les composés de formule (I) objets de l'invention, un premier sous-groupe de composés est constitué par les composés pour lesquels :
**R6** représente un groupement -(CH₂)ₙ-L dans lequel :
   - n= 0, 1, 2 ou 3, et
   - **L** est un groupe sélectionné parmi les groupes suivants :
      o un hétéroaryle comprenant 5 chaînons et comprenant (i) 2 hétéroatomes choisis, indépendamment l'un de l'autre, parmi l'azote, l'oxygène et le soufre, ou (ii) 3 hétéroatomes choisis, indépendamment l'un de l'autre, parmi l'azote et le soufre,
      o un hétéroaryle comprenant 6 chainons et comprenant 1 ou 2 hétéroatome(s),
      o un hétérocycle comprenant 5 chaînons et comprenant un hétéroatome choisi parmi l'azote et l'oxygène, ledit hétérocycle étant éventuellement un lactame, et
      o un hétérocycle comprenant 6 chaînons et comprenant 2 hétéroatomes choisis parmi l'azote et l'oxygène,
   ledit groupe hétéroaryle ou hétérocycle étant éventuellement substitué par au moins un substituant choisi parmi (i) les groupes (C1-C4)alkyle, linéaires ou ramifiés, (ii) les groupes (C3-C5)cycloalkyle, (iii) les atomes d'halogène, (iv) les aryles et (v) le benzyle,
   étant entendu que, lorsque L est un hétéroaryle ou un hétérocycle, ledit hétéroaryle ou hétérocycle comprenant au moins un atome d'azote, ce dernier peut éventuellement être substitué par ledit substituant.

Parmi les composés de formule (I) objets de l'invention, un deuxième sous-groupe de composés est constitué par les composés pour lesquels L est :
- un hétéroaryle comprenant 6 chaînons choisi parmi la pyridine, la pyrazine, la pyridazine, la pyrimidine, ou
- un aryle tel que le phényle, ou
- un hétéroaryle comprenant 5 chainons choisi parmi le thiazole, l'imidazole, le pyrazole, l'isoxazole et le 1,3,4 thiadiazole, ou
- un hétérocycle saturé comprenant 5 chaînons choisi parmi la pyrrolidine, le tetrahydrofurane et le 2-oxo-pyrrolidine, ou
- un hétérocycle saturé comprenant 6 chaînons choisi parmi la morpholine, la pipérazine, la pipéridine,
ledit groupe aryle, hétéroaryle ou hétérocycle étant éventuellement substitué par au moins un substituant choisi parmi (i) les groupes (C1-C4)alkyle, linéaires ou ramifiés, (ii) les groupes (C3-C5)cycloalkyle et (iii) les aryles,
étant entendu que, lorsque L est un hétéroaryle ou un hétérocycle, ledit hétéroaryle ou hétérocycle comprenant au moins un atome d'azote, ce dernier peut éventuellement être substitué par ledit substituant.

Parmi les composés de formule (I) objets de l'invention, un troisième sous-groupe de composés est constitué par les composés pour lesquels L est choisi parmi :
- la pyridine, éventuellement substitué par au moins un groupe (C1-C4)alkyle, linéaire ou ramifié,
- la morpholine, éventuellement substitué par au moins (i) un groupe (C3-C5)cycloalkyle ou (ii) un groupe (C1-C4)alkyle, linéaire ou ramifié,
- une pyrrolidine, éventuellement substitué par au moins (i) un groupe (C1-C4)alkyle, linéaire ou ramifié, ou (ii) un benzyle,
- un thiazole, éventuellement substitué par au moins (i) un groupe (C1-C4)alkyle, linéaire ou ramifié, ou (ii) un atome de chlore,
- un imidazole, éventuellement substitué par au moins un groupe (C1-C4)alkyle, linéaire ou ramifié,
- un gamma-lactame,
- un 1, 3, 4-thiadiazole, éventuellement substitué par au moins (i) un groupe (C1-C4)alkyle, linéaire ou ramifié, ou (ii) un groupe (C3-C5)cycloalkyle,
- un isoxazole, éventuellement substitué par au moins un groupe (C1-C4)alkyle, linéaire ou ramifié,
- un pyrazole, éventuellement substitué par au moins un groupe (C1-C4)alkyle, linéaire ou ramifié,
- une pyrazine,
- un isothiazole, éventuellement substitué par au moins un groupe (C1-C4)alkyle, linéaire ou ramifié,
- un phényle,
- un tetrahydrofuranne,
étant entendu que, lorsque L est un hétéroaryle ou un hétérocycle, ledit hétéroaryle ou hétérocycle comprenant au moins un atome d'azote, ce dernier peut éventuellement être substitué.

Parmi les composés de formule (I) objets de l'invention, on peut notamment citer les composés suivants :
2-{4-[7-Amino-8-ethyl-6-(1H-imidazol-2-yl)-5-oxo-5,8-dihydro-[1,8]naphthyridin-2-yl]-phenyl}-N-pyridin-2-yl-acetamide (composé 1)
2-{6-[7-Amino-8-ethyl-6-(1H-imidazol-2-yl)-5-oxo-5,8-dihydro-[1,8]naphthyridin-2-yl]-pyridin-3-yl}-N-pyridin-2-yl-acetamide (composé 2)
2-{4-[7-Amino-8-ethyl-6-(1H-imidazol-2-yl)-5-oxo-5,8-dihydro-[1,8]naphthyridin-2-yl]-phenyl}-N-(4-cyclopropyl-morpholin-3-ylmethyl)-acetamide (composé 3)
2-{4-[7-Amino-8-ethyl-6-(1H-imidazol-2-yl)-5-oxo-5,8-dihydro-[1,8]naphthyridin-2-yl]-phenyl}-N-(4-isopropyl-morpholin-3-ylmethyl)-acetamide (composé 4)
2-Amino-1-ethyl-3-(1H-imidazol-2-yl)-7-{4-[2-oxo-2-((S)-2-pyrrolidin-1-ylmethyl-pyrrolidin-1-yl)-ethyl]-phenyl}-1H-[1,8]naphthyridin-4-one (composé 5)
2-{4-(7-Amino-8-ethyl-6-(1H-imidazol-2-yl)-5-oxo-5,8-dihydro-[1,8]naphthyridin-2-yl]-phenyl}-N-(2-pyridin-4-yl-ethyl)-acetamide (composé 6)
2-{4-[7-Amino-8-ethyl-6-(1H-imidazol-2-yl)-5-oxo-5,8-dihydro-[1,8]naphthyridin-2-yl]-phenyl}-N-methyl-N-(2-pyridin-4-yl-ethyl)-acetamide (composé 7)
2-{4-[7-Amino-8-ethyl-6-(1H-imidazol-2-yl)-5-oxo-5,8-dihydro-[1,8]naphthyridin-2-yl]-phenyl}-N-(5-methyl-thiazol-2-yl)-acetamide (composé 8)
2-{4-[7-Amino-8-ethyl-6-(1H-imidazol-2-yl)-5-oxo-5,8-dihydro-[1,8]naphthyridin-2-yl]-3-methyl-phenyl}-N-(1-ethyl-pyrrolidin-2-ylmethyl)-acetamide (composé 9)
2-{4-[7-Amino-8-ethyl-6-(1H-imidazol-2-yl)-5-oxo-5,8-dihydro-[1,8]naphthyridin-2-yl]-phenyl}-N-(6-methyl-pyridin-3-yl)-acetamide (composé 10)
2-{4-[7-Amino-8-ethyl-6-(1H-imidazol-2-yl)-5-oxo-5,8-dihydro-[1,8]naphthyridin-2-yl]-phenyl}-N-(1,3-dimethyl-1H-pyrazol-4-ylmethyl)-acetamide (composé 11)
2-{4-[7-Amino-8-ethyl-6-(1H-imidazol-2-yl)-5-oxo-5,8-dihydro-[1,8]naphthyridin-2-yl]-phenyl}-N-thiazol-2-ylmethyl-acetamide (composé 12)
2-{4-[7-Amino-8-ethyl-6-(1H-imidazol-2-yl)-5-oxo-5,8-dihydro-[1,8]naphthyridin-2-yl]-phenyl}-N-(5-oxo-pyrrolidin-2-ylmethyl)-acetamide (composé 13)
2-{4-[7-Amino-8-ethyl-6-(1H-imidazol-2-yl)-5-oxo-5,8-dihydro-[1,8]naphthyridin-2-yl]-phenyl}-N-(5-methyl-[1,3,4]thiadiazol-2-yl)-acetamide (composé 14)
2-{4-[7-Amino-8-ethyl-6-(1H-imidazol-2-yl)-5-oxo-5,8-dihydro-[1,8]naphthyridin-2-yl]-phenyl}-N-[1,3,4]thiadiazol-2-yl-acetamide (composé 15)
2-{4-[7-Amino-8-ethyl-6-(1H-imidazol-2-yl)-5-oxo-5,8-dihydro-[1,8]naphthyridin-2-yl]-phenyl}-N-(3-methyl-isoxazol-5-yl)-acetamide (composé 16)
2-{4-[7-Amino-8-ethyl-6-(1H-imidazol-2-yl)-5-oxo-5,8-dihydro-[1,8]naphthyridin-2-yl]-phenyl}-N-[2-(1-methyl-pyrrolidin-2-yl)-ethyl]-acetamide (composé 17)
2-{4-[7-Amino-8-ethyl-6-(1H-imidazol-2-yl)-5-oxo-5,8-dihydro-[1,8]naphthyridin-2-yl]-phenyl}-N-(4-methyl-thiazol-2-yl)-acetamide (composé 18)
2-{4-[7-Amino-8-ethyl-6-(1H-imidazol-2-yl)-5-oxo-5,8-dihydro-[1,8]naphthyridin-2-yl]-phenyl}-N-pyridin-4-ylmethyl-acetamide (composé 19)
2-{4-[7-Amino-8-ethyl-6-(1H-imidazol-2-yl)-5-oxo-5,8-dihydro-[1,8]naphthyridin-2-yl]-phenyl}-N-(5-cyclopropyl-[1,3,4]thiadiazol-2-yl)-acetamide (composé 20)
2-{4-[7-Amino-8-ethyl-6-(1H-imidazol-2-yl)-5-oxo-5,8-dihydro-[1,8]naphthyridin-2-yl]-phenyl}-N-(2-pyridin-3-yl-ethyl)-acetamide (composé 21)
2-{4-[7-Amino-8-ethyl-6-(1H-imidazol-2-yl)-5-oxo-5,8-dihydro-[1,8]naphthyridin-2-yl]-phenyl}-N-(2,5-dimethyl-2H-pyrazol-3-ylmethyl)-acetamide (composé 22)
2-{4-[7-Amino-8-ethyl-6-(1H-imidazol-2-yl)-5-oxo-5,8-dihydro-[1,8]naphthyridin-2-yl]-phenyl}-N-(1-methyl-1H-imidazol-4-ylmethyl)-acetamide (composé 23)
2-{4-[7-Amino-8-ethyl-6-(1H-imidazol-2-yl)-5-oxo-5,8-dihydro-[1,8]naphthyridin-2-yl]-phenyl}-N-pyrazin-2-yl-acetamide (composé 24)
2-{4-[7-Amino-8-ethyl-6-(1H-imidazol-2-yl)-5-oxo-5,8-dihydro-[1,8]naphthyridin-2-yl]-phenyl}-N-(2-pyridin-2-yl-ethyl)-acetamide (composé 25)
2-{4-[7-Amino-8-ethyl-6-(1H-imidazol-2-yl)-5-oxo-5,8-dihydro-[1,8]naphthyridin-2-yl]-phenyl}-N-(5-chloro-thiazol-2-yl)-acetamide (composé 26)
2-{4-[7-Amino-8-ethyl-6-(1H-imidazol-2-yl)-5-oxo-5,8-dihydro-[1,8]naphthyridin-2-yl]-phenyl}-N-(3,4-dimethyl-isoxazol-5-yl)-acetamide (composé 27)
2-{4-[7-Amino-8-ethyl-6-(1H-imidazol-2-yl)-5-oxo-5,8-dihydro-[1,8]naphthyridin-2-yl]-phenyl}-N-(2-methyl-pyridin-4-yl)-acetamide (composé 28)
2-{4-[7-Amino-8-ethyl-6-(1H-imidazol-2-yl)-5-oxo-5,8-dihydro-[1,8]naphthyridin-2-yl]-phenyl}-N-pyrazin-2-ylmethyl-acetamide (composé 29)
2-{4-[7-Amino-8-ethyl-6-(1H-imidazol-2-yl)-5-oxo-5,8-dihydro-[1,8]naphthyridin-2-yl]-phenyl}-N-(5-ethyl-[1,3,4]thiadiazol-2-yl)-acetamide (composé 30)
2-{4-[7-Amino-8-ethyl-6-(1H-imidazol-2-yl)-5-oxo-5,8-dihydro-[1,8]naphthyridin-2-yl]-phenyl}-N-(tetrahydro-furan-2-ylmethyl)-acetamide (composé 31)
2-{4-[7-Amino-8-ethyl-6-(1H-imidazol-2-yl)-5-oxo-5,8-dihydro-[1,8]naphthyridin-2-yl]-phenyl}-N-(3-methyl-pyridin-2-yl)-acetamide (composé 32)
2-{4-[7-Amino-8-ethyl-6-(1H-imidazol-2-yl)-5-oxo-5,8-dihydro-[1,8]naphthyridin-2-yl]-phenyl}-N-(4-methyl-pyridin-2-yl)-acetamide (composé 33)
2-{4-[7-Amino-8-ethyl-6-(1H-imidazol-2-yl)-5-oxo-5,8-dihydro-[1,8]naphthyridin-2-yl]-phenyl}-N-(4,6-dimethyl-pyridin-2-yl)-acetamide (composé 34)
2-{4-[7-Amino-8-ethyl-6-(1H-imidazol-2-yl)-5-oxo-5,8-dihydro-[1,8]naphthyridin-2-yl]-phenyl}-N-(6-methyl-pyridin-2-yl)-acetamide (composé 35)
2-{4-[7-Amino-8-ethyl-6-(1H-imidazol-2-yl)-5-oxo-5,8-dihydro-[1,8]naphthyridin-2-yl]-phenyl}-N-pyridin-3-ylmethyl-acetamide (composé 36)
2-{4-[7-Amino-8-ethyl-6-(1H-imidazol-2-yl)-5-oxo-5,8-dihydro-[1,8]naphthyridin-2-yl]-phenyl}-N-(2-ethyl-2H-pyrazol-3-yl)-acetamide (composé 37)
2-{4-[7-Amino-8-ethyl-6-(1H-imidazol-2-yl)-5-oxo-5,8-dihydro-[1,8]naphthyridin-2-yl]-phenyl}-N-(3-methyl-isothiazol-5-yl)-acetamide (composé 38)
2-Amino-1-ethyl-3-(1H-imidazol-2-yl)-7-{4-[2-oxo-2-(2-pyridin-3-yl-pyrrolidin-1-yl)-ethyl]-phenyl}-1 H-[1,8]naphthyridin-4-one (composé 39)
2-{4-[7-Amino-8-ethyl-6-(1H-imidazol-2-yl)-5-oxo-5,8-dihydro-[1,8]naphthyridin-2-yl]-phenyl}-N-(1-benzyl-pyrrolidin-3-yl)-acetamide (composé 40)
2-{4-[7-Amino-8-ethyl-6-(1H-imidazol-2-yl)-5-oxo-5,8-dihydro-[1,8]naphthyridin-2-yl]-phenyl}-N-[(S)-1-(tetrahydro-furan-2-yl)methyl]-acetamide (composé 41)
2-{4-[7-Amino-8-ethyl-6-(1H-imidazol-2-yl)-5-oxo-5,8-dihydro-[1,8]naphthyridin-2-yi]-phenyl}-N-(4-methyl-pyridin-3-yl)-acetamide (composé 42)
2-{4-[7-Amino-8-ethyl-6-(1H-imidazol-2-yl)-5-oxo-5,8-dihydro-[1,8]naphthyridin-2-yl]-phenyl}-N-(4-ethyl-pyridin-2-yl)-acetamide (composé 43)
2-{4-[7-Amino-8-ethyl-6-(1H-imidazol-2-yl)-5-oxo-5,8-dihydro-[1,8]naphthyridin-2-yl]-phenyl}-N-ethyl-N-pyridin-4-ylmethyl-acetamide (composé 44)
2-{4-[7-Amino-8-ethyl-6-(1H-imidazol-2-yl)-5-oxo-5,8-dihydro-[1,8]naphthyridin-2-yl]-phenyl}-N-(6-ethyl-pyridin-2-yl)-acetamide (composé 45)
2-{4-[7-Amino-8-ethyl-6-(1H-imidazol-2-yl)-5-oxo-5,8-dihydro-[1,8]naphthyridin-2-yl]-phenyl}-N-benzyl-acetamide (composé 46)
2-{4-[7-Amino-8-ethyl-6-(1H-imidazol-2-yl)-5-oxo-5,8-dihydro-[1,8]naphthyridin-2-yl]-phenyl}-N-pyridin-4-ylmethyl-propionamide (composé 47)
4-[7-Amino-8-ethyl-6-(1H-imidazol-2-yl)-5-oxo-5,8-dihydro-[1,8]naphthyridin-2-yl]-N-pyridin-4-ylmethyl-benzamide (composé 48)
4-{4-[7-Amino-8-ethyl-6-(1H-imidazol-2-yl)-5-oxo-5,8-dihydro-[1,8]naphthyridin-2-yl]-phenyl}-N-pyridin-4-ylmethyl-butyramide (composé 49)
2-{4-[7-Amino-8-ethyl-6-(4-methyl-1H-imidazol-2-yl)-5-oxo-5,8-dihydro-[1,8]naphthyridin-2-yl]-phenyl}-N-pyridin-4-ylmethyl-acetamide (composé 50)
2-{4-[7-Amino-8-cyclopropylmethyl-6-(1H-imidazol-2-yl)-5-oxo-5,8-dihydro-[1,8]naphthyridin-2-yl]-phenyl}-N-pyridin-4-ylmethyl-acetamide (composé 51)
2-{4-[7-Amino-8-cyclopentyl-6-(1H-imidazol-2-yl)-5-oxo-5,8-dihydro-[1,8]naphthyridin-2-yl]-phenyl}-N-pyridin-4-ylmethyl-acetamide (composé 52)
2-{5-[7-Amino-8-ethyl-6-(1H-imidazol-2-yl)-5-oxo-5,8-dihydro-[1,8]naphthyridin-2-yl]-pyridin-2-yl}-N-pyridin-4-ylmethyl-acetamide (composé 53)
2-{4-[7-Amino-6-(1H-imidazol-2-yl)-8-(3-methoxy-propyl)-5-oxo-5,8-dihydro-[1,8]naphthyridin-2-yl]-phenyl}-N-pyridin-4-ylmethyl-acetamide (composé 54)
2-{4-[7-Amino-8-ethyl-6-(1H-imidazol-2-yl)-5-oxo-5,8-dihydro-[1,8]naphthyridin-2-yl]-phenyl}-N-(3-phenyl-propyl)-acetamide (composé 55)
2-{4-[7-Amino-8-ethyl-6-(1H-imidazol-2-yl)-5-oxo-5,8-dihydro-[1,8]naphthyridin-2-yl]-2-fluoro-phenyl}-N-(1-ethyl-pyrrolidin-2-ylmethyl)-acetamide (composé 56)

Il est à noter que les composés ci-dessus ont été nommés en nomenclature IUPAC par l'intermédiaire du logiciel Autonom.

Dans ce qui suit, on entend par groupe protecteur, noté Pg, un groupe qui permet, d'une part, de protéger une fonction réactive telle qu'un hydroxy ou une amine pendant une synthèse et, d'autre part, de régénérer la fonction réactive intacte en fin de synthèse. Des exemples de groupes protecteurs ainsi que des méthodes de protection et de déprotection sont données, par exemple, dans « Protective Groups in Organic Synthesis », Green et al., 2nd Edition (John Wiley & Sons, Inc., New York), 1991.

On entend par groupe partant, dans ce qui suit, un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxy activé tel qu'un méthanesulfonate, benzènesulfonate, p-toluènesulfonate, triflate, acétate, etc. Des exemples de groupes partants ainsi que des références pour leur préparation sont donnés dans « Advances in Organic Chemistry », J. March, 3rd Edition, Wiley Interscience, 1985, p. 310-316.

Conformément à l'invention, on peut préparer les composés de formule générale (I) selon le procédé décrit dans les schémas 1, 2 et 3 suivants.

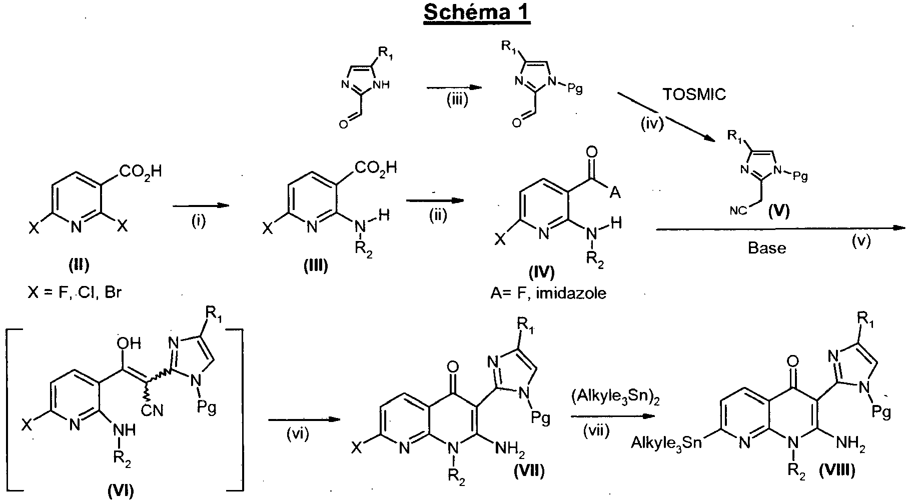

Selon le schéma 1, dans l'étape (i), un acide 2,6-dihalogéno-nicotinique de formule (II) est mono-substitué en position 2 par une amine de formule R₂-NH₂ (où R₂ est tel que défini précédemment en rapport avec les composés de formule (I)), à température ambiante, ou à une température de 50°C à 100°C, en chauffage classique ou par micro-onde et dans un solvant protique tel qu'un alcool par exemple l'éthanol, le n-butanol, *tert*-butanol ou l'eau. L'acide (III), issue de l'étape (i), est ensuite activé en dérivé de formule (IV), suivant l'étape (ii) soit sous forme de fluorure d'acide par l'action du fluorure de cyanuryle à température ambiante, en présence d'une base telle que la triéthylamine ou la pyridine et dans un solvant aprotique tel que le dichlorométhane ou le THF, comme décrit par G. OLAH et coll. dans Synthesis (1973), 487, ou sous forme d'imidazolide par l'action du carbodiimidazole dans un solvant polaire aprotique tel que le DMF ou le THF ou par d'autres méthodes connues de l'Homme de l'art, telles que celles décrites par MUKAIYAMA et TANAKA dans Chem. Lett. (1976), 303 ou par ISHIKAWA et SASAKI dans Chem. Lett. (1976), 1407.

Les cyanoacétylimidazoles de formule (V), sont préparés en deux étapes à partir d'un imidazole-2-carboxaldehyde substitués ou non en position (4,5) de l'imidazole. A l'étape (iii) l'azote libre de l'imidazole-2-carboxaldehyde est protégé par un groupement protecteur, noté Pg dans le schéma 1, tel que par exemple un groupement SEM, Boc ou trityle, dans des conditions opératoires classiques connu de l'homme de l'art « Protective Groups in Organic Synthesis », Green et al., 2nd Edition (John Wiley & Sons, Inc., New York). S'il y a lieu les deux isomères τ et π de l'imidazole protégé sont obtenus et utilisés indifféremment dans les réactions suivantes. L'imidazole-2-carboxaldehyde protégé, est ensuite transformé dans l'étape (iv) en cyanoacétylimidazole de formule (V) par réaction de la fonction aldéhyde avec l'anion du TOSMIC, formé par addition de *tert*-butylate de potassium, dans le DME anhydre à basse température (-50°C), suivi par l'ouverture du cycle de l'intermédiaire anionique, 4-tosyl-2-oxazoline formé, puis le milieu réactionnel est ensuite chauffé au reflux en présence de méthanol pour permettre la formation de la fonction acétylnitrile suivant la méthode décrite par Van Leusen A. et coll (Synthetic Comm, 10(5) 1980, 399-403).

Le fluorure d'acide ou l'imidazolide de formule (IV) obtenu à l'issue de l'étape (ii), très réactifs mais stables, est mis ensuite en réaction, à l'étape (v), avec un cyanoacétylimidazole de formule (V), substitué ou non en position (4,5), en présence d'un équivalent d'une base telle que l'hydrure de sodium ou le tert-butoxyde de potassium, dans un solvant aprotique polaire tel que THF ou DMF, à une température de -5°C à température ambiante, puis un deuxième équivalent de la base utilisée est additionné et le composé de formule (VI) formé, se cyclise in situ, à température ambiante, pour fournir le composé pyridino-pyridinone de formule (VII), suivant l'étape (vi).

L"intermédiaire halogéné de formule (VII) peut ensuite être transformé en dérivé de pyridino[2,3-b]pyridinone-7-stanneux de formule (VIII), en le faisant réagir, dans l'étape (vii) avec un composé hexaalkyldistanneux, où l'alkyle peut être soit un butyle ou un méthyle, en présence d'un complexe de palladium (à l'état d'oxydation (0) ou (II)) tel que par exemple Pd(PPh₃)₄, PdCl₂(PPh₃)₂, et éventuellement avec l'ajout d'un ligand tel que la triphényle arsine, le trifurylphosphine dans un solvant polaire ou non tel que le dioxane, le THF, le DMF ou un solvant apolaire tel que le toluène à une température comprise entre 50 et 110°C selon la methodologie décrite par Stille JK et coll. (JACS, 1987, 109, 813*).*

Pour l'obtention des composés de formule (I) objet de la présente invention deux méthodes sont utilisables à partir de l'intermédiaire halogéné de formule (VII), selon la methode 1 décrite dans le schéma 2, où à partir du composé stanneux de formule (VIII), selon la methode 2 décrite dans le schéma 3.

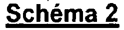

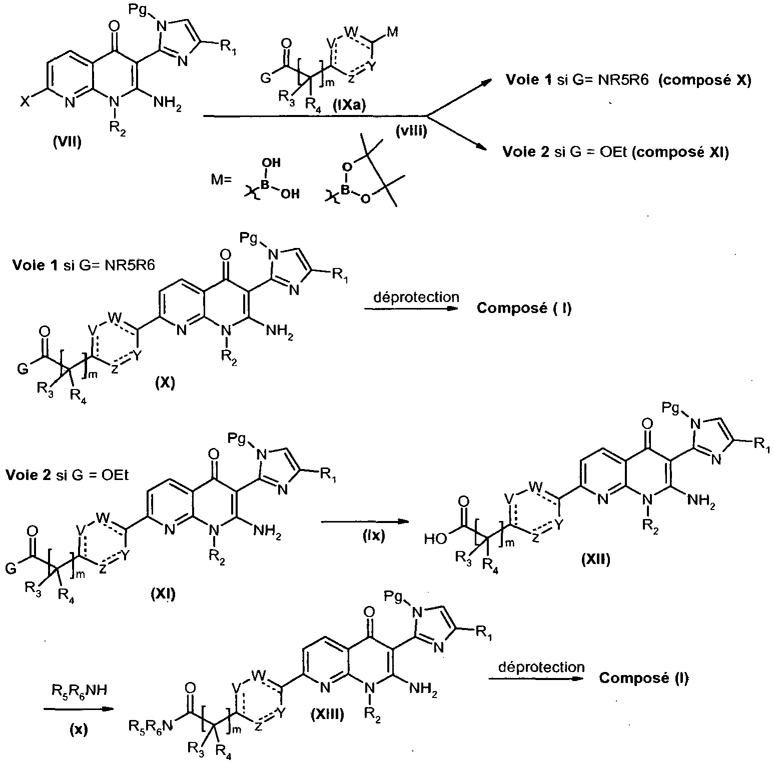

Suivant la méthode 1 décrite dans le **schéma 2,** l'intermédiaire (VII) est engagé à l'étape (viii) dans une réaction de couplage de Suzuki avec un acide boronique [formule (IXa) avec M= -B(OH)₂] ou un ester boronique de pinacol [formule (IXa) avec Pg étant un groupement protecteur tel que défini précédemment, M=B(OC(CH₃)₂)₂] où m, R1, R2, R3, R4, V, W, Y et Z sont tels que défini précédemment en rapport avec les composés de formule (I) objets de l'invention étant entendu que le cycle doit comprendre entre 5 et 6 chaînons, et G est soit un groupement (C1-C4)alcoxy tel que OEt ou un motif -NR5R6, où R5 et R6 sont tels que définis pour le composé de formule (I). Ces composés (IXa) sont soit commerciaux soit préparés à partir des dérivés halogénés correspondants suivant la méthode de Miyaura et Coll (Chem Rev 1995, 95, 2457).

Cette réaction (viii) est faite en présence d'un complexe de palladium (à l'état d'oxydation (0) ou (II)) tel que par exemple Pd(PPh₃)₄, PdCl₂(PPh₃)₂, Pd₂dba₃, Xphos ou PdCl₂(dppf), dans un solvant polaire, protique ou non tel que le DME, l'éthanol, le DMF, le dioxanne, ou des mélanges de ces solvants, en présence d'une base telle que le carbonate de césium, l'hydrogénocarbonate de sodium aqueux, ou K₃PO₄, en chauffant classiquement entre 80 et 120 °C ou bien sous l'action des micro-ondes entre 130 et 170°C.

Dans le cas où G est un motif NR5R6, où R5 et R6 sont tels que définis pour le composé de formule (I), c'est la **voie 1,** qui est suivie conduisant au composé (X), qui après une étape classique de déprotection, par exemple en présence d'un acide tel que l'HCl (4N) dans le dioxanne ou l'acide trifluoroacétique dans un solvant tel que l'ethanol ou le dichlorométhane, à une température comprise entre -5°C et 60°C, donne le composé de formule (I) objet de l'invention.

Dans le cas ou G est un groupement (C1-C4)alcoxy tel que OEt, c'est la voie 2 qui est suivie, le composé (XI), obtenu par le couplage de Suzuki de l'étape (viii), est saponifié, à l'étape (ix) en présence de LiOH ou de NaOH, dans un mélange de solvant protique tel l'eau , le methanol ou l'éthanol et de solvant aprotique tel que le THF ou le DMF, à température ambiante ou en chauffant à une température comprise entre 50 et 100°C, pour donner le composé (XII). Celui-ci est ensuite engagé dans une réaction de couplage peptidique avec l'amine HNR5R6, où R5 et R6 sont tels que définis pour le composé de formule (I), dans l'étape (x), en présence d'un agent de couplage tel que TBTU, HBTU ou le CDI et d'une base, par exemple la diisopropyléthylamine ou le NaHCO3, dans un solvant aprotique tel que le dichlorométhane, le THF ou le DMF ou par d'autres méthodes connues de l'Homme de l'art, telles que celles décrites par « Principales of Peptide Synthesis », 2nd Ed 1993 M Bodanszky, Springer Laboratory , pour donner le composé de formule (XIII), qui après une étape classique de déprotection, comme décrite précédemment, donne le composé de formule (I) objet de l'invention.

Selon la méthode 2 décrite dans le **schéma 3,** le composé (VIII) est engagé dans une réaction de couplage de Stille avec un dérivé halogéné de formule (IXb) où Pg, m, R1 , R2, R3, R4, V, W, Y et Z sont tels que définis précédemment en rapport avec les composés de formule (I) objets de l'invention étant entendu que le cycle doit comprendre entre 5 et 6 chaînons, X est un atome d'halogène et G est soit un groupement (C1-C4)alcoxy tel que OEt ou un motif -NR5R6, où R5 et R6 sont tels que définis pour le composé de formule (I). Cette réaction (xi) est faite en présence d'un complexe de palladium (à l'état d'oxydation (0) ou (II)) tel que par exemple Pd(PPh₃)₄, PdCl₂(PPh₃)₂, ou Pd₂(dba)₃ et éventuellement avec l'ajout d'un ligand tel que la triphényle arsine, le trifurylphosphine ou la triphenylphosphine dans un solvant polaire aprotique tel que le DMF, le dioxanne ou le THF en chauffant à une température comprise entre 50 et 120 °C.

Dans le cas où le groupement G, sur le composé (IXb), est un motif NR5R6, où R5 et R6 sont tels que définis pour le composé de formule (I), c'est la **voie 1,** qui conduit à la formation du composé (X), qui après une étape classique de déprotection, comme décrite précédemment, donne le composé de formule (I) objet de l'invention.

Dans le cas ou G est un groupement (C1-C4)alcoxy tel que OEt, c'est la **voie 2** qui est suivie, et le composé (XI) est obtenu par le couplage de Stille de l'étape (xi) et les étapes suivantes, (xii), (xiii) et de déprotection pour donner respectivement les composés de formule (XII) et (XIII) et (I) objet de l'invention, sont identiques respectivement aux étapes (ix) ,(x) et de déprotection décrites précédemment

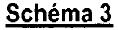

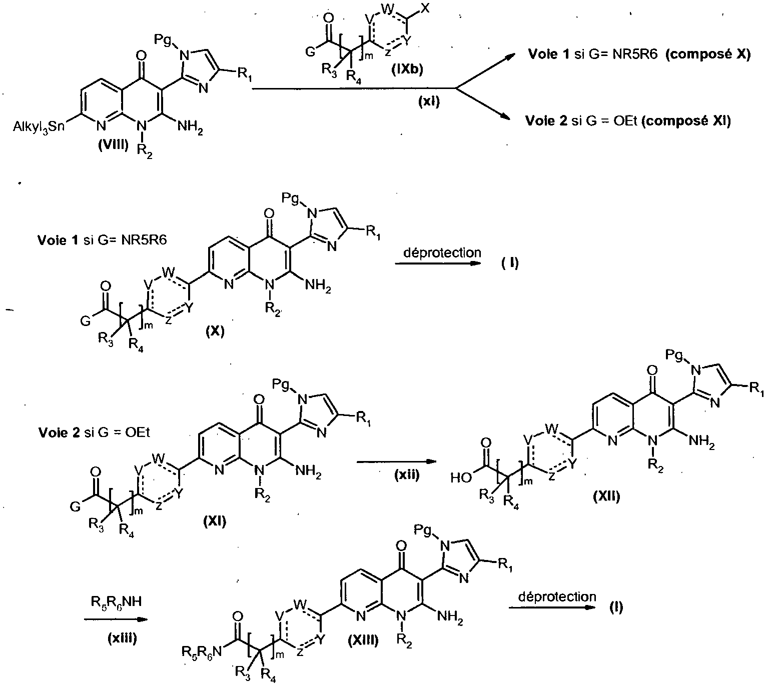

Si nécessaire, certaines fonctions réactives situées sur le groupe R5, R6, R7 ou R2 peuvent être protégées au cours de ces couplages par des groupes protecteurs, tels que décrits dans « Protective Groups in Organic Synthesis », Green et coll. 2nd Edition »

Dans les schémas 1, 2, 3, les composés de départ et les réactifs, quand leur mode de préparation n'est pas décrit, sont disponibles dans le commerce ou décrits dans la littérature, ou bien peuvent être préparés selon des méthodes qui y sont décrites ou qui sont connues de l'Homme du métier.

L'invention, selon un autre de ses aspects, a également pour objet les composés de formules (VII), (VIII), (XI). Ces composés sont utiles comme intermédiaires de synthèse des composés de formule (I).

Les exemples suivants illustrent la préparation de certains composés conformes à l'invention. Ces exemples ne sont pas limitatifs et ne font qu'illuster la présente invention. Les numéros des composés exemplifiés renvoient à ceux donnés dans le tableau ci-après, qui illustre les structures chimiques et les propriétes physiques de quelques composés selon l'invention.

Les abréviations et formules brutes suivantes sont utilisées :

| | |
|---|---|
| AcOEt | Acétate d'éthyle |
| CDI | Carbonyldiimidazole |
| DCM | Dichlorométhane |
| °C | degré Celsius |
| DME | Diméthoxyéthane |
| DMF | Diméthylformamide |
| DMSO | Diméthyl sulfoxide |
| EDC .HCl | Chlorhydrate de N-[3-(diméthylamino)propyl-N'-éthyl carbodiimide |
| HBTU | Hexafluorophosphate de O-(-benzotriazol-1-yl)-N, N, N', N'-tetraméthyluronium. |
| h | Heure(s) |
| HCl | Acide chlorhydrique |
| LiOH | Hydroxyde de lithium |
| Na₂CO₃ | Carbonate de sodium |
| NH₄Cl | Chlorure d'ammonium |
| NaHCO₃ | Hydrogénocarbonate de sodium |
| Na₂SO₄ | Sulfate de sodium |
| NaCl | Chlorure de sodium |
| NaOH | Hydroxyde de sodium |
| NH₄OH | hydoxyde d'ammonium |
| Na₂SO₄ | Sulfate de sodium |
| min. | minutes |
| ml | millilitre |
| P₂O₅ | di-phosphore pentaoxide |
| SEM | 2-Trimethylsilanyl-ethoxymethyl |
| TBTU | Tétrafluoroborate de N-[(1H-benzotriazol-1-yloxy)(diméthylamino) méthylidène]-N-méthylméthanaminium |
| TFA | Acide Trifluoroacétique |
| THF | Tétrahydrofurane |
| T.A. | Température ambiante |
| Tr | temps de rétention |
| TOSMIC | Toulènesulphonylmethyl isocanide |
| Xphos | 2-Dicyclohexylphosphinol-2',4',6'-triisopropylbiphényle |

### Equipements utilisés:

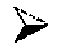 **Appareil de micro-ondes :** Biotage, initiator
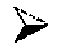 **Condition d'analyse :**

### Conditions de couplage LC/UV/MS :

Instrument (Agilent) : chaîne HPLC : Série 1100, Spectromètre de masse MSD SL (Agilent), Logiciel : Chemstation version B.01.03 de Agilent

### LC/UV

Colonne : Symmetry C18 3.5µm (2.1 x 50 mm) (Waters), Temp. colonne :25°C,
Post run : 5 min Détection UV : 220 nm. Volume d'injection : 2µl d'une solution à 0,5 mg/ml

### Condition 1 : pH 3 gradient 15 minutes

Eluants: A: H₂O + 0,005 % TFA / B : CH₃CN + 0,005 % TFA, Débit: 0.4 ml/min.
Gradient : 0 à 10 min 0 à 100% B et de 10 à 15 min 100 B%

### Condition 2 : pH 3 gradient 30 minutes

Colonne: Symmetry C18 3.5µm (2.1 x 50 mm) (Waters), Temp. colonne :25°C, Eluants : A : H₂O + 0,005 % TFA / B : CH₃CN + 0,005 % TFA, Débit : 0.4ml/min.
Gradient : 0 à 30 min 0 à 100% B et de 30 à 35 min 100 B%
Post run : 6 min. Détection UV : 220 nm. Volume d'injection : 2µl d'une solution à 0,5 mg/ml

### Condition 3 : pH 7 gradient 20 minutes

Colonne : X terra MS C18 3.5 µm (2.1X50 mm), Temp colonne : 20°C Eluants : A H₂O + AcNH₄ (5 nM) + 3% CH₃CN / B : CH₃CN. Gradient 0 à 20 min 0 à 100% de B. Détection UV : 210 nm.

### Condition GC Cl/CH4+) : ionisation CI/CH4 +, 30 minutes

Colonne : Agilent HP-5MS 30 m x 250 µm film 0,25 µm d'épaisseur. Température 250 °C, Gaz vecteur : Helium, débit constant 1,4 ml/ min

### MS:

mode d'ionisation : Electrospray mode positif ESI+, Gamme de masse : 90-1500 uma Spray Chamber Gas temp. : 350°C Drying gas (N₂) :10.0 l/min Neb. pressure : 30 psig Vcap : 4000 V

Les spectres RMN ¹H ont été obtenu en utilisant des spectromètres RMN Bruker 250, 300 ou 400 MHz dans du DMSO-d6, en utilisant le pic du DMSO-d5 comme référence. Les déplacements chimiques δ sont exprimés en partie par million (ppm). Les signaux observés sont exprimés ainsi : s = singulet ; d = doublet ; t = triplet ; m = massif ou large singulet ; H = proton.

Les points de fusions inférieurs à 260°C ont été mesurés avec un appareil banc Koffler et les points de fusions supérieurs à 260°C ont été mesurés avec un appareil Buchi B-545.

Les pouvoirs rotatoires ont été mesurés sur un polarimètre de type: Polarimeter Perkin-Elmer, energy 55µA.

### Example 1: 2-{6-[7-Amino-8-ethyl-6-(1H-imidazol-2-yl)-5-oxo-5,8-dihydro-[1,8]naphthyridin-2-yl]-pyridin-3-yl}-N-pyridin-2-yl-acetamide (composé 2)

### 1.1 / Acide 6-chloro-2-(ethylamino)pyridine-3-carboxylique

Une solution de 18,0 g (84,4 mmol) d'acide 2,6-dichloronicotinique dans 180 ml (3.45 mol) d'une solution d'éthylamine à 70 % dans l'eau est chauffée à 50°C pendant 10 heures. L'excès d'amine est alors évaporé sous pression réduite, puis une solution aqueuse d'acide acétique à 10 % est additionnée jusqu'à la précipitation du produit. Le solide beige est filtré, rincé avec de l'eau froide et séché à l'étuve. On obtient 10,5 g du produit attendu. Rendement = 62 %. Point de fusion : 158-160°C. MH⁺: 201,1 (tr: 7.7 min, condition 1).

### 1.2 / Fluorure de 6-chloro-2-(ethylamino)pyridine-3-carboxylique

A une suspension de 10,5 g (52,3 mmol) du composé obtenu à l'issue de l'étape 1.1 dans le dichlorométhane (250 ml), 4,2 ml (52,3 mmol) de pyridine et 8,4 ml (99,6 mmol) de fluorure cyanurique sont ajoutés successivement. Le mélange est agité pendant 3 heures à température ambiante puis filtré. Le solide est rincé au dichlorométhane (100 ml) et le filtrat est lavé deux fois à l'eau glacée (60 ml). La phase organique est séchée sur Na₂SO₄ puis concentrée sous pression réduite. 10,44 g de produit sont obtenus, sous forme d'huile orange. Rendement = 99 %. Le produit est utilisé sans purification dans l'étape suivante.

### 1.3/ / [1-(2-Trimethylsilanyl-ethoxymethyl)-1H-imidazol-2-yl]-acetonitrile

Sous atmosphère inerte, 0,91 g (4,6 mmol) de TOSMIC en solution dans du DME anhydre (4 ml) sont ajoutés sur 0,96 g (8,6 mmol) de *tert*-butylate de potassium en suspension dans 4 ml de DME anhydre à -35 °C. Le mélange réactionnel est refroidi à - 50°C puis 1 g (4,4 mmol) 1-(2-trimethylsilanyl-ethoxymethyl)-1H-imidazole-2-carbaldehyde (Whitten JP, JOC 1986, 51 (10) 1891-1894) en solution dans 4 ml de DME anhydre est ajouté goutte à goutte en maintenant la température inférieure à -45°C. Le mélange réactionnel est agité à cette température pendant 30 min, puis 11 ml de méthanol sont additionnés, et le mélange est chauffé à 80 °C pendant 15 minutes. Les solvants sont concentrés sous pression réduite, et le résidu est repris avec un mélange eau/acide acétique (13 ml / 0,5 ml), la phase aqueuse est extraite au dichlorométhane (3 X 100 ml), les phases organiques sont ensuite lavées avec une solution saturée de NaHCO₃, puis séchées sur Na₂SO₄. Après filtration le filtrat est concentré sous pression réduite et le résidu est purifié par filtration sur alumine basique (éluant :A/B = DCM/ AcEt 0 à 50% de B), 0,71 g de composé sont ainsi obtenus sous forme d'une huile jaune orange. Rendement = 67%. MH⁺: 238 (tr: 6.9 min, condition 1).
RMN ¹H (300MHZ, DMSO-d₆), δ (ppm) :7,31 (d, 1H); 6,89 (d, 1H); 5,34 (s, 2H); 4,19 (s, 2H); 3,46 (dd, 2H); 0,87 (dd, 2H); 0 (s, 9H).

### 1.4 / 2-Amino-7-chloro-1-ethyl-3-[1-(2-trimethylsilanyl-ethoxymethyl)-1H-imidazol-2-yl]-1H-[1,8]naphthyridin-4-one

Sous atmosphère inerte, 2,4 g (20,5 mmol) de *tert*-butylate de potassium sont ajoutés par portions sur 4,9 g (20,5 mmol) de composé obtenue à l'issue de l'étape 1.3, en solution dans du THF anhydre (50 ml) refroidi à 0°C. Après 45 minutes d'agitation à température ambiante, le milieu réactionnel est refroidi à 0°C, et 4,2 g (20,5 mmol) du composé obtenu à l'issue de l'étape 1.2, en solution dans du THF anhydre (20 ml), sont ajoutés goutte à goutte. Le mélange est agité à température ambiante pendant une heure, puis 3,5 g (30,8 mmol) de *tert*-butylate de potassium sont ajoutés et l'agitation est poursuivie 2 heures. 500 ml d'une solution saturée de chlorure d'ammonium sont ajoutés, et le milieu est acidifié à pH=4 par addition d'une solution (1N) d'HCl. La phase aqueuse est extraite à l'acétate d'éthyle (2x 500 ml). Les phases organiques sont lavées avec une solution saturée de chlorure de sodium, séchées sur Na₂SO₄, filtrées et concentrées sous pression réduite. Le résidu est purifié par flash chromatographie sur silica gel (éluant:A/B = DCM/ Méthanol, de 0 a 5% de B). 6,1 g de composé sont ainsi obtenus sous forme d'un solide marron. Rendement = 70%. Point de fusion = 90°C MH⁺: 419 (tr: 6.6 min, condition 1).
RMN ¹H (300MHZ, DMSO-d₆), δ (ppm) : 8,47 (d, 1H); 7,67 (s, 2H); 7,44 (d, 1H); 7,33 (d, 1 H); 7,1 (d, 1 H); 5,27 (s, 2H); 4,45 (q, 2H); 3,22 (dd, 2H); 1,28 (t, 3H); 0,63 (dd, 2H); -0,2 (s, 9H).

### 1.5 / 2-Amino-1-ethyl-3-[1-(2-trimethylsilanyl-ethoxymethyl)-1H-imidazol-2-yl]-7-trimethylstannanyl-1H-[1,8]naphthyridin-4-one

Dans un tube scellé, une solution de 2 g du composé obtenu à l'issue de l'étape 1.4 dans 12 ml de dioxanne est dégazé à l'argon pendant 15 minutes, puis 2,05 g (6,2 mmol) d'hexamethylditin, 0,16 g (0,50 mmol) de triphénylarsine et 0,54 g (0,75 mmol) de bis(triphénylphosphine)dichloropalladium II sont ajoutés successivement sous argon, puis le tube est fermé. Le mélange réactionnel est chauffé 4h30 à 85°C, puis concentré sous pression réduite. Le résidu est purifié directement sur silice greffée nitrile, (éluant :A/B = (heptane/DCM 1/1) /AcEt de 0 à 100% de B), 2 g de composé sont obtenus sous forme d'une poudre marron. Rendement= 76%. Point de Fusion = 76°C. MH⁺: 550 (tr: 7.2 min, condition 1).
RMN ¹H (400MHZ, DMSO-d₆), δ (ppm) : 8,28 (d, 1H, 7,5 Hz); 7,56 (d, 1 H, 7,5 Hz); 7,53 (s, 2H); 7,32 (d, 1H, 1,3 Hz); 7,1(d, 1H, 1,3 Hz); 5,26 (s, 2H); 4,59 (q, 2H, 6,9 Hz); 3,18 (dd, 2H, 8,2- 8,04 Hz); 1,29 (t, 3H, 6,9 Hz); 0,6 (dd, 2H, 8,2- 8,04 Hz); 0 36 (t, 9H, 28 Hz); 0 (s, 9H).

### 1.6 / 2-(6-Chloro-pyridin-3-yl)-N-pyridin-2-yl-acetamide

Sous atmosphère inerte, 6,2 g (38,5 mmol) de N,N-carbodiimidazole sont ajoutés à une suspension de 6 g (35 mmol) d'acide 2-chloropyridyle acétique dans le THF anhydre (90 ml) à température ambiante. Le mélange réactionnel est agité à cette température pendant 2 heures puis 5,4 g (57,7 mmol) de 2-aminopyridine sont ajoutés et le mélange est chauffé pendant 2 heures à reflux. 200 ml de dichlorométhane sont ajoutés au mélange réactionnel, refroidi préalablement à température ambiante, la phase organique ainsi obtenue est lavée avec une solution saturée de chlorure d'ammonium, puis avec une solution aqueuse de soude (1N), séchée sur Na₂SO₄, filtrée et concentrée sous pression réduite. Le résidu est purifié par flash chromatographie sur silica gel (eluant : A/B = dichlorométhane / acétate d'éthyle de 0% a 70% de B). 5,6 g de composé sont obtenus sous forme d'une poudre blanche. Rendement 65%. Point de fusion : 130°C. MH⁺: 248 (tr: 5.6 min, condition 1).
RMN ¹H 400MHZ, DMSO-d₆), δ (ppm) : 10,81 (s, 1H); 8,35 (d, 1H, 2,3 Hz); 8,33 (ddd, 1 H, 0,9-1,9- 4,9 Hz); 8,03 (d, 1 H, 8,2 Hz); 7,82 (dd, 1 H, 2,5- 8,2Hz); 7,77 (ddd, 1 H, 1,9-7,3, 8,2 Hz); 7,49 (d, 1 H, 8,2Hz); 7,11 (ddd, 1 H, 0,9- 4,9- 7,3Hz); 3,81 (s, 2H).

### 1.7 / 2-(6-{7-Amino-8-ethyl-5-oxo-6-[1-(2-trimethylsilanyl-ethoxymethyl)-1H-imidazol-2-yl]-5,8-dihydro-[1,8]naphthyridin-2-yl}-pyridin-3-yl)-N-pyridin-2-yl-acetamide

Une solution de 0,365 g (1,5 mmol) du composé obtenu à l'issue de l'étape 1.6 et de 1.2 g (2.2 mmol) du composé obtenu à l'issue de l'étape 1.5 dans le dioxanne anhydre (7 ml), est dégazée à l'argon pendant 10 minutes, puis 0.170 g (0,23 mmol) de palladium II dichlorodi(triphénylphosphine) et 0.05 g (0,16 mmol) de triphénylarsine sont ajoutés, le tube est scellé et le mélange réactionnel est chauffé à 100 °C pendant 18 heures. Le mélange réactionnel est dilué avec 100 ml de dichlorométhane, puis la phase organique est lavée avec une solution aqueuse à 10% ammoniaquée, séchée sur Na₂SO₄, filtrée et concentrée sous pression réduite. Le résidu est purifié par flash chromatographie sur silica gel (éluant :A/B = dichlorométhane/méthanol de 0% à 10% de B) 0,32 g de composé sont obtenus, sous forme d'une poudre jaune. Rendement : 37%. MH+ : 597 (tr : 6.1 min, condition 1)

### 1.8/ 2-{6-[7-Amino-8-ethyl-6-(1H-imidazol-2-yl)-5-oxo-5,8-dihydro-[1,8]naphthyridin-2-yl]-pyridin-3-yl}-N-pyridin-2-yl-acetamide chlorhydraté (composé 2)

Sous atmosphère inerte, 1 ml d'acide trifluoroacétique en solution dans 0.5 ml de dichlorométhane sont ajoutés goutte à goutte à une suspension de 0,2 g (0,34 mmol) du composé obtenu à l'issue de l'étape 1.7 en suspension dans le dichlorométhane (1 ml), refroidie à 0°C. Le mélange réactionnel est agité à cette température pendant 10 minutes, puis à température ambiante pendant 5 heures, puis à 10°C pendant une nuit. Le mélange est ensuite versé sur une solution de Na₂CO₃ (2N) (6 ml), préalablement refroidie, le précipité jaune formé est filtré et rincé à l'eau, puis séché sous vide sur P₂O₅. Le solide est purifié par flash chromatographie sur silica gel (éluant :A/B =dichlorométhane/(méthanol/ 1% NH₄OH), de 0% à 10% de B). 0,14 g de composé sont obtenus sous forme d'une poudre jaune. Rendement 87%. 0,1 ml d'une solution concentrée d'HCl (35%) sont ajoutés goutte à goutte au 0,14 g (0,3 mmol) de ce composé en suspension dans le méthanol, le mélange est ensuite agité à température ambiante 45 minutes, puis concentré sous pression réduite. Le solide est trituré dans l'éther diéthylique, filtré et séché sous vide sur P₂O₅. 0,15 g de composé chlorhydraté sont ainsi obtenus sous forme de poudre beige. Rendement : 86%. Point de fusion= 200°C. MH+ 467,2 (tr: 5.49 min, condition 1)
RMN ¹H (400MHZ, DMSO-d₆), δ (ppm) : 11,47 (s, 1 H); 8,76 (d, 1 H, 1,9Hz); 8,59 (d, 1 H, 8 Hz); 8,52 (d, 1 H, 8,2 Hz); 8,39 (d, 1 H, 8 Hz); 8,36 (m,1 H); 8,08 (dd, 1 H, 2-8,3 Hz); 8,03 (d, 1 H, 8,3 Hz); 7,92 (m, 1 H); 7,82 (large s, 1 H+ 1 HCl); 7,66 (s, 2H); 7,22 (m, 1 H); 5,52 (s, 2 H+ 2 HCl); 4,75 (m, 2H); 4,0 (s, 2H); 1,37 (t, 3H, 6,85Hz).

### Exemple 2 : 2-{4-[7-Amino-8-ethyl-6-(1H-imidazol-2-yl)-5-oxo-5,8-dihydro-[1,8]naphthyridin-2-yl]-phenyl}-N-(4-cyclopropyl-morpholin-3-ylmethyl)-acetamide (composé 3)

### 2.1 / ethyl [4-(4,4,5,5-tetramethyl-1,3,2-dioxaborolan-2-yl)phenyl]acetate

Un mélange de 9,5 g (33 mmol) de (4-iodo-phényl)-acétate d'éthyle et de 9,2 g (36 mmol) de bispinacolatodiborane en solution dans le diméthylsulfoxide anhydre (65 ml) est dégazé à l'argon 15 minutes, puis 28 g (98 mmol) d'acétate de potassium et 1,34 g (1,6 mmol) de palladium dichloro(phosphinoferrocène) sont ajoutés et le mélange réactionnel est chauffé à 55°C pendant 1h30 sous argon. Le mélange réactionnel est dilué avec 220 ml d'acétate d'éthyle, puis la phase organique est lavée trois fois avec de l'eau (200 ml), puis séchée sur Na₂SO₄, et concentrée sous pression réduite. 11 g de composé sont obtenus sous forme d'une huile marron, mais utilisée telle que dans l'étape suivante. MH⁺: 291.2 (tr: 9.4 min, condition 1)

### 2.2 / Acide (4-{7-Amino-8-ethyl-5-oxo-6-[1-(2-trimethylsilanyl-ethoxymethyl)-1H-imidazol-2-yl]-5,8-dihydro-[1,8]naphthyridin-2-yl}-phenyl)-acetique

Dans une tube, une suspension de 0,7 g (2,4 mmol) du composé obtenu à l'issue de l'étape 2.1, de 0,5 g (1,2 mmol) du composé obtenu à l'issue de l'étape 1.4 et de 3,3 ml d'une solution saturée d'hydrogénocarbonate dans 7 ml d'un mélange de DME /éthanol (2/1), est dégazée à l'argon pendant 10 minutes, puis 0.08 g (0,07 mmol) de palladium tétrakistriphénylphosphine sont ajoutés et le tube est scellé. Le mélange réactionnel est chauffé à 170°C pendant 15 minutes dans un micro-ondes (Biotage *initiator*). Le mélange réactionnel est ensuite repris dans l'eau, et acidifié par addition d'une solution d'HCl 1(N). Le solide est collecté par filtration, rincé à l'eau puis séché sur P₂O₅ sous vide. 0,87 g de produit sont obtenus sous forme d'un mélange d'acide et d'ester. Ce mélange est utilisé directement dans l'étape suivante.

A 0,87 g du mélange d'acide et d'ester obtenu à l'issue de l'étape précédente, en suspension dans un mélange de solvant THF/ eau/ méthanol (1/ 1/ 1), 0,1 g (2,4 mmol) d'hydroxyde de lithium monohydraté sont ajoutés. Le mélange réactionnel est chauffé à 70°C pendant 5 heures. Au mélange réactionnel refroidi à température ambiante, 3,5 ml d'eau sont ajoutés suivi de l'addition d'une solution d'HCl (1N) jusqu'à pH 1. Le solide est collecté par filtration, rincé au dichlorométhane, puis séché sur P₂O₅ sous vide. 0,4 g de produit sont ainsi isolés, sans autre purification, sous forme de poudre grise. Rendement : 62% pour les deux étapes. Point de fusion : 220°C. MH⁺: 467.2 (tr: 5.49 min, condition 1).
RMN ¹H (400MHZ, DMSO-d₆), δ (ppm) : 14,42 (s, 1H); 12,46 (s, 1H); 8,46 (d, 1H, 7,2 Hz); 8,19 (d, 2H, 7,2 Hz); 8,02 (d, 1H, 7,2 Hz); 7,89 (s, 1H); 7,83 (s, 1H); 7,58 (s, 2H); 7,47 (d, 2H, 7,2 Hz); 5,35 (s, 2H); 4,68 (m, 2H); 3,7(s, 2H); 3 42 (m, 2H); 1,37(m, 3H); 0,77 (m, 2H); -0,11(s, 9H).

### 2.3/ 4-Cyclopropyl-morpholine-3-carboxamide

A 1,2 g (7,2 mmol) de morpholine-3-carboxylic acid amide chlorhydratée (WO2005026156 Hennequin L.F.A. et coll) en solution dans le méthanol (36 ml) 2,9 g de tamis moléculaire 3A , 4,3 g (72 mmol) d'acide acétique, 7,6 g (43,2 mmol) de 2(1-éthoxy-cyclopropyl)oxy]trimethylsilane et 4,4 g (31,7 mmol) de cyanoborohydrure de sodium sont ajoutés successivement. Le mélange réactionnel est chauffé à 70°C pendant 3 heures et demie, puis, refroidi à température ambiante, il est filtré. Le filtrat est concentré sous pression réduite, puis le résidu est repris dans le dichlorométhane (200 ml) et lavé 3 fois avec une solution aqueuse de NaOH (1N) (100 ml). La phase organique est séchée sur Na₂SO₄, filtrée puis concentrée sous pression réduite. 0,58 g de produit sont obtenus, sous forme d'une poudre blanche. Rendement 47%. Point de fusion 116°C. MH⁺: 171 (tr: 1.03 min, condition 2).
RMN ¹H (250MHZ, DMSO-d₆), δ (ppm) :7,33 (s, 1 H); 6,98 (s, 1 H); 3,67-3,43 (large m, 4H); 2,97 (dd, 2H, 7,3 - 3,6 Hz); 2,35 (ddd, 1 H, 11,7- 8,3 - 3,4 Hz); 1,89(ddd, 1 H, 10,3-6,6- 3,6 Hz); 0,56-0,22 (large m, 4H).

### 2.4/ 1-(4-cyclopropylmorpholin-3-yl)methanamine chlorhydraté

Sous atmosphère inerte, à une solution de 0,58 g (3,4 mmol) du composé, obtenu à l'issue de l'étape 2.3, dans le THF anhydre, refroidie à 0°C, 13,6 ml (13,6 mmol) d'une solution (1 N) de triborohydure complexé au tétrahydrofurane dans le THF sont ajoutés goutte à goutte. Le mélange réactionnel est chauffé à 70°C pendant 3h. 15 ml d'une solution (1 N) d'HCl sont ajoutés sur le mélange réactionnel refroidi à température ambiante, après 30 min d'agitation la phase aqueuse est décantée et extraite 2 fois à l'éther (15 ml) puis basifiée par addition d'une solution (1N) de soude. La phase aqueuse est ensuite extraite à l'acétate d'éthyle 4 fois (20 ml) et au dichlorométhane 4 fois (20 ml). Les phases organiques réunies sont séchées sur Na₂SO₄, filtrées et concentrées sous pression réduite. Le résidu est repris dans du méthanol (4 ml) et 3,4 ml d'une solution d'HCl (1N) dans l'éther sont ajoutés, la solution est agitée 30 minutes puis 5 ml d'éther sont ajoutés, le solide formé est collecté par filtration et séché sous vide sur P₂O₅. 0,51 g de produit sont obtenus, sous forme d'une poudre blanche. Rendement 78%.. MH⁺: 157 (tr: 0.4 min, condition 2).
RMN ¹H (400MHZ, DMSO-d₆), δ (ppm) : 11,67(s, 1 H); 8,07 (m, 2H); 4,09 (m, 1 H); 3,94 (m, 1 H); 3,74 (m, 2H); 3,54 (m, 2H); 3,36 (m, 1 H); 3,2 (m, 1 H); 2,99 (m, 1 H); 1,25 (m, 1H); 1,09- 0,72(m, 4H).

### 2.5/ 2-{4-[7-amino-8-ethyl-5-oxo-6-(1-{[2-(trimethylsilyl)ethoxy]methyl}-1H-imidazol-2-yl)-5,8-dihydro-1,8-naphthyridin-2-yl]phenyl}-N-[(4-cyclopropylmorpholin-3-yl)methyl]acetamide

Sous atmosphère inerte à 0,4 g (0,8 mmol) de composé obtenu à l'issue de l'étape 2.2, en suspension dans le DMF (8 ml), 0,4 ml (2,3 mmol) de diisopropyle éthyle amine sont ajoutés, après solubilisation, le mélange réactionnel est refroidi à 0°C puis une solution dans le DMF (2 ml) de 0,18 g (0,94 mmol) de composé obtenu à l'issue de l'étape 2.4 et de 0,2 ml (1,2 mmol) de diisopropyle éthyle amine, suivi de l'addition de 0,275 g (0,86 mmol) de TBTU. Le mélange réactionnel est agité à température ambiante pendant 3 heures, puis concentré à sec. Le résidu est directement purifié par flash chromatographie sur colonne de silica gel (éluant :A/B = dichlorométhane/ méthanol (1% NH4OH) gradient de 0 à 10% de B). 0,34 g de produit sont obtenus sous forme d'une poudre jaune. Rendement : 65%. Point de fusion : 116°C.
MH+ : 657 (tr : 5.51 min, condition 1)

### 2.6 / 2-{4-[7-Amino-8-ethyl-6-(1H-imidazol-2-yl)-5-oxo-5,8-dihydro-[1,8]naphthyridin-2-yl]-phenyl}-N-(4-cyclopropyl-morpholin-3-ylmethyl)-acetamide Chlorhyraté (composé 3)

A 0,3 g (0,47 mmol) de composé obtenu à l'issue de l'étape 2.5, en solution dans 0.4 ml le dichlorométhane, refroidi à -10°C, 1.4 ml (19 mmol) d'acide trifluoracétique en solution dans 0.4 ml de dichlorométhane, sont ajoutés goutte à goutte, puis le mélange réactionnel est agité à 5 °C pendant une nuit puis versé sur une solution de Na₂CO₃ (2M) (10 ml) à froid (bain de glace). Le précipité jaune formé est collecté par filtration et rincé à l'eau puis séché sur P₂O₅ sous vide. Les 0.18g de solide sont repris dans 2ml de méthanol et 70µl d'une solution d'HCl à 36% sont ajoutés, le mélange est agité une heure à température ambiante puis concentré sous vide. Le résidu est purifié par flash chromatographie sur colonne phase inverse C8 (éluant: eau HCl (N/ 1000). 0,034 g de produit sont obtenus sous forme d'une poudre jaune. Rendement = 16%. Point de fusion : 210°C. MH+ : 528.2 (tr: 4.78 min, condition 1).
RMN ¹H (400MHZ, DMSO-d₆), δ (ppm) : 11,29 (s, 1 H); 8,52 (d, 1 H, 8,2 Hz); 8,48 (s, 1 H échangeable); 8,18 (d, 2H + 1 H échangeable, 8,3 Hz); 7,99 (d, 1 H + 1 H échangeable, 8,2 Hz); 7,54 (s, 2H); 7,48 (d, 2H, 8,3 Hz); 4,7 (large q, 2H, 7 Hz); 3,91 (m, 3H); 3,73 (m, 1 H); 3,58 (s, 3H); 3,47 (m, 2H); 3,34 (m, 2H); 2,93 (m, 1 H); 1,36 (t, 3H, 7Hz); 1,25 (m, 1H); 0,95 (m, 2H); 0,81 (m, 1 H).

### Exemple 3: 2-{4-[7-Amino-8-ethyl-6-(1H-imidazol-2-yl)-5-oxo-5,8-dihydro-[1,8]naphthyridin-2-yl]-phenyl}-N-pyridin-4-ylmethyl-propionamide (composé 47)

### 3.1/ ethyl 2-[4-(4,4,5,5-tetramethyl-1,3,2-dioxaborolan-2-yl)phenyl]propanoate

Même mode opératoire que celui décrit dans l'exemple 2, étape 2.1, à partir de 0,6 g (2 mmol) d'éthyle 2-(4-iodophenyl)propanoate en solution dans du DMSO anhydre (4 ml), 0,57 g (2.2 mmol) de bispinacolatodiborane , 1.7 g (6 mmol) d'acétate de potassium et 83 mg (0.1 mmol) de palladium dichloro(phosphinoferrocène). 0.9 g de composé sont obtenus sous forme d'une huile marron, utilisée telle que dans l'étape suivante. MH⁺: 305 (tr: 9.9 min, condition 1)

### 3.2 / Acide 2-{4-[7-amino-8-ethyl-5-oxo-6-(1-{[2-(trimethylsilyl)ethoxy]methyl}-1H-imidazol-2-yl)-5,8-dihydro-1,8-naphthyridin-2-yl]phenyl}propanoique

Même mode opératoire que celui décrit dans l'exemple 2, étape 2.2, à partir de 0,57 g (1,4 mmol) du composé obtenu à l'issue de l'étape 3.1, de 0,82 g (2.7 mmol) du composé obtenu à l'issue de l'étape 1.4, de 94 mg (0,08 mmol) de palladium tétrakistriphénylphosphine, de 4 ml d'une solution saturée d'hydrogénocarbonate dans un mélange de DME/ éthanol (2/1) (7 ml), 1.1 g de produit sont obtenus sous forme d'un mélange d'acide et d'ester. Ce mélange est utilisé directement dans l'étape suivante, en présence de 0.16 g (3.8 mmol) d'hydroxyde de lithium monohydraté suspension dans un mélange de solvant THF/ eau/ méthanol (1/ 1/ 1) (10 ml). 0,4 g de produit sont obtenus sous forme de poudre marron. Rendement : 39% pour les deux étapes. Point de fusion :248°C. MH+ 534.1 (tr: 6,5 min, condition 1)

### 3.3/ 2-{4-[7-amino-8-ethyl-5-oxo-6-(1-{[2-(trimethylsilyl)ethoxy]methyl}-1H-imidazol-2-yl)-5,8-dihydro-1,8-naphthyridin-2-yl]phenyl}-N-(pyridin-4-ylmethyl)propanamide

Même mode opératoire que celui décrit dans l'exemple 2, étape 2.5, à partir de 0,3 g (0,56 mmol) de composé obtenu à l'issue de l'étape 3.2, de 0,25 g (2.25 mmol) de 1-(pyridin-4-yl)methanamine, de 0,36 g (1.1 mmol) de TBTU et de 0,14 g (1.1 mmol) de diisopropyléthylamine dans le DMF anhydre (5 ml), 0,29 g de produit sont obtenus sous forme de poudre jaune. Rendement : 82%. Point de fusion : 140°C. MH⁺ : 624 (tr: 5.66 min, condition 1).

### 3.4/ 2-{4-[7-Amino-8-ethyl-6-(1H-imidazol-2-yl)-5-oxo-5,8-dihydro-[1,8]naphthyridin-2-yl]-phenyl}-N-pyridin-4-ylmethyl-propionamide (composé 47)

Même mode opératoire que celui décrit dans l'exemple 2, étape 2.6, à partir de 0,26 g (0,42 mmol) de composé obtenu à l'issue de l'étape 3.3 en solution dans 1.2ml de dichlorométhane, de 1.3 ml (17 mml) d'acide trifluoroacétique en solution dans 0.5 ml de dichlorométhane. 0,051 g de produit sont obtenus sous forme d'une poudre jaune. Rendement = 24%. Point de fusion : 186°C. MH⁺ : 494.2 (tr: 5.05 min, condition 1). RMN ¹H (400MHZ, DMSO-d₆), δ (ppm) : 13.20 (s, 1 H); 11.51 (s, 1 H); 8.62 (m, 2H); 8.45 (dd, 2H, 4.5, 1.5 Hz); 8.29 (d, 2H, 8.2 Hz); 8.02 (large s, 1 H échangeable); 7.97 (d, 1 H, 8.2 Hz); 7.53 (d, 2H, 8.2 Hz); 7.16 (m, 3H); 7.04 (m, 1H); 4.72 (m, 2H); 4.29 (d, 2H, 6 Hz); 3.81 (q, 1 H, 7 Hz); 1.45 (d, 3H, 7.1 Hz); 1.38 (t, 3H, 7 Hz).

### Exemple 4: 4-{4-[7-Amino-8-ethyl-6-(1H-imidazol-2-yl)-5-oxo-5,8-dihydro-[1,8]naphthyridin-2-yl]-phenyl}-N-pyridin-4-ylmethyl-butyramide (composé 49)

### 4.1/ Acide 4-[4-(4,4,5,5-tetramethyl-1,3,2-dioxaborolan-2-yl)phenyl]butanoique

Même mode opératoire que celui décrit dans l'exemple 2, étape 2.1, à partir de 0,3 g (1 mmol) 4-(4-iodophenyl)butanoic acide en solution dans du DMSO anhydre (2 ml), 0,29 g (1.1mmol) de bispinacolatodiborane, 0.3 g (3.1 mmol) d'acétate de potassium et 42 mg (0.05 mmol) de palladium dichloro(phosphinoferrocène). 0.21 g de composé sont obtenus sous forme d'une huile marron, utilisée telle que dans l'étape suivante. MH⁺: 291 (tr: 8.35 min, condition 1)

### 4.2 / Acide 4-[4-(7-amino-8-ethyl-5-oxo-6-{1-[2-(trimethylsilyl)ethoxy]-1H-imidazol-2-yl}-5,8-dihydro-1,8-naphthyridin-2-yl)phenyl]butanoique

Même mode opératoire que celui décrit dans l'exemple 2, étape 2.2, à partir de 0,17 g (0.57 mmol) du composé obtenu à l'issue de l'étape 4.1, de 0,18 g (0.44 mmol) du composé obtenu à l'issue de l'étape 1.4, de 25 mg (0,02 mmol) de palladium tétrakistriphénylphosphine, de 1.2 ml d'une solution saturée d'hydrogénocarbonate dans un mélange de DME/éthanol (2/1) (3 ml), 0.25 g de produit sont obtenus sous forme d'une huile marron utilisée telle que dans l'étape suivante. MH⁺ : 548 (tr: 6,5 min, condition 1)

### 4.3/ 4-[4-(7-amino-8-ethyl-5-oxo-6-{1-[2-(trimethylsilyl)ethoxy]-1H-imidazol-2-yl}-5,8-dihydro-1,8-naphthyridin-2-yl)phenyl]-N-(pyridin-4-ylmethyl)butanamide

Même mode opératoire que celui décrit dans l'exemple 2, étape 2.5, à partir de 0,5 g (0,46 mmol) de composé obtenu à l'issue de l'étape 4.2, de 0,2 g (1.8 mmol) de 1-(pyridin-4-yl)methanamine, de 0,3 g (0.9 mmol) de TBTU et de 0,12 g (0.9 mmol) de diisopropyléthylamine dans le DMF anhydre (5 ml), 0,1 g de produit sont obtenus sous forme de poudre beige. Rendement : 34%. Point de fusion : 223°C. MH⁺ : 638 (tr: 5.75 min, condition 1).

### 4.4/ 4-{4-[7-Amino-8-ethyl-6-(1H-imidazol-2-yl)-5-oxo-5,8-dihydro-[1,8]naphthyridin-2-yl]-phenyl}-N-pyridin-4-ylmethyl-butyramide (composé 49)

Même mode opératoire que celui décrit dans l'exemple 2, étape 2.6, à partir de 0,095 g (0,15 mmol) de composé obtenu à l'issue de l'étape 4.3 en solution dans 0.5 ml de dichlorométhane, de 0.44 ml (6 mmol) d'acide trifluoracétique en solution dans 0.3 ml de dichlorométhane. 0.045g de produit sont obtenus sous forme d'une poudre jaune. Rendement = 60%. Point de fusion : 240°C. MH⁺ : 508.2 (tr: 4.97 min, condition 1).
RMN ¹H (400MHZ, DMSO-d₆), δ (ppm) : 13.21 (s, 1H); 11.48 (s, 1H); 8.61 (d, 1H, 8 Hz); 8.49 (d, 2H, 4.5 Hz); 8.44 (t, 1H, 6 Hz); 8.45 (large s, 1 H échangeable); 8.17 (d, 2H, 8.1 Hz); 7.97 (d, 1 H, 8.1 Hz); 7.39 (t, 2H, 8.1 Hz); 7.24 (d, 1 H, 4.8Hz); 7.15 (s, 1 H); 7.03 (s, 1 H); 4.72 (m, 2H); 4.30 (d, 2H, 5.8 Hz); 3.81 (q, 1 H, 7 Hz); 2.68 (t, 2H, 7.5 Hz); 2.24 (t, 2H, 7.3 Hz); 1.92 (m, 2H); 1.38 (t, 3H, 7 Hz).

### Exemple 5 : 2-{4-[7-Amino-8-ethyl-6-(4-methyl-1H-imidazol-2-yl)-5-oxo-5,8-dihydro-[1,8]naphthyridin-2-yl]-phenyl}-N-pyridin-4-ylmethyl-acetamide (composé 50)

### 5.1/ [1-(2-Trimethylsilanyl-ethoxymethyl)-1H-(4-methyl-1H-imidazol-2-yl)acetonitrile

Même mode opératoire que celui décrit dans l'exemple 1, étape 1.3, à partir de 4.6 g (19 mmol) de [1-(2-Trimethylsilanyl-ethoxymethyl)-1H-(4-methyl-1H-imidazol-2-yl)carbaldéhyde, 4 g (20.3 mmol) de TOSMIC et de 4.3 g de *tert*-butylate de potassuim en solution dans du DME anhydre (32 ml). 2.2 g de composé sont obtenus sous forme d'une huile jaune en mélange 70/30 de régioisomères τ et π (. Rendement 45%. MH⁺: 252 (tr: 6.38 et 6.55 min, condition 1)

### 5.2 / 2-amino-7-chloro-1-ethyl-3-(4-methyl-1-{[2-(trimethylsilyl)ethoxy]methyl}-1H-imidazol-2-yl)-1,8-naphthyridin-4(1H)-one

Même mode opératoire que celui décrit dans l'exemple 1, étape 1.4, à partir de 1 g (4 mmol) du composé obtenu à l'issue de l'étape 5.1, de 0,8 g (4 mmol) du composé obtenu à l'issue de l'étape 1.2 et de 1.15g (10 mmol) de *tert*-butylate de potassuim en solution dans du THF anhydre (13 ml). 0.42 g de produit sont obtenus sous forme d'une poudre beige. Rendement 24%. Point de fusion : 120°C. MH⁺ : 435 (tr: 10.5 et 10.6 min, condition 1)

### 5.3/ Acide {4-[7-amino-8-ethyl-6-(4-methyl-1-{[2-(trimethylsilyl)ethoxy]methyl}-1H-imidazol-2-yl)-5-oxo-5,8-dihydro-1,8-naphthyridin-2-yl]phenyl}acetique

Même mode opératoire que celui décrit dans l'exemple 2, étape 2.2, à partir de 0,42 g (1 mmol) de composé obtenu à l'issue de l'étape 5.2, de 0,84 g (2.9 mmol) de composé obtenu à l'issue de l'étape 2.1, de 67 mg (0.06 mmol) de palladium tétrakistriphénylphosphine, de 1.2 ml d'une solution saturée d'hydrogénocarbonate dans un mélange de solvant DME/EtOH (2 /1) (7 ml). 0.69 g de produit sont obtenus sous forme d'un mélange d'acide et d'ester. Ce mélange est utilisé directement dans l'étape suivante, en présence de 0.12 g (3 mmol) d'hydroxyde de lithium monohydraté suspension dans un mélange de solvant THF/ eau/ méthanol (1/ 1/ 1) (7 ml). 0,67 g de produit sont obtenus sous forme de poudre marron utilisée sans purification. MH⁺ : 534 (tr: 4.38 min, condition 1).

### 5.4/ 2-{4-[7-amino-8-ethyl-6-(4-methyl-1-{[2-(trimethylsilyl)ethoxy]methyl}-1H-imidazol-2-yl)-5-oxo-5,8-dihydro-1,8-naphthyridin-2-yl]phenyl}-N-(pyridin-4-ylmethyl)acetamide

Même mode opératoire que celui décrit dans l'exemple 2, étape 2.5, à partir de 0,67 g (1.2 mmol) de composé obtenu à l'issue de l'étape 5.3, de 0,55 g (5 mmol) de 1-(pyridin-4-yl)methanamine, de 0,8 g (2.5 mmol) de TBTU et de 0,32 g (2.4 mmol) de diisopropyléthylamine dans le DMF anhydre (12 ml), 0.22 g de produit sont obtenus sous forme de poudre jaune. Rendement : 28%. MH⁺ : 624 (tr: 5.6 min, condition 1).

### 5.5/ 2-{4-[7-Amino-8-ethyl-6-(4-methyl-1H-imidazol-2-yl)-5-oxo-5,8-dihydro-[1,8]naphthyridin-2-yl]-phenyl}-N-pyridin-4-ylmethyl-acetamide sel de TFA (composé 50)

Même mode opératoire que celui décrit dans l'exemple 2, étape 2.6, à partir de 0,22 g (0,36 mmol) de composé obtenu à l'issue de l'étape 5.4 en solution dans 0.5 ml de dichlorométhane, de 1.6 ml (14 mmol) d'acide trifluoroacétique en solution dans 0.5 ml de dichlorométhane, purification par HPLC. 0.124 g de produit sous forme de sel d'acide trifluoroacétique, sont obtenus sous forme d'une poudre jaune. Rendement = 60%. Point de fusion : 144°C. MH⁺ : 494 (tr: 4.54 min, condition 1).
RMN ¹H (400MHZ, DMSO-d₆), δ (ppm) : 8.83 (t, 1 H, 5.9 Hz); 8.68 (d, 2H, 5.2 Hz); 8.52 (d, 1 H, 8.1 Hz); 8.19 (d, 2H, 8.3 Hz); 8.09 (large s, 1 H échangeable); 8.0 (d, 1 H, 8.2 Hz); 7.6 (d, 2H, 5.9 Hz); 7.5 (d, 2H, 8.3 Hz); 7.2 (s, 1H); 4.7 (m, 2H); 5.4-4 (large s, 2H échangeables); 4.46 (d, 2H, 6 Hz); 3.66 (s, 2H); 2.3 (s, 3H); 1.4 (t, 3H, 7 Hz)

### Exemple 6: 2-{4-[7-Amino-8-cyclopropylmethyl-6-(1H-imidazol-2-yl)-5-oxo-5,8-dihydro-[1,8]naphthyridin-2-yl]-phenyl}-N-pyridin-4-ylmethyl-acetamide (composé 51)

### 6.1/ Acide 6-chloro-2-[(cyclopropylmethyl)amino]pyridine-3-carboxylique

Dans un tube à sceller à 3 g (14 mmol) d'acide 2,6-dichloronitinique en solution dans le *tert*-butanol (14 ml), 3 g (42 mmol) de cyclopropylmethylamine sont ajoutés, le tube est scellé et chauffé a 170°C pendant 30 minutes dans un appareil à micro-ondes Biotage Initiator. Le mélange réactionnel est refroidi à température ambiante, dilué dans le dichlorométhane (100 ml) et lavé avec une solution d'acide acétique aqueuse a 10% (12 ml). La phase organique est séchée sur Na₂SO₄, filtrée, concentrée et séchée sous vide. 3.4 g de produit sont obtenus sous forme d'une huile orangée. Rendement quantitatif. MH⁺ : 227 (tr: 4.54 min, condition 1)

### 6.2/ 6-chloro-2-[(cyclopropylmethyl)amino]pyridine-3-carbonyl fluorure

Même mode opératoire que celui décrit dans l'exemple 1, étape 1.2, à partir de 0.43 g (2 mmol) de composé obtenu à l'issue de l'étape 6.1 en solution dans 4 ml de dichlorométhane, de 0.52 g (3.8 mmol) de fluorure cyanurique, et de 0.4 g (3.8 mmol) de triéthylamine. Le produit, obtenu sous forme d'une huile verte, est utilisé sans purification dans l'étape suivante.

### 6.3/ 2-amino-7-chloro-1-(cyclopropylmethyl)-3-(1-{[2-(trimethylsilyl)ethoxy]methyl}-1H-imidazol-2-yl)-1,8-naphthyridin-4(1H)-one

Même mode opératoire que celui décrit dans l'exemple 1, étape 1.4, à partir de 0.43 g (2 mmol) de composé obtenu à l'issue de l'étape 6.2, de 0.5 g (2 mmol)) de composé obtenu à l'issue de l'étape 1.3 en solution dans 6 ml de THF anhydre et de 0.55 g (5 mmol) de *tert*-butylate de potassium et de 0.4 g (3.8 mmol) de triéthylamine 0.5 g de produit sont obtenus sous forme d'une poudre marron. Rendement = 60%. Point de fusion: 70°C. MH⁺ : 447 (tr: 6.68 min, condition 1).

### 6.4/ Acide {4-[7-amino-8-(cyclopropylmethyl)-5-oxo-6-(1-{[2-(trimethylsilyl)ethoxy]methyl}-1H-imidazol-2-yl)-5,8-dihydro-1,8-naphthyridin-2-yl]phenyl}acetique

Même mode opératoire que celui décrit dans l'exemple 2, étape 2.2, à partir de 0,44 g (1 mmol) de composé obtenu à l'issue de l'étape 6.3, de 1 g (3.5 mmol) de composé obtenu à l'issue de l'étape 2.1, de 70 mg (0.06 mmol) de palladium tétrakistriphénylphosphine, de 2.8 ml d'une solution saturée d'hydrogénocarbonate dans un mélange de solvant DME/EtOH (2/1) (8 ml). 0.8 g de produit sont obtenus sous forme d'un mélange d'acide et d'ester. Ce mélange est utilisé directement dans l'étape suivante, en présence de 0.15 g (3.6 mmol) d'hydroxyde de lithium monohydraté en suspension dans un mélange de solvant THF/ eau/ méthanol (1/ 1/ 1) (9 ml). 0,91 g de produit sont obtenus sous forme de poudre marron, utilisés sans purification dans l'étape suivante. MH⁺ : 546 (tr: 4.39 min, condition 1).

### 6.5/ 2-{4-[7-amino-8-(cyclopropylmethyl)-5-oxo-6-(1-{[2-(trimethylsilyl)ethoxy]methyl}-1H-imidazol-2-yl)-5,8-dihydro-1,8-naphthyridin-2-yl]phenyl}-N-(pyridin-4-ylmethyl)acetamide

Même mode opératoire que celui décrit dans l'exemple 2, étape 2.5, à partir de 0,9 g (1.7 mmol) de composé obtenu à l'issue de l'étape 6.4, de 0,73 g (6.7 mmol) de 1-(pyridin-4-yl)methanamine, de 1.1 g (3.3 mmol) de TBTU et de 0,43 g (3.3 mmol) de diisopropyléthylamine dans le DMF anhydre (17 ml), 0.3 g de produit sont obtenus sous forme de poudre beige. Rendement : 28%. Point de fusion : 114°C. MH⁺ : 635 (tr: 5.6 min, condition 1).

### 6.6/ 2-{4-[7-Amino-8-cyclopropylmethyl-6-(1H-imidazol-2-yl)-5-oxo-5,8-dihydro-[1,8]naphthyridin-2-yl]-phenyl}-N-pyridin-4-ylmethyl-acetamide sel de TFA (composé 51)

Même mode opératoire que celui décrit dans l'exemple 2, étape 2.6, à partir de 0,28 g (0,45 mmol) de composé obtenu à l'issue de l'étape 6.5 en solution dans 1.5 ml de dichlorométhane, de 1.3 ml (18 mmol) d'acide trifluoroacétique en solution dans 0.8 ml de dichlorométhane. 0.2 g de produit sont obtenus sous forme d'une poudre jaune. Rendement = 60%. Point de fusion : 118°C.. MH⁺ : 506 (tr: 4.96 min, condition 1). RMN ¹H (400MHZ, DMSO-d₆), δ (ppm) : 8.85 (t, 1 H, 5.7 Hz); 8.7 (d, 2H, 5.8 Hz); 8.58 (large s, 1H échangeable); 8.56 (d, 1H, 8.1 Hz); 8.16 (d, 2H, 8.1 Hz); 8.0 (d, 1H, 8.2 Hz); 7.64 (d, 2H, 5.8 Hz); 7.50 (d, 2H, 8.2 Hz); 7.4 (s, 2H); 4.7 (m, 2H); 5.4-4.0 (large s, 2H échangeable); 4.50 (d, 2H, 5.8 Hz); 3.67 (s, 2H); 1.4 (m, 1 H); 0.6 (m, 2H); 0.5 (m, 2H).

### Exemple 7: 2-{5-[7-Amino-8-ethyl-6-(1H-imidazol-2-yl)-5-oxo-5,8-dihydro-[1,8]naphthyridin-2-yl]-pyridin-2-yl}-N-pyridin-4-ylmethyl-acetamide (composé 53)

### 7.1/ 2-(5-bromopyridin-2-yl)-N-(pyridin-4-ylmethyl)acetamide

Même mode opératoire que celui décrit dans l'exemple 2, étape 2.5, à partir de 0,33 g (1.5 mmol) d'acide (5-bromo-pyridin-2-yl)-acétique (dont une synthèse est décrite dans Tetrahedron, 1997, 53(24) 8257-8268 Gurnos J. et coll) de 0,67 g (6.1 mmol) de 1-(pyridin-4-yl)methanamine, de 1 g (3 mmol) de TBTU et de 0,4 g (3 mmol) de diisopropyléthylamine dans le DMF anhydre (15 ml), 0.59 g de produit sont obtenus sous forme de poudre beige, utilisés sans purification dans l'étape suivante. MH⁺ : 307 (tr: 2.92 min, condition 1).

### 7.2/ 2-{5-[7-amino-8-ethyl-5-oxo-6-(1-{[2-(trimethylsilyl)ethoxy]methyl}-1H-imidazol-2-yl)-5,8-dihydro-1,8-naphthyridin-2-yl]pyridin-2-yl}-N-(pyridin-4-ylmethyl)acetamide

Même mode opératoire que celui décrit dans l'exemple 1, étape 1.7, à partir de 0,58 g (1.9 mmol) de composé obtenu à l'issue de l'étape 7.1, de 1.2 g (2.3 mmol) de composé obtenu à l'issue de l'étape 1.5, de 220 mg (0,3 mmol) de palladium II dichlorodi(triphénylphosphine) et de 65 mg (0,21 mmol) de triphénylarsine en solution dans 9 ml de dioxanne anhydre. 0,38 g de composé sont obtenus, sous forme d'une poudre jaune. Rendement : 32%. MH⁺: 611 (tr: 5.43 min, condition 1).

### 7.3/ 2-{5-[7-Amino-8-ethyl-6-(1H-imidazol-2-yl)-5-oxo-5,8-dihydro-[1,8]naphthyridin-2-yl]-pyridin-2-yl}-N-pyridin-4-ylmethyl-acetamide (composé 53)

Même mode opératoire que celui décrit dans l'exemple 1, étape 1.8, à partir de 0,36 g (0,6 mmol) de composé obtenu à l'issue de l'étape 7.2 en solution dans 1.8 ml de dichlorométhane, de 1.8 ml (24 mmol) d'acide trifluoroacétique en solution dans 1 ml de dichlorométhane. 0.314 g de produit sont obtenus sous forme d'une poudre jaune. Rendement = 78%. Point de fusion : 120°C. MH⁺ : 481 (tr: 4.32 min, condition 1).
RMN ¹H (400MHZ, DMSO-d₆), δ (ppm) : 9.38 (d, 1 H, 2.3 Hz); 8.93 (t, 1 H, 6 Hz); 8.77 (d, 2H, 6.3 Hz); 8.68 (large s, 1 H échangeable); 8.62 (d, 1 H, 8Hz); 8.57 (dd, 1 H, 8.2, 2.4 Hz); 8.1 (d, 1 H, 8 Hz); 7.77 (d, 2H, 6 Hz); 7.60 (d, 1 H, 8.2Hz); 7.41 (s, 2H); 4.71 (m, 2H); 5.4-4 (large s, 2H échangeables); 4.54 (d, 2H, 6 Hz); 3.90 (s, 2H); 1.38 (t, 3H, 7 Hz).

### Exemple 8: 2-{4-[7-Amino-8-ethyl-6-(1H-imidazol-2-yl)-5-oxo-5,8-dihydro-[1,8]naphthyridin-2-yl]-2-fluoro-phenyl}-N-(1-ethyl-pyrrolidin-2-ylmethyl)-acetamide (composé 56)

### 8.1/ Acide (4-bromo-2-fluorophenyl)acetique

A 0.37 g (1.4 mmol) d'ester ethylique de l'acide (4-bromo-2-fluorophenyl)acetique en solution dans 8 ml d'un mélange de solvant THF/ méthanol/ eau (1/1/1), 0.09g d'hydoxyde de lithium monhydraté sont ajoutés, et le mélange réactionnel est agité a température ambiante pendant 3h à température ambiante. Le mélange réactionnel est acidifié avec une solution (1 N) d'HCl, puis extraite au dichlorométhane (20ml). Les phases organiques sont reunies, séchées sur Na₂SO₄, filtrées et concentrées sous pression réduite. 0.3 g de produit sont obtenus sous forme de gomme et utilisés sans purification dans l'étape suivante. Rendement 91%.

### 8.2/ (4-bromo-2-fluorophenyl)acetyl chlorure

A 0.28 g (1.2 mmol) du composé obtenu à l'issue de l'étape 8.1 en solution dans 7 ml de 1,2-dichloroéthane, 0.3 g (2.4 mmol) de chlorure d'oxalyle sont ajoutés. Le mélange réactionnel est agité à température ambiante pendat 1h30 puis concentré sous pression réduite, 0.322 g de composé sont obtenus sous forme d'une huile et utilisé sans purification dans l'étape suivante.

### 8.3/ 2-(4-bromo-2-fluorophenyl)-N-[(1-ethylpyrrolidin-2-yl)methyl]acetamide

A 0.3 g (1.2 mmol) du composé obtenu a l'issue de l'étape 8.2 en solution dans 8 ml de dichlorométhane refroidi par un bain de glace et sous atmosphère inerte, 0.15 g (1.2 mmol) de 1-(1-ethylpyrrolidin-2-yl)methanamine sont ajoutés et l'agitation est poursuivie pendant 4h. Le mélange réactionnel est dilué avec 20 ml de dichlorométhane et lavé avec 20 ml d'eau, puis la phase organique est séchée sur Na₂SO₄ filtrée et concentrée sous pression réduite. Le résidu est ensuite purifié par flash chromatographie sur silica gel, (éluant :A/B = dichlorométhane/ méthanol (1% NH4OH) gradient de 0 à 10% de B). 0,2 g de produit sont obtenus sous forme d'une poudre beige. Rendement : 50%. MH⁺ 343 (tr : 4.94 min , condition 1).

### 8.4 / 2-{4-[7-amino-8-ethyl-5-oxo-6-(1-{[2-(trimethylsilyl)ethoxy]methyl}-1H-imidazol-2-yl)-5,8-dihydro-1,8-naphthyridin-2-yl]-2-fluorophenyl}-N-[(1-ethylpyrrolidin-2-yl)methyl]acetamide

Même mode opératoire que celui décrit dans l'exemple 1, étape 1.7, à partir de 0,074 g (0.22 mmol) de composé obtenu à l'issue de l'étape 8.3, de 0.15 g (0.3 mmol) de composé obtenu à l'issue de l'étape 1.5, de 24 mg (0,03 mmol) de palladium II dichlorodi(triphénylphosphine) et de 8 mg (0,02 mmol) de triphénylarsine en solution dans 1 ml de dioxanne anhydre. 0,063 g de composé sont obtenus, sous forme d'une poudre jaune. Rendement : 45%. MH⁺: 648 (tr: 5.31 min, condition 1).

### 8.5/ 2-{4-[7-Amino-8-ethyl-6-(1H-imidazol-2-yl)-5-oxo-5,8-dihydro-[1,8]naphthyridin-2-yl]-2-fluoro-phenyl}-N-(1-ethyl-pyrrolidin-2-ylmethyl)-acetamide (composé 56)

Même mode opératoire que celui décrit dans l'exemple 2, étape 2.6, à partir de 0,055 g (0,08 mmol) de composé obtenu à l'issue de l'étape 8.4 en solution dans 0.25 ml de dichlorométhane, de 0.25 ml (3.4 mmol) d'acide trifluoroacétique en solution dans 0.1 ml de dichlorométhane. 0.017 g de produit sont obtenus sous forme d'une poudre jaune. Rendement = 39%. Point de fusion : 100°C. MH⁺ : 518.3 (tr: 4.71 min, condition 1).
RMN ¹H (400MHZ, DMSO-d₆), δ (ppm) : 9.15 (s, 1H); 8.62 (d, 1H, 8.1 Hz); 8.50 (t, 1H, 5.7 Hz); 8.07-8.0 (m, 3H); 7.54 (t, 1H, 8Hz); 7.24 (s, 2H); 4.70 (m, 2H); 3.92-3.32 (m, 6H + 3H échangeables); 3.08 (m, 2H); 2.13 (m, 1H); 1.97 (m, 1H); 1.86 (m, 1H); 1.74 (m, 1 H); 1.38 (t, 3H, 7 Hz); 1.22 (t, 3H, 7.2Hz).

Les composés 1, 4, 5, 6, 7, 8, 10, 11, 12, 13 , 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 48 et 55 ont été synthétisés suivant la voie de synthèse décrite dans l'exemple 2. Le composé 9 suivant la voie de synthèses décrite dans l'exmple 1 et les composés 52 et 54 ont été synthétisés suivant la voie de synthèse décrite dans l'exemple 6.

Le tableau qui suit illustre les structures chimiques et les propriétés physiques de quelques exemples de composés selon l'invention.

Dans ce tableau :
- dans la colonne « sel » :
   - « - » représente un composé sous forme de base libre, alors que
   - « HCl » ou « TFA» signifie respectivement un composé sous forme de chlorhydrate ou de sel d'acide trifluoroacétique.
- Me, Et, représentent respectivement des groupes méthyle et éthyle. 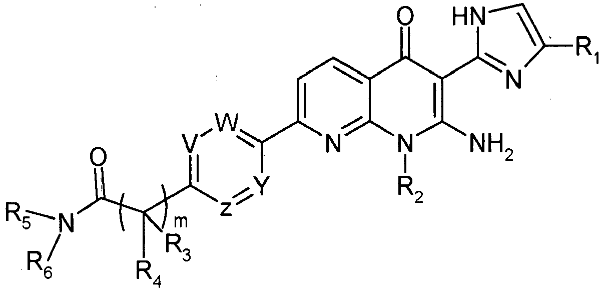
avec R6 représente -(CH₂)ₙ-L

| **N°** | **m** | **n** | **R1** | **R3** | **R4** | **R5** | **L** | **R2** | **V** | **W** | **Y** | **Z** | **Sel** | LCMS MH⁺ |
|---|---|---|---|---|---|---|---|---|---|---|---|---|---|---|
| 1 | 1 | 0 | H | H | H | H | 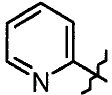 | Et | CH | CH | CH | CH | HCl | 466 |
| **2** | 1 | 0 | H | H | H | H | 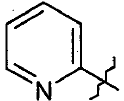 | Et | CH | CH | N | CH | HCl | 467.1 |
| **3** | 1 | 1 | H | H | H | H | 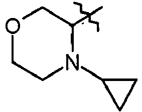 | Et | CH | CH | CH | CH | HCl | 528.2 |
| **4** | 1 | 1 | H | H | H | H | 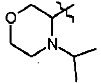 | Et | CH | CH | CH | CH | HCl | 530 |
| **5** | 1 | 0 | H | H | H | 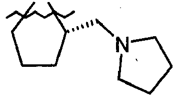 | | Et | CH | CH | CH | CH | TFA | 526.31 |
| **6** | 1 | 2 | H | H | H | H | 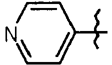 | Et | CH | CH | CH | CH | TFA | 494.24 |
| **7** | 1 | 2 | H | H | H | Me | 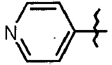 | Et | CH | CH | CH | CH | TFA | 508.25 |
| **8** | 1 | 0 | H | H | H | H | 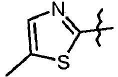 | Et | CH | CH | CH | CH | TFA | 486.18 |
| **9** | 1 | 2 | H | Me | H | H | 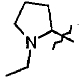 | Et | CH | CCH3 | CH | CH | - | 514.26 |
| **10** | 1 | 0 | H | H | H | H | 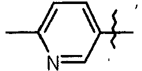 | Et | CH | CH | CH | CH | TFA | 480.23 |
| **11** | 1 | 1 | H | H | H | H | 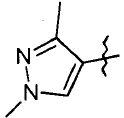 | Et | CH | CH | CH | CH | TFA | 497.24 |
| **12** | 1 | 1 | H | H | H | H | 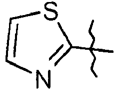 | Et | CH | CH | CH | CH | TFA | 486.25 |
| **13** | 1 | 1 | H | H | H | H | 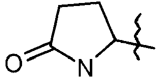 | Et | CH | CH | CH | CH | TFA | 486.24 |
| **14** | 1 | 0 | H | H | H | H | 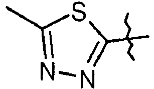 | Et | CH | CH | CH | CH | TFA | 487.19 |
| **15** | 1 | 0 | H | H | H | H | 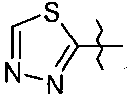 | Et | CH | CH | CH | CH | TFA | 473.19 |
| **16** | 1 | 0 | H | H | H | H | 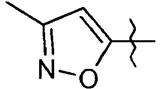 | Et | CH | CH | CH | CH | TFA | 470.22 |
| **17** | 1 | 2 | H | H | H | H | 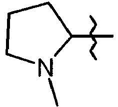 | Et | CH | CH | CH | CH | TFA | 500.3 |
| **18** | 1 | 0 | H | H | H | H | 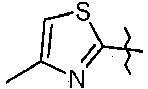 | Et | CH | CH | CH | CH | TFA | 486.25 |
| **19** | 1 | 1 | H | H | H | H | 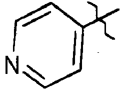 | Et | CH | CH | CH | CH | TFA | 480.21 |
| **20** | 1 | 0 | H | H | H | H | 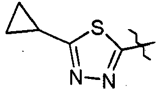 | Et | CH | CH | CH | CH | TFA | 513.2 |
| **21** | 1 | 2 | H | H | H | H | 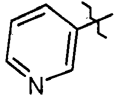 | Et | CH | CH | CH | CH | TFA | 494.28 |
| **22** | 1 | 1 | H | H | H | H | 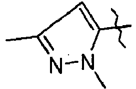 | Et | CH | CH | CH | CH | TFA | 497.25 |
| **23** | 1 | 1 | H | H | H | H | 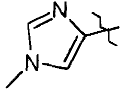 | Et | CH | CH | CH | CH | TFA | 483.25 |
| **24** | 1 | 0 | H | H | H | H | 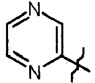 | Et | CH | CH | CH | CH | TFA | 467.22 |
| **25** | 1 | 2 | H | H | H | H | 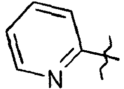 | Et | CH | CH | CH | CH | TFA | 494.26 |
| **26** | 1 | 0 | H | H | H | H | 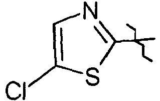 | Et | CH | CH | CH | CH | TFA | 506.13 |
| **27** | 1 | 0 | H | H | H | H | 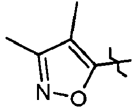 | Et | CH | CH | CH | CH | TFA | 484.27 |
| **28** | 1 | 0 | H | H | H | H | 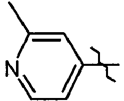 | Et | CH | CH | CH | CH | TFA | 480.24 |
| **29** | 1 | 1 | H | H | H | H | 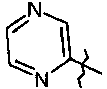 | Et | CH | CH | CH | CH | TFA | 481.25 |
| **30** | 1 | 0 | H | H | H | H | 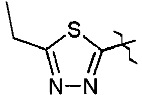 | Et | CH | CH | CH | CH | TFA | 501.21 |
| **31** | 1 | 1 | H | H | H | H | 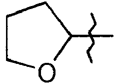 | Et | CH | CH | CH | CH | TFA | 473.27 |
| **32** | 1 | 0 | H | H | H | H | 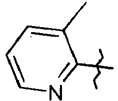 | Et | CH | CH | CH | CH | TFA | 480.24 |
| **33** | 1 | 0 | H | H | H | H | 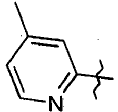 | Et | CH | CH | CH | CH | TFA | 480.23 |
| **34** | 1 | 0 | H | H | H | H | 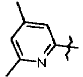 | Et | CH | CH | CH | CH | TFA | 494.25 |
| **35** | 1 | 0 | H | H | H | H | 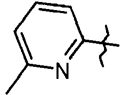 | Et | CH | CH | CH | CH | TFA | 480.26 |
| **36** | 1 | 1 | H | H | H | H | 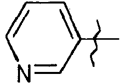 | Et | CH | CH | CH | CH | TFA | 480.24 |
| **37** | 1 | 0 | H | H | H | H | 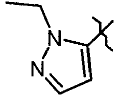 | Et | CH | CH | CH | CH | TFA | 483.23 |
| **38** | 1 | 0 | H | H | H | H | 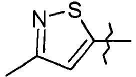 | Et | CH | CH | CH | CH | TFA | 486.2 |
| **39** | 1 | 0 | H | H | H | 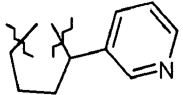 | | Et | CH | CH | CH | CH | TFA | 520.28 |
| **40** | 1 | 0 | H | H | H | H | 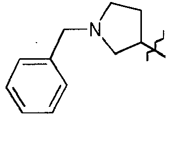 | Et | CH | CH | CH | CH | TFA | 548.29 |
| **41** | 1 | 1 | H | H | H | H | 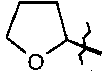 | Et | CH | CH | CH | CH | TFA | 473.22 |
| **42** | 1 | 0 | H | H | H | H | 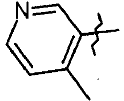 | Et | CH | CH | CH | CH | TFA | 480.23 |
| **43** | 1 | 0 | H | H | H | H | 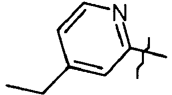 | Et | CH | CH | CH | CH | TFA | 494.26 |
| **44** | 1 | 1 | H | H | H | Me | 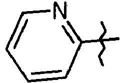 | Et | CH | CH | CH | CH | TFA | 508.26 |
| **45** | 1 | 0 | H | H | H | H | 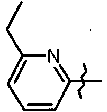 | Et | CH | CH | CH | CH | TFA | 494.25 |
| **46** | 1 | 1 | H | H | H | H | 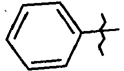 | Et | CH | CH | CH | CH | TFA | 479.15 |
| **47** | 1 | 1 | H | Me | H | H | 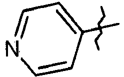 | Et | CH | CH | CH | CH | - | 494.23 |
| **48** | 0 | 1 | H | H | H | H | 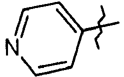 | Et | CH | CH | CH | CH | - | 466.0 |
| **49** | 3 | 1 | H | H | H | H | 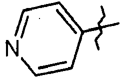 | Et | CH | CH | CH | CH | - | 508.0 |
| **50** | 1 | 1 | Me | H | H | H | 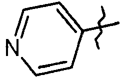 | Et | CH | CH | CH | CH | HCl | 494.0 |
| **51** | 1 | 1 | H | H | H | H | 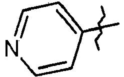 | 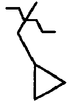 | CH | CH | CH | CH | TFA | 506 |
| **52** | 1 | 1 | H | H | H | H | 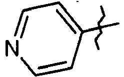 | 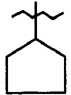 | CH | CH | CH | CH | TFA | 520 |
| **53** | 1 | 1 | H | H | H | H | 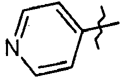 | Et | CH | CH | CH | N | TFA | 481 |
| **54** | 1 | 1 | H | H | H | H | 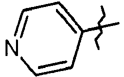 | 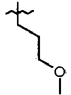 | CH | CH | CH | CH | - | 524 |
| **55** | 1 | 3 | H | H | H | H | 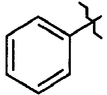 | Et | CH | CH | CH | CH | HCl | 507 |
| **56** | 1 | 2 | H | Me | H | H | 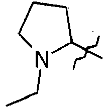 | Et | CF | CH | CH | CH | - | 518.3 |

Les composés selon l'invention ont fait l'objet d'essais pharmacologiques permettant de déterminer leur effet inhibiteur de protéine à activité tyrosine kinases.

A titre d'exemple, leurs effets inhibiteurs de l'activité tyrosine kinase de PDGF-R et/ou Flt-3, ont été mesurés in vitro dans des modèles cellulaires.

L'activité inhibitrice vis à vis des récepteurs PDGF ou Flt-3 est donnée par la concentration qui inhibe 50% de l'activité de prolifération respectivement des cellules Baf3 tel/PDGF ou MV4-11.

### Mesure de l'inhibition de l'activité tyrosine kinase du récepteur au PDGF beta (PDGF-Rβ) (Baf-3 tel/PDGFRβ) :

Ce test consiste à évaluer les effets des composés sur l'activité tyrosine kinase du récepteur PDGF beta.

L'effet d'inhibition des composés selon l'invention envers l'activité tyrosine kinase du récepteur PDGF-R a été évalué sur la lignée cellulaire murine hématopoïétique BaF/3 transfectée avec un plasmide codant pour la protéine de fusion Tel/PDGF-R beta. Cette protéine de fusion est retrouvée dans les leucémies chroniques myéloïdes myélomonocytaires (CMML). Elle comprend la partie N-Terminale du facteur de transcription Tel et la partie transmembranaire et intracellulaire du récepteur PDGF-R beta. Cette protéine de fusion est présente sous forme dimérisée (présence d'un domaine d'oligomérisation dans la partie N-Terminale de Tel) et de ce fait conduit à l'activité constitutive du domaine kinase de PDGF-R beta. Cette lignée BaF3 Tel/PDGF a été décrite dans la littérature à plusieurs reprises et notamment de façon détaillée dans l'article de M CARROLL et coll, PNAS,1996, 93, 14845-14850, M CARROLL et coll.,Blood 2002, 99, 14845-14850.

Les cellules BaF3 Tel/PDGF sont lavées par du tampon phosphate et ensemencées dans des plaques de 96 puits, à la densité de 5.10⁴ cellules/ml (100 ml par puits), dans du RPMI 1640 contenant 10% de SVF, en présence ou en absence des composés à tester. Après 72 h d'incubation, les cellules viables sont quantifiées par mesure de l'ATP cellulaire grâce à l'utilisation du kit CellTiter-Glo^{®} (Promega, Cat G7571). Les cellules sont traitées selon les instructions données par le fournisseur du kit et la luminescence est mesurée à l'aide d'un Luminoskan (Ascent, Labsystem) avec les paramètres suivants : mesure : unique ; temps d'intégration : 1000 ms, lag time : 5 s.

Il apparaît ainsi que les composés selon l'invention ont une activité inhibitrice sur l'activité tyrosine kinase de PDGF-R beta. Cette activité est donnée par la concentration qui inhibe 50% de la prolifération des cellules Baf3 tel/PDGF (CI₅₀). Les CI₅₀ des composés selon l'invention sont inférieures à 1,0 µM.

Par exemple, les composés n° 2, 8, 9, 10, 14, 20, 27, 43, 50, 51, 52, 55 ont montré une CI₅₀ de respectivement 7.9, 2, 9.8, 2.5, 2.4, 8, 2, 1.4, 6.6, 8.9, 13.6, 5.9 nM dans le test de mesure de l'activité tyrosine kinase du récepteur PDGF.

Outre leurs propriétés d'inhibition du PDGF-R tyrosine kinase, il apparaît également que des composés conformes à l'invention présentent des propriétés d'inhibition de l'activité tyrosine kinase du récepteur Flt-3, comme décrit ci-après.

### Mesure de l'inhibition de l'activité tyrosine kinase du récepteur Flt-3

L'effet d'inhibition des composés selon l'invention envers l'activité tyrosine kinase du récepteur Flt-3 a été évalué sur la lignée cellulaire MV4-11, lignée établie à partir d'une leucémie de type AML et porteuse d'un mutant Flt3ITD, actif de façon constitutive. L'activité inhibitrice est corrélée à l'inhibition de la croissance des cellules, selon les protocoles décrits par SPIEKERMANN, K. et coll, Blood, 2003, 101, (4) 1494-1504 et O'FARRELL, A.-M. et coll., Blood, 2003, 101, (9) 3597-3605.

Les cellules MV4-11 sont lavées par du tampon PBS et ensemencées dans des plaques de 96 puits, à la densité de 1.10⁵ cellules/ml (100 µl par puits), dans du RPMI 1640 contenant 10% de SVF, en présence ou en absence des composés à tester. Après 72 h d'incubation, les cellules viables sont quantifiées par mesure de l'ATP cellulaire grâce à l'utilisation du kit CellTiter-Glo^{®} (Promega, Cat G7571). Les cellules sont traitées selon les instructions données par le fournisseur du kit et la luminescence est mesurée à l'aide d'un Luminoskan (Ascent, Labsystem) avec les paramètres suivants : mesure : unique ; temps d'intégration : 1000 ms, lag time : 5 s.

L'activité inhibitrice vis à vis du récepteur Flt-3 est donnée par la concentration qui inhibe 50% de la prolifération des cellules MV4-11. Il apparaît ainsi que des composés selon l'invention ont une activité inhibitrice sur l'activité tyrosine kinase du récepteur Flt-3 avec des CI₅₀ inférieures à 1,0 µM.

Par exemple les composés n° 8, 20, 26, 27, 32, 42, 43, 50 ont montré une CI₅₀ de respectivement 52, 26, 95, 30, 69, 84, 19, 115 nM, dans le test de mesure de l'activité tyrosine kinase du récepteur Flt-3.

Les composés selon l'invention sont donc des inhibiteurs de protéines kinases, notamment des récepteurs tyrosine kinase PDGF alpha et beta et, pour certains d'entre eux, également du récepteur Flt-3 tyrosine kinase.

Ainsi, selon un des objets de la présente invention, les composés de formule (I) présentent une activité très intéressante d'inhibition de la phosphorylation du domaine kinase du récepteur PDGF-R beta dans des cellules BaF3 Tel/PDGF, induite par l'activité inhibitrice du produit présent dans le plasma des animaux traités.

Les composés selon l'invention sont donc des inhibiteurs de protéines kinases, notamment des récepteurs tyrosine kinase PDGF alpha et beta et, pour certains d'entre eux, également du récepteur Flt-3 tyrosine kinase.

Les composés selon l'invention peuvent donc être utilisés pour la préparation de médicaments, en particulier de médicaments inhibiteurs de protéine kinases.

Il s'agit de médicaments inhibiteurs de protéine kinase, notamment de médicaments inhibiteurs du recepteur PDGF-R tyrosine kinase et éventuellement du récepteur Flt-3 tyrosine kinases.

Ainsi, selon un autre de ses aspects, l'invention a pour objet des médicaments qui comprennent un composé de formule (I), ou un sel d'addition de ce dernier à un acide pharmaceutiquement acceptable, ou encore un solvat du composé de formule (I).

Ces médicaments trouvent leur emploi en thérapeutique, notamment dans le traitement des maladies liées à l'activité des protéines kinases et notamment des maladies prolifératives telles que les cancers à tumeurs liquides, les leucémies chroniques ou aiguës, les lymphomes lymphocytaires, la maladie de Hodgkin, les syndromes myeloproliferatifs et les syndromes myelodysplasiques.

Ces médicaments trouvent aussi leur emploi en thérapeutique dans le traitement des maladies prolifératives telles que les cancers à tumeurs solides comprenant les cancers du poumon (NSCLC), des os, du pancréas, de la peau, le syndrome de Kaposi, les mélanomes intraoculaires, les cancers liés aux organes sexuels comprenant le cancer du sein, de l'utérus, du col de l'utérus, des ovaires, de l'endomètre, du vagin, de la vulve, de l'urètre, du pénis, de la prostate, les carcinomes des trompes de Fallope, les cancers de type Tumeurs Stromales Gastro-Intestinales (abrégé GIST) comprenant les cancers de la région anale, du rectum, de l'intestin grêle, du colon, de l'estomac, de l'oesophage, les cancers des glandes endocrines, thyroïdes, parathyroïdes ou surrénales, les sarcomes des tissus mous, les sarcomes d'Ewing, les ostésarcomes, les dermatofibrosarcomes et autres fibrosarcomes, les cancers de la vessie ou du rein, les néoplasmes du système nerveux central, les tumeurs de la colonne vertébrale et desmoïdes, les gliomes du tronc cérébral et glioblastomes, les adénomes pituitaires et leurs métastases.

Un autre aspect de l'invention comprend une association entre au moins un composé selon l'invention et au moins un agent de chimiothérapie.

En effet, les composés de la présente invention peuvent être utilisés seuls ou en mélange avec au moins un agent de chimiothérapie pouvant être choisi parmi des agents cytotoxiques et/ou des antiangiogènes. Par exemple, les agents antiangiogènes peuvent être un composé inhibiteur de l'activité kinase de VEGF-R ou un composé antagoniste d'un facteur de croissance.

Il est également possible de combiner les composés selon l'invention avec un traitement par radiations.

Les combinaisons des composés de l'invention avec les agents de chimiothérapie cités ci-dessus et/ou les radiations sont un autre objet de la présente invention.

Les agents de chimiothérapie cités ci-dessus et/ou les radiations peuvent être administrés simultanément, séparément ou séquentiellement. Le traitement sera adapté par le praticien en fonction du malade à traiter.

Ces médicaments trouvent également leur emploi en thérapeutique, dans le traitement de maladies prolifératives non-malignes telles que la resténose, l'athérosclérose, la thrombose, l'insuffisance cardiaque, l'hypertrophie cardiaque, hypertension artérielle pulmonaire, la fibrose, la néphropathie diabétique, la glomérulonéphrite, la pyélonéphrite chronique, les hemangiomes, les maladies auto-immunes telles que le psoriasis, les sclérodermatites, l'immunosuppression (rejet de greffes par exemple), les pathologies liées à l'oeil telles que la fibrose post-opératoire ou la dégénérescence maculaire liée à l'âge.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable de ce dernier, ou encore un un solvat dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intra-veineuse, topique, intratrachéale, intranasale, transdermique ou rectale, le principe actif de formule (I) ci-dessus, ou son sel ou solvat éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |

| | |
|---|---|
| Hydroxypropyl-méthylcellulose | 2,25 mg |
| Stéarate de magnésium | 3,0 mg |

La présente invention, selon un autre de ses aspects, concerne également un composé selon l'invention ou un de ses sels pharmaceutiquement acceptables ou solvats pour être utilisés dans une méthode de traitement des pathologies ci-dessus indiquées qui comprend l'administration, à un patient, d'une dose efficace dudit composé.

## Revendications

1. Composé répondant à la formule (I) : 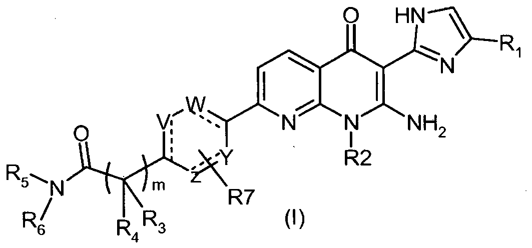 dans laquelle,
**R1** représente un atome d'hydrogène ou un groupe (C1-C4)alkyle ;
**R2** représente un groupement -(CH₂)_{n'}-B où :
• n'=0, 1, 2, 3 ou 4 ; et
• B représente (i) un groupe (C3-C5)cycloalkyle ou un groupe (C1-C4)alkyle, ledit groupe étant éventuellement substitué par un ou plusieurs atomes de fluor, ou (ii) un groupe (C1-C4)alcoxy ;
**Y, Z, V** et **W** représentent, indépendamment les uns des autres :
• un groupe -CH-,
• un atome de carbone éventuellement substitué par un groupe R7, ledit groupe R7 représentant un groupe (C1-C4)alkyle ou un atome d'halogène,
• un hétéroatome tel qu'un atome d'azote, un atome de soufre ou un atome d'oxygène, ou
• pas d'atome,
étant entendu que le cycle, dans lequel V, W, Y et Z sont compris, est un cycle comprenant 5 ou 6 chaînons, étant entendu que les pointillés dans ledit cycle indique que le cycle résultant est un cycle aromatique et étant entendu que ledit cycle comprend 0, 1 ou 2 hétéroatomes ;
**R3** et **R4** représentent, indépendamment l'un de l'autre, des groupes identiques ou différents, R3 et R4 étant choisis parmi :
• un atome d'hydrogène ; et
• un groupe (C1-C4)alkyle linéaire ;
ou **R3** et **R4** forment ensemble avec le carbone auxquels ils sont liés un groupe (C3-C5)cycloalkyle ;
m est un nombre entier égal à 1, 2, 3 ou 4 ;
**R5** représente un atome d'hydrogène ou un groupe (C1-C4)alkyle ;
**R6** représente un groupement -(CH₂)ₙ-L dans lequel :
• n= 0, 1, 2 ou 3, et
• L est un groupe sélectionné parmi les groupes suivants :
o un aryle comprenant 6 atomes de carbone ;
o un hétéroaryle comprenant entre 5 et 6 chaînons et comprenant au moins un hétéroatome choisi parmi l'azote, l'oxygène et le soufre ;
o un hétérocycle saturé comprenant 5, 6 ou 7 chaînons et comprenant au moins un hétéroatome choisi parmi l'azote et l'oxygène, ledit hétérocycle étant éventuellement un lactame ;
ledit groupe aryle, hétéroaryle ou hétérocycle étant éventuellement substitué par au moins un substituant choisi parmi (i) les groupes (C1-C4)alkyle, linéaires ou ramifiés, (ii) les groupes (C3-C5)cycloalkyle, (iii) les atomes d'halogène, (iv) les aryles et (v) le benzyle ;
étant entendu que, lorsque **L** est un hétéroaryle ou un hétérocycle, ledit hétéroaryle ou hétérocycle comprenant au moins un atome d'azote, ce dernier peut éventuellement être substitué par ledit substituant ;
ou **R5** et **R6** forment ensemble avec l'atome d'azote auxquels ils sont liés un groupe hétérocycle, éventuellement substitué par au moins
• un hétéroaryle, ou
• un groupe (C1-C3)alkyle, lui-même pouvant être substitué par un heterocycle comprenant 5 ou 6 atomes et comprenant au moins un hétéroatome choisi parmi l'azote et l'oxygène, étant entendu que lorsqu'il s'agit d'un hétérocycle comprenant au moins un atome d'azote, ce dernier peut éventuellement être substitué ;
ledit composé de formule (I), ses énantiomères et diastéréoisomères, y compris leurs mélanges, étant à l'état de base ou de sel d'addition à un acide et/ou à l'état de solvat.

2. Composé selon la revendication précédente, **caractérisé en ce que R6** représente un groupement -(CH₂)ₙ-L dans lequel :
• n= 0, 1, 2 ou 3, et
• **L** est un groupe sélectionné parmi les groupes suivants :
o un hétéroaryle comprenant 5 chaînons et comprenant (i) 2 hétéroatomes choisis, indépendamment l'un de l'autre, parmi l'azote, l'oxygène et le soufre, ou (ii) 3 hétéroatomes choisis, indépendamment l'un de l'autre, parmi l'azote et le soufre,
o un hétéroaryle comprenant 6 chainons et comprenant 1 ou 2 hétéroatome(s),
o un hétérocycle comprenant 5 chaînons et comprenant un hétéroatome choisi parmi l'azote et l'oxygène, ledit hétérocycle étant éventuellement un lactame, et
o un hétérocycle comprenant 6 chaînons et comprenant 2 hétéroatomes choisis parmi l'azote et l'oxygène,
ledit groupe hétéroaryle ou hétérocycle étant éventuellement substitué par au moins un substituant choisi parmi (i) les groupes (C1-C4)alkyle, linéaires ou ramifiés, (ii) les groupes (C3-C5)cycloalkyle, (iii) les atomes d'halogène, (iv) les aryles et (v) le benzyle,
étant entendu que, lorsque **L** est un hétéroaryle ou un hétérocycle, ledit hétéroaryle ou hétérocycle comprenant au moins un atome d'azote, ce dernier peut éventuellement être substitué par ledit substituant.

3. Composé selon l'une quelconque des revendications précédentes, **caractérisé en ce que L** est **:**
• un hétéroaryle comprenant 6 chaînons choisi parmi la pyridine, la pyrazine, pyridazine, pyrimidine;
• un aryle tel que le phényle, ou
• un hétéroaryle comprenant 5 chainons choisi parmi le thiazole, l'imidazole, le pyrazole, l'isoxazole et le 1, 3, 4 thiadiazole, ou
• un hétérocycle saturé comprenant 5 chaînons choisi parmi la pyrrolidine, le tetrahydrofurane et le 2-oxo-pyrrolidine ou
• un hétérocycle saturé comprenant 6 chaînons choisi parmi la morpholine, la pipérazine, pipéridine,
ledit groupe aryle, hétéroaryle ou hétérocycle étant éventuellement substitué par au moins un substituant choisi parmi (i) les groupes (C1-C4)alkyle, linéaires ou ramifiés, (ii) les groupes (C3-C5)cycloalkyle et (iii) les aryles,
étant entendu que, lorsque **L** est un hétéroaryle ou un hétérocycle, ledit hétéroaryle ou hétérocycle comprenant au moins un atome d'azote, ce dernier peut éventuellement être substitué par ledit substituant.

4. Composé selon l'une quelconque des revendications précédentes, **caractérisé en ce que L** est choisi parmi :
• la pyridine, éventuellement substitué par au moins un groupe (C1-C4)alkyle, linéaire ou ramifié,
• la morpholine, éventuellement substitué par au moins (i) un groupe (C3-C5)cycloalkyle ou (ii) un groupe (C1-C4)alkyle, linéaire ou ramifié,
• une pyrrolidine, éventuellement substitué par au moins (i) un groupe (C1-C4)alkyle, linéaire ou ramifié, ou (ii) un benzyle,
• un thiazole, éventuellement substitué par au moins (i) un groupe (C1-C4)alkyle, linéaire ou ramifié, ou (ii) un atome de chlore,
• un imidazole, éventuellement substitué par au moins un groupe (C1-C4)alkyle, linéaire ou ramifié,
• une 2-oxo-pyrrolidine,
• un 1, 3, 4-thiadiazole, éventuellement substitué par au moins (i) un groupe (C1-C4)alkyle, linéaire ou ramifié, ou (ii) un groupe (C3-C5)cycloalkyle,
• un isoxazole, éventuellement substitué par au moins un groupe (C1-C4)alkyle, linéaire ou ramifié,
• un pyrazole, éventuellement substitué par au moins un groupe (C1-C4)alkyle, linéaire ou ramifié,
• une pyrazine,
• un isothiazole, éventuellement substitué par au moins un groupe (C1-C4)alkyle, linéaire ou ramifié,
• un phényle,
• un tetrahydrofuranne,
étant entendu que, lorsque **L** est un hétéroaryle ou un hétérocycle, ledit hétéroaryle ou hétérocycle comprenant au moins un atome d'azote, ce dernier peut éventuellement être substitué.

5. Composé selon l'une quelconque des revendications précédentes, **caractérisé en ce que R5** représente un atome d'hydrogène ou un méthyle.

6. Composé selon l'une quelconque des revendications précédentes, **caractérisé en ce que** :
• m est égal à 1 ou 3, et/ou
• **R3** et **R4** représentent, indépendamment l'un de l'autre, des groupes identiques ou différents, R3 et R4 étant choisis parmi :
oUn atome d'hydrogène, et
oUn méthyle.

7. Composé selon l'une quelconque des revendications précédentes, **caractérisé en ce que Y, Z, V et W** représentent, indépendamment les uns des autres, :
• un groupe -CH- ;
• un atome de carbone substitué par un groupe R7, ledit groupe R7 représentant un groupe (C1-C4)alkyle ou un atome de fluor ; ou
• un hétéroatome tel qu'un atome d'azote, un atome de soufre ou un atome d'oxygène, avantageusement un atome d'azote.

8. Composé selon l'une quelconque des revendications précédentes, **caractérisé en ce que R1** représente un atome d'hydrogène ou un méthyle.

9. Composé selon l'une quelconque des revendications précédentes, **caractérisé en ce que R2** représente un groupement -(CH₂)ₙ-B où :
o n'=0,1 ou 3 ; et/ou
o B représente (i) un groupe (C3-C5)cycloalkyle, (ii) un groupe (C1-C4)alkyle ou (iii) un groupe (C1-C4)alcoxy.

10. Composé selon l'une quelconque des revendications 1 et 6 à 9, caractérisé en ce **R5** et **R6** forment ensemble avec l'atome d'azote auxquels ils sont liés un groupe hétérocycle, éventuellement substitué par au moins
• un hétéroaryle, avantageusement une pyridine ; ou
• un groupe (C1-C3)alkyle, lui-même pouvant être substitué par un heterocycle comprenant 5' ou 6 atomes et comprenant au moins un hétéroatome choisi parmi l'azote et l'oxygène, avantageusement il s'agit d'un groupe C1alkyle, lui-même substitué par un hétérocycle comprenant 5 atomes dont un atome d'azote.

11. Composé selon l'une quelconque des revendications précédentes, **caractérisé en ce qu'**il se présente à l'état de base ou de sel d'addition à un acide tel que l'acide chlorhydrique ou l'acide trifluoroacétique.

12. Composé selon l'une quelconque des revendications précédentes, **caractérisé en ce qu'**il s'agit du :
2-{4-[7-Amino-8-ethyl-6-(1H-imidazol-2-yl)5-oxo-5,8-dihydro-[1,8]naphthyridin-2-yl]-phenyl}-N-pyridin-2-yl-acetamide (composé 1) ;
2-{6-[7-Amino-8-ethyl-6-(1H-imidazol-2-yl)-5-oxo-5,8-dihydro-[1,8]naphthyridin-2-yl]-pyridin-3-yl}-N-pyridin-2-yl-acetamide (composé 2) ;
2-(4-[7-Amino-8-ethyl-6-(1H-imidazol-2-yl)-5-oxo-5,8-dihydro-[1,8]naphthyridin-2-yl]-phenyl}-N-(4-cyclopropyl-morpholin-3-ylmethyl)-acetamide (composé 3) ;
2-{4-[7-Amino-8-ethyl-6-(1H-imidazol-2-yl)-5-oxo-5,8-dihydro-[1,8]naphthyridin-2-yl]-phenyl}-N-(4-isopropyl-morpholin-3-ylmethyl)-acetamide (composé 4) ;
2-Amino-1-ethyl-3-(1H-imidazol-2-yl)-7-{4-[2-oxo-2-((S)-2-pyrrolidin-1-ylmethyl-pyrrolidin-1-yl)-ethyl]-phenyl}-1H-[1,8]naphthyridin-4-one (composé 5) ;
2-{4-[7-Amino-8-ethyl-6-(1H-imidazol-2-yl)-5-oxo-5,8-dihydro-[1,8]naphthyridin-2-yl]-phenyl}-N-(2-pyridin-4-yl-ethyl)-acetamide (composé 6) ;
2-{4-[7-Amino-8-ethyl-6-(1H-imidazol-2-yl)-5-oxo-5,8-dihydro-[1,8]naphthyridin-2-yl]-phenyl}-N-methyl-N-(2-pyridin-4-yl-ethyl)-acetamide (composé 7) ;
2-{4-[7-Amino-8-ethyl-6-(1H-imidazol-2-yl)-5-oxo-5,8-dihydro-[1,8]naphthyridin-2-yl]-phenyl}-N-(5-methyl-thiazol-2-yl)-acetamide (composé 8) ;
2-{4-[7-Amino-8-ethyl-6-(1H-imidazol-2-yl)-5-oxo-5,8-dihydro-[1,8]naphthyridin-2-yl]-3-methyl-phenyl}-N-(1-ethyl-pyrrolidin-2-ylmethyl)-acetamide (composé 9) ;
2-{4-[7-Amino-8-ethyl-6-(1H-imidazol-2-yl)-5-oxo-5,8-dihydro-[1,8]naphthyridin-2-yl]-phenyl}-N-(6-methyl-pyridin-3-yl)-acetamide (composé 10) ;
2-{4-[7-Amino-8-ethyl-6-(1H-imidazol-2-yl)-5-oxo-5,8-dihydro-[1,8]naphthyridin-2-yl]-phenyl}-N-(1,3-dimethyl-1H-pyrazol-4-ylmethyl)-acetamide (composé 11) ;
2-{4-[7-Amino-8-ethyl-6-(1H-imidazol-2-yl)-5-oxo-5,8-dihydro-[1,8]naphthyridin-2-yl]-phenyl}-N-thiazol-2-ylmethyl-acetamide (composé 12) ;
2-f4-[7-Amino-8-ethyl-6-(1H-imidazol-2-yl)-5-oxo-5,8-dihydro-[1,8]naphthyridin-2-yl]-phenyl}-N-(5-oxo-pyrrolidin-2-ylmethyl)-acetamide (composé 13) ;
2-{4-[7-Amino-8-ethyl-6-(1H-imidazol-2-yl)-5-oxo-5,8-dihydro-[1,8]naphthyridin-2-yl]-phenyl}-N-(5-methyl-[1,3,4]thiadiazol-2-yl)-acetamide (composé 14) ;
2-{4-[7-Amino-8-ethyl-6-(1H-imidazol-2-yl)5-oxo-5,8-dihydro-[1,8]naphthyridin-2-yl]-phenyl}-N-[1,3,4]thiadiazol-2-yl-acetamide (composé 15) ;
2-{4-[7-Amino-8-ethyl-6-(1H-imidazol-2-yl)-5-oxo-5,8-dihydro-[1,8]naphthyridin-2-yl]-phenyl}-N-(3-methyl-isoxazol-5-yl)-acetamide (composé 16) ;
2-{4-[7-Amino-8-ethyl-6-(1H-imidazol-2-yl)-5-oxo-5,8-dihydro-[1,8]naphthyridin-2-yl]-phenyl}-N-[2-(1-methyl-pyrrolidin-2-yl)-ethyl]-acetamide (composé 17) ;
2-{4-[7-Amino-8-ethyl-6-(1H-imidazol-2-yl)-5-oxo-5,8-dihydro-[1,8]naphthyridin-2-yl]-phenyl}-N-(4-methyl-thiazol-2-yl)-acetamide (composé 18) ;
2-{4-[7-Amino-8-ethyl-6-(1H-imidazol-2-yl)-5-oxo-5,8-dihydro-[1,8]naphthyridin-2-yl]-phenyl}-N-pyridin-4-ylmethyl-acetamide (composé 19) ;
2-{4-[7-Amino-8-ethyl-6-(1H-imidazol-2-yl)-5-oxo-5,8-dihydro-[1,8]naphthyridin-2-yl]-phenyl}-N-(5-cyclopropyl-[1,3,4]thiadiazol-2-yl)-acetamide (composé 20) ;
2-{4-[7-Amino-8-ethyl-6-(1H-imidazôl-2-yl)-5-oxo-5,8-dihydro-[1,8]naphthyridin-2-yl]-phenyl}-N-(2-pyridin-3-yl-ethyl)-acetamide (composé 21) ;
2-{4-[7-Amino-8-ethyl-6-(1H-imidazol-2-yl)-5-oxo-5,8-dihydro-[1,8]naphthyridin-2-yl]-phenyl}-N-(2,5-dimethyl-2H-pyrazol-3-ylmethyl)-acetamide (composé 22) ;
2-{4-[7-Amino-8-ethyl-6-(1H-imidazol-2-yl)-5-oxo-5,8-dihydro-[1,8]naphthyridin-2-yl]-phenyl}-N-(1-methyl-1H-imidazol-4-ylmethyl)-acetamide (composé 23) ;
2-{4-[7-Amino-8-ethyl-6-(1H-imidazol-2-yl)-5-oxo-5,8-dihydro-[1,8]naphthyridin-2-yl]-phenyl}-N-pyrazin-2-yl-acetamide (composé 24) ;
2-{4-[7-Amino-8-ethyl-6-(1H-imidazol-2-yl)-5-oxo-5,8-dihydro-[1,8]naphthyridin-2-yl]-phenyl}-N-(2-pyridin-2-yl-ethyl)-acetamide (composé 25) ;
2-{4-[7-Amino-8-ethyl-6-(1H-imidazol-2-yl)-5-oxo-5,8-dihydro-[1,8]naphthyridin-2-yl]-phenyl}-N-(5-chloro-thiazol-2-yl)-acetamide (composé 26) ;
2-{4-[7-Amino-8-ethyl-6-(1H-imidazol-2-yl)-5-oxo-5,8-dihydro-[1,8]naphthyridin-2-yl]-phenyl}-N-(3,4-dimethyl-isoxazol-5-yl)-acetamide (composé 27) ;
2-{4-[7-Amino-8-ethyl-6-(1H-imidazol-2-yl)-5-oxo-5,8-dihydro-[1,8]naphthyridin-2-yl]-phenyl}-N-(2-methyl-pyridin-4-yl)-acetamide (composé 28) ;
2-{4-[7-Amino-8-ethyl-6-(1H-imidazol-2-yl)-5-oxo-5,8-dihydro-[1,8]naphthyridin-2-yl]-phenyl}-N-pyrazin-2-ylmethyl-acetamide (composé 29) ;
2-{4-[7-Amino-8-ethyl-6-(1H-imidazol-2-yl)-5-oxo-5,8-dihydro-[1,8]naphthyridin-2-yl]-phenyl}-N-(5-ethyl-[1,3,4]thiadiazol-2-yl)-acetamide (composé 30) ;
2-{4-[7-Amino-8-ethyl-6-(1H-imidazol-2-yl)-5-oxo-5,8-dihydro-[1,8]naphthyridin-2-yl]-phenyl}-N-(tetrahydro-furan-2-ylmethyl)-acetamide (composé 31) ;
2-{4-[7-Amino-8-ethyl-6-(1H-imidazol-2-yl)-5-oxo-5,8-dihydro-[1,8]naphthyridin-2-yl]-phenyl}-N-(3-methyl-pyridin-2-yl)-acetamide (composé 32) ;
2-{4-[7-Amino-8-ethyl-6-(1H-imidazol-2-yl)-5-oxo-5,8-dihydro-[1,8]naphthyridin-2-yl]-phenyl}-N-(4-methyl-pyridin-2-yl)-acetamide (composé 33) ;
2-{4-[7-Amino-8-ethyl-6-(1H-imidazol-2-yl)-5-oxo-5,8-dihydro-[1,8]naphthyridin-2-yl]-phenyl}-N-(4,6-dimethyl-pyridin-2-yl)-acetamide (composé 34) ;
2-{4-[7-Amino-8-ethyl-6-(1H-imidazol-2-yl)-5-oxo-5,8-dihydro-[1,8]naphthyridin-2-yl]-phenyl}-N-(6-methyl-pyridin-2-yl)-acetamide (composé 35) ;
2-{4-[7-Amino-8-ethyl-6-(H-imidazol-2-yl)-5-oxo-5,8-dihydro-[1,8]naphthyridin-2-yl]-phenyl}-N-pyridin-3-ylmethyl-acetamide (composé 36) ;
2-{4-[7-Amino-8-ethyl-6-(1H-imidazol-2-yl)-5-oxo-5,8-dihydro-[1,8]naphthyridin-2-yl]-phenyl}-N-(2-ethyl-2H-pyrazol-3-yl)-acetamide (composé 37) ;
2-{4-[7-Amino-8-ethyl-6-(1H-imidazol-2-yl)-5-oxo-5,8-dihydro-[1,8]naphthyridin-2-yl]-phenyl}-N-(3-methyl-isothiazol-5-yl)-acetamide (composé 38) ;
2-Amino-1-ethyl-3-(1H-imidazol-2-yl)-7-{4-[2-oxo-2-(2-pyridin-3-yl-pyrrolidin-1-yl)-ethyl]-phenyl}-1H-[1,8]naphthyridin-4-one (composé 39) ;
2-{4-[7-Amino-8-ethyl-6-(1H-imidazol-2-yl)-5-oxo-5,8-dihydro-[1,8]naphthyridin-2-yl]-phenyl}-N-(1-benzyl-pyrrolidin-3-yl)-acetamide (composé 40) ;
2-{4-[7-Amino-8-ethyl-6-(1H-imidazol-2-yl)-5-oxo-5,8-dihydro-[1,8]naphthyridin-2-yl]-phenyl}-N-[(S)-1-(tetrahydro-furan-2-yl)methyl]-acetamide (composé 41) ;
2-{4-[7-Amino-8-ethyl-6-(1H-imidazol-2-yl)-5-oxo-5,8-dihydro-[1,8]naphthyridin-2-yl]-phenyl}-N-(4-methyl-pyridin-3-yl)-acetamide (composé 42) ;
2-{4-[7-Amino-8-ethyl-6-(1H-imidazol-2-yl)-5-oxo-5,8-dihydro-[1,8]naphthyridin-2-yl]-phenyl}-N-(4-ethyl-pyridin-2-yl)-acetamide (composé 43) ;
2-{4-[7-Amino-8-ethyl-6-(1H-imidazol-2-yl)-5-oxo-5,8-dihydro-[1,8]naphthyridin-2-yl]-phenyl}-N-ethyl-N-pyridin-4-ylmethyl-acetamide (composé 44) ;
2-{4-[7-Amino-8-ethyl-6-(1H-imidazol-2-yl)-5-oxo-5,8-dihydro-[1,8]naphthyridin-2-yl]-phenyl}-N-(6-ethyl-pyridin-2-yl)-acetamide (composé 45) ;
2-{4-[7-Amino-8-ethyl-6-(1H-imidazol-2-yl)-5-oxo-5,8-dihydro-[1,8]naphthyridin-2-yl]-phenyl}-N-benzyl-acetamide (composé 46) ;
2-{4-[7-Amino-8-ethyl-6-(1H-imidazol-2-yl)-5-oxo-5,8-dihydro-[1,8]naphthyridin-2-yl]-phenyl}-N-pyridin-4-ylmethyl-propionamide (composé 47) ;
4-[7-Amino-8-ethyl-6-(1H-imidazol-2-yl)-5-oxo-5,8-dihydro-[1,8]naphthyridin-2-yl]-N-pyridin-4-ylmethyl-benzamide (composé 48) ;
4-{4-[7-Amino-8-ethyl-6-(1H-imidazol-2-yl)-5-oxo-5,8-dihydro-[1,8]naphthyridin-2-yl]-phenyl}-N-pyridin-4-ylmethyl-butyramide (composé 49) ;
2-{4-[7-Amino-8-ethyl-6-(4-methyl-1H-imidazol-2-yl)-5-oxo-5,8-dihydro-[1,8]naphthyridin-2-yl]-phenyl}-N-pyridin-4-ylmethyl-acetamide (composé 50) ;
2-{4-[7-Amino-8-cyclopropylmethyl-6-(1H-imidazol-2-yl)-5-oxo-5,8-dihydro-[1,8]naphthyridin-2-yl]-phenyl}-N-pyridin-4-ylmethyl-acetamide (composé 51) ;
2-{4-[7-Amino-8-cyclopentyl-6-(1H-imidazol-2-yl)-5-oxo-5,8-dihydro-[1,8]naphthyridin-2-yl]-phenyl}-N-pyridin-4-ylmethyl-acetamide (composé 52) ;
2-{5-[7-Amino-8-ethyl-6-(1H-imidazol-2-yl)-5-oxo-5,8-dihydro-[1,8]naphthyridin-2-yl]-pyridin-2-yl}-N-pyridin-4-ylmethyl-acetamide (composé 53) ;
2-{4-[7-Amino-6-(1H-imidazol-2-yl)-8-(3-methoxy-propyl)-5-oxo-5,8-dihydro-[1,8]naphthyridin-2-yl]-phenyl}-N-pyridin-4-ylmethyl-acetamide (composé 54) ;
2-{4-[7-Amino-8-ethyl-6-(1H-imidazol-2-yl)-5-oxo-5,8-dihydro-[1,8]naphthyridin-2-yl]-phenyl}-N-(3-phenyl-propyl)-acetamide (composé 55) ;
2-{4-[7-Amino-8-ethyl-6-(1H-imidazol-2-yl)-5-oxo-5,8-dihydro-[1,8]naphthyridin-2-yl]-2 -fluoro-phenyl}-N-(1-ethyl-pyrrolidin-2-ylmethyl)-acetamide (composé 56).

13. Procédé de préparation d'un composé de formule (I) selon l'une quelconque des revendications 1 à 12, **caractérisé en ce que** l'on fait réagir selon une réaction de couplage de Suzuki (i) un composé de formule (VII) suivante: 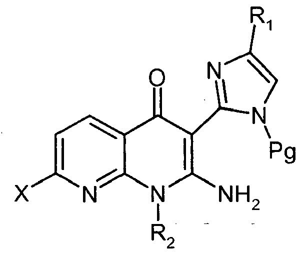 dans laquelle Pg représentant un groupe protecteur et X représentant un atome d'halogène,
avec (ii) un acide boronique ou un ester boronique de pinacol de formule (IXa) : 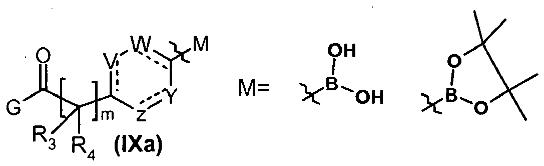 dans laquelle G représente un groupement -NR5R6 ou G représente un groupement -OEt ;
afin d'obtenir respectivement (iii) un composé de formule (X) suivante : 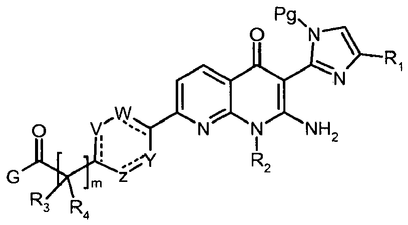 dans lequel G représente un groupement -NR5R6 et Pg est tel que défini précédemment ;
ou (iv) un composé de formule (XI) suivante : 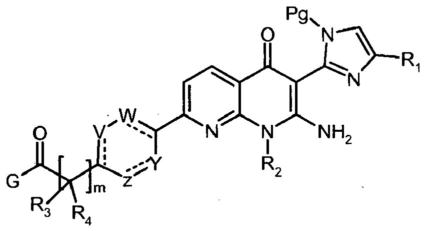 dans lequel G représente un groupement -OEt et Pg est tel que défini précédemment ; étant entendu que m, R1, R2, R3, R4, R5, R6, V, W, Y et Z sont tels que définis dans la revendication 1.

14. Procédé de préparation d'un composé de formule (I) selon l'une quelconque des revendications 1 à 12, **caractérisé en ce que** l'on fait réagir selon une réaction de couplage de Stille (i) un composé de formule (VIII) : 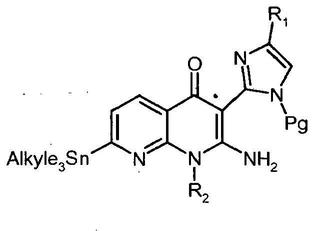 dans lequelle Pg représentant un groupe protecteur ;
avec (ii) un halogénure d'aryle ou d'hétéroaryle de formule (IXb) suivante: 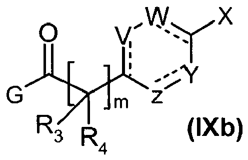 dans laquelle G représente un groupement -OEt ou G représente un groupement -NR5R6 et X représente un atome d'halogène ;
afin d'obtenir (iii) un composé de formule (X) suivante : 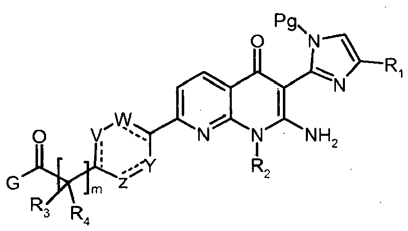 pour lequel G représente un groupement -NR5R6 et Pg est tel que défini précédemment ; ou (iv) un composé (XI) de formule suivante : 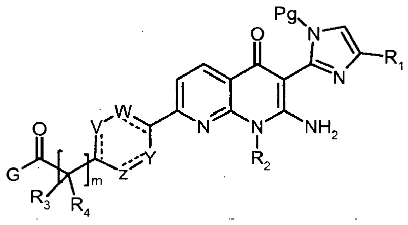 pour lequel G représente un groupement -OEt,
étant entendu que m, R1, R2, R3, R4, R5, R6, V, W, Y et Z sont tels que définis dans la revendication 1.

15. Procédé de préparation d'un composé de formule (I) selon l'une des revendications 13 ou 14, **caractérisé en ce que** le composé (XI) pour lequel G représente un groupement -OEt subit ensuite une étape de saponification puis une étape de couplage peptidique avec l'amine HNR5R6 conduisant à l'obtention du composé (XIII) de formule suivante : 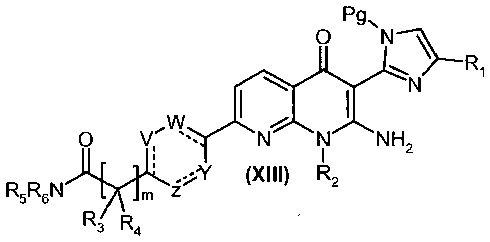 étant entendu que Pg représente un groupe protecteur et que m, R1, R2, R3, R4, R5, R6, V, W, Y et Z sont tels que définis dans la revendication 1.

16. Médicament, **caractérisé en ce qu'**il comprend un composé de formule (I) selon l'une quelconque des revendications 1 à 12, ou un sel d'addition de ce composé à un acide pharmaceutiquement acceptable, ou encore un solvat du composé de formule (I).

17. Composition pharmaceutique, **caractérisée en ce qu'**elle comprend un composé de formule (I) selon l'une quelconque des revendications 1 à 12, ou un sel pharmaceutiquement acceptable, un solvat de ce composé, ainsi qu'au moins un excipient pharmaceutiquement acceptable.

18. Utilisation d'un composé de formule (I) selon l'une quelconque des revendications 1 à 12 pour la préparation d'un médicament destiné au traitement des maladies liées à l'activité des protéines kinases, ledit composé de formule (I) inhibant l'activité de tyrosine kinase de PDGF-R, en particulier PDGF-R beta, dans des cellules Baf3 tel/PDGF et/ou inhibant l'activité de tyrosine kinase de Flt-3 dans des cellules MV4-11.

19. Utilisation d'un composé de formule (I) selon l'une quelconque des revendications 1 à 12 pour la préparation d'un médicament destiné au traitement des maladies prolifératives telles que les cancers à tumeurs liquides, les leucémies chroniques ou aiguës, les lymphomes lymphocytaires, la maladie de Hodgkin, les syndromes myeloproliferatifs et les syndromes myelodysplasiques.

20. Utilisation d'un composé de formule (I) selon l'une quelconque des revendications 1 à 12 pour la préparation d'un médicament destiné au traitement des maladies prolifératives telles que les cancers à tumeurs solides comprenant les cancers du poumon (NSCLC), des os, du pancréas, de la peau, le syndrome de Kaposi, les mélanomes intraoculaires, les cancers liés aux organes sexuels comprenant le cancer du sein, de l'utérus, du col de l'utérus, des ovaires, de l'endomètre, du vagin, de la vulve, de l'urètre, du pénis, de la prostate, les carcinomes des trompes de Fallope, les cancers de type Tumeurs Stromales Gastro-Intestinales (abrégé GIST) comprenant les cancers de la région anale, du rectum, de l'intestin grêle, du colon, de l'estomac, de l'oesophage, les cancers des glandes endocrines, thyroïdes, parathyroïdes ou surrénales, les sarcomes des tissus mous, les sarcomes d'Ewing, les ostésarcomes, les dermatofibrosarcomes et autres fibrosarcomes, les cancers de la vessie ou du rein, les néoplasmes du système nerveux central, les tumeurs de la colonne vertébrale et desmoïdes, les gliomes du tronc cérébral et glioblastomes, les adénomes pituitaires et leurs métastases.

21. Utilisation d'un composé de formule (I) selon l'une quelconque des revendications 1 à 12 pour la préparation d'un médicament destiné au traitement de maladies prolifératives non-malignes telles que la resténose, l'athérosclérose, la thrombose, l'insuffisance cardiaque, l'hypertrophie cardiaque, hypertension artérielle pulmonaire, la fibrose, la néphropathie diabétique, la glomérulonéphrite, la pyélonéphrite chronique, les hemangiomes, les maladies auto-immunes telles que le psoriasis, les sclérodermatites, l'immunosuppression, les pathologies de l'oeil telles que la fibrose post-opératoire et la dégénérescence maculaire liée à l'âge.

22. Composé de formule (I) selon l'une quelconque des revendications 1 à 12 pour la préparation d'un médicament destiné au traitement des maladies prolifératives telles que les cancers à tumeurs liquides, les leucémies chroniques ou aiguës, les lymphomes lymphocytaires, la maladie de Hodgkin, les syndromes myeloproliferatifs et les syndromes myetodysptasiques.

23. Composé de formule (I) selon l'une quelconque des revendications 1 à 12 pour la préparation d'un médicament destiné au traitement des maladies prolifératives telles que les cancers à tumeurs solides comprenant les cancers du poumon (NSCLC), des os, du pancréas, de la peau, le syndrome de Kaposi, les mélanomes intraoculaires, les cancers liés aux organes sexuels comprenant le cancer du sein, de l'utérus, du col de l'utérus, des ovaires, de l'endomètre, du vagin, de la vulve, de l'urètre, du pénis, de la prostate, les carcinomes des trompes de Fallope, les cancers de type Tumeurs Stromales Gastro-Intestinales (abrégé GIST) comprenant les cancers de la région anale, du rectum, de l'intestin grêle, du colon, de l'estomac, de l'oesophage, les cancers des glandes endocrines, thyroïdes, parathyroïdes ou surrénales, les sarcomes des tissus mous, les sarcomes d'Ewing, les ostésarcomes, les dermatofibrosarcomes et autres fibrosarcomes, les cancers de la vessie ou du rein, les néoplasmes du système nerveux central, les tumeurs de la colonne vertébrale et desmoïdes, les gliomes du tronc cérébral et glioblastomes, les adénomes pituitaires et leurs métastases.

24. Composé de formule (I) selon l'une quelconque des revendications 1 à 12 pour la préparation d'un médicament destiné au traitement de maladies prolifératives non-malignes telles que la resténose, l'athérosclérose, la thrombose, l'insuffisance cardiaque, l'hypertrophie cardiaque, hypertension artérielle pulmonaire, la fibrose, la néphropathie diabétique, la glomérulonéphrite, la pyélonéphrite chronique, les hemangiomes, les maladies auto-immunes telles que le psoriasis, les sclérodermatites, l'immunosuppression, les pathologies liées à l'oeil telles que la fibrose post-opératoire ou la dégénérescence maculaire liée à l'âge.

25. Association d'au moins un composé de formule (I) selon l'une quelconque des revendications 1 à 12 avec au moins un agent de chimiothérapie.

## Patentansprüche

1. Verbindung der Formel (I) 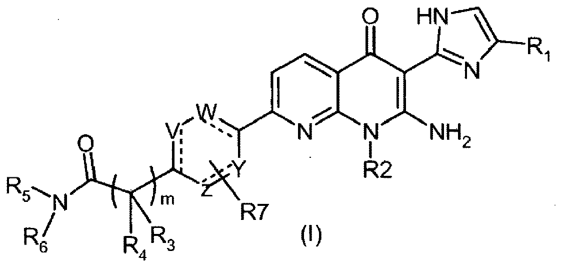 worin
**R1** für ein Wasserstoffatom oder eine (C1-C4)-Alkylgruppe steht,
**R2** für eine Gruppe -(CH₂)_{n'}-B steht, wobei:
• n' = 0, 1, 2, 3 oder 4 und
• B für (i) eine (C3-C5)-Cycloalkylgruppe oder eine (C1-C4)-Alkylgruppe, die gegebenenfalls durch ein oder mehrere Fluoratome substituiert ist, oder (ii) eine (C1-C4)-Alkoxygruppe steht;
**Y, Z, V** und **W** unabhängig voneinander für:
• eine -CH-Gruppe,
• ein Kohlenstoffatom, das gegebenenfalls durch eine Gruppe R7 substituiert ist, wobei die Gruppe R7 für eine (C1-C4)-Alkylgruppe oder ein Halogenatom steht,
• ein Heteroatom wie ein Stickstoffatom, ein Schwefelatom oder ein Sauerstoffatom oder
• kein Atom
stehen, mit der Maßgabe, dass der Ring, in dem V, W, Y und Z enthalten sind, ein 5- oder 6-gliedriger Ring ist, mit der Maßgabe, dass die gestrichelten Linien in dem Ring zeigen, dass der resultierende Ring ein aromatischer Ring ist, und mit der Maßgabe, dass der Ring 0, 1 oder 2 Heteroatome umfasst;
**R3** und **R4** unabhängig voneinander für gleiche oder verschiedene Gruppen stehen, wobei R3 und R4 aus:
• einem Wasserstoffatom und
• einer linearen (C1-C4)-Alkylgruppe ausgewählt sind,
oder **R3** und **R4** zusammen mit dem Kohlenstoff, an den sie gebunden sind, eine (C3-C5)-Cycloalkylgruppe bilden,
**m** für eine ganze Zahl mit einem Wert von 1, 2, 3 oder 4 steht,
**R5** für ein Wasserstoffatom oder eine (C1-C4)-Alkylgruppe steht,
**R6** für eine Gruppe -(CH₂)ₙ-**L** steht, wobei
• n = 0, 1, 2 oder 3 und
• **L** für eine Gruppe steht, die aus den folgenden Gruppen ausgewählt ist:
• einem Aryl mit 6 Kohlenstoffatomen;
• einem 5- oder 6-gliedrigen Heteroaryl mit mindestens einem aus Stickstoff, Sauerstoff und Schwefel ausgewählten Heteroatom;
• einem 5-, 6-oder 7-gliedrigen gesättigten Heterocyclus mit mindestens einem aus Stickstoff und Sauerstoff ausgewählten Heteroatom, wobei es sich bei dem Heterocyclus gegebenenfalls um ein Lactam handelt;
wobei die Aryl-, Heteroaryl- oder Heterocyclusgruppe gegebenenfalls durch mindestens einen Substituenten, der aus (i) linearen oder verzweigten (C1-C4)-Alkylgruppen, (ii) (C3-C5)-Cycloalkylgruppen, (iii) Halogenatomen, (iv) Arylgruppen und (v) Benzyl ausgewählt ist, substituiert ist;
mit der Maßgabe, dass dann, wenn **L** für ein Heteroaryl oder einen Heterocyclus steht, das bzw. der mindestens ein Stickstoffatom umfasst, letzteres gegebenenfalls durch den Substituenten substituiert sein kann;
oder **R5** und **R6** zusammen mit dem Stickstoffatom, an das sie gebunden sind, eine Heterocyclusgruppe bilden, die gegebenenfalls durch mindestens
• ein Heteroaryl oder
• eine (C1-C3)-Alkylgruppe, die ihrerseits durch einen Heterocyclus mit 5 oder 6 Atomen und mindestens einem aus Stickstoff und Sauerstoff ausgewählten Heteroatom substituiert sein kann, mit der Maßgabe, dass dann, wenn es sich um einen Heterocyclus mit mindestens einem Stickstoffatom handelt, letzterer gegebenenfalls substituiert sein kann;
substituiert ist;
wobei die Verbindung der Formel (I), ihre Enantiomere und Diastereoisomere einschließlich Gemischen davon in Basen- oder Säureadditionssalzform und/oder Solvatform vorliegen.

2. Verbindung nach dem vorhergehenden Anspruch, **dadurch gekennzeichnet, dass R6** für eine Gruppe -(CH₂)ₙ-**L** steht, wobei
• n = 0, 1, 2 oder 3 und
• **L** für eine Gruppe steht, die aus den folgenden Gruppen ausgewählt ist:
• einem 5-gliedrigen Heteroaryl mit (i) 2 unabhängig voneinander aus Stickstoff, Sauerstoff und Schwefel ausgewählten Heteroatomen oder (ii) 3 unabhängig voneinander aus Stickstoff und Schwefel ausgewählten Heteroatomen,
• einem 6-gliedrigen Heteroaryl mit 1 oder 2 Heteroatomen,
• einem 5-gliedrigen Heterocyclus mit einem aus Stickstoff und Sauerstoff ausgewählten Heteroatom, wobei es sich bei dem Heterocyclus gegebenenfalls um ein Lactam handelt, und
• einem 6-gliedrigen Heterocyclus mit 2 aus Stickstoff und Sauerstoff ausgewählten Heteroatomen,
wobei die Heteroaryl- oder Heterocyclusgruppe gegebenenfalls durch mindestens einen Substituenten, der aus (i) linearen oder verzweigten (C1-C4)-Alkylgruppen, (ii) (C3-C5)-Cycloalkylgruppen, (iii) Halogenatomen, (iv) Arylgruppen und (v) Benzyl ausgewählt ist, substituiert ist;
mit der Maßgabe, dass dann, wenn **L** für ein Heteroaryl oder einen Heterocyclus steht, das bzw. der mindestens ein Stickstoffatom umfasst, letzteres gegebenenfalls durch den Substituenten substituiert sein kann.

3. Verbindung nach einem der vorhergehenden Ansprüche, **dadurch gekennzeichnet, dass L** für:
• ein 6-gliedriges Heteroaryl, das aus Pyridin, Pyrazin, Pyridazin und Pyrimidin ausgewählt ist;
• ein Aryl wie Phenyl oder
• ein 5-gliedriges Heteroaryl, das aus Thiazol, Imidazol, Pyrazol, Isoxazol und 1,3,4-Thiadiazol ausgewählt ist, oder
• einen gesättigten 5-gliedrigen Heterocyclus, der aus Pyrrolidin, Tetrahydrofuran und 2-Oxopyrrolidin ausgewählt ist, oder
• einen gesättigten 6-gliedrigen Heterocyclus, der aus Morpholin, Piperazin und Piperidin ausgewählt ist,
steht, wobei die Aryl-, Heteroaryl- oder Heterocyclusgruppe gegebenenfalls durch mindestens einen Substituenten, der aus (i) linearen oder verzweigten (C1-C4)-Alkylgruppen, (ii) (C3-C5)-Cycloalkylgruppen und (iii) Arylgruppen ausgewählt ist, substituiert ist;
mit der Maßgabe, dass dann, wenn **L** für ein Heteroaryl oder einen Heterocyclus steht, das bzw. der mindestens ein Stickstoffatom umfasst, letzteres gegebenenfalls durch den Substituenten substituiert sein kann.

4. Verbindung nach einem der vorhergehenden Ansprüche, **dadurch gekennzeichnet, dass L** aus:
• Pyridin, das gegebenenfalls durch mindestens eine lineare oder verzweigte (C1-C4)-Alkylgruppe substituiert ist,
• Morpholin, das gegebenenfalls durch mindestens (i) eine (C3-C5)-Cycloalkylgruppe oder (ii) eine lineare oder verzweigte (C1-C4)-Alkylgruppe substituiert ist,
• einem Pyrrolidin, das gegebenenfalls durch mindestens (i) eine lineare oder verzweigte (C1-C4)-Alkylgruppe oder (ii) ein Benzyl substituiert ist,
• einem Thiazol, das gegebenenfalls durch mindestens (i) eine lineare oder verzweigte (C1-C4)-Alkylgruppe oder (ii) ein Chloratom substituiert ist,
• einem Imidazol, das gegebenenfalls durch mindestens eine lineare oder verzweigte (C1-C4)-Alkylgruppe substituiert ist,
• einem 2-Oxopyrrolidin,
• einem 1,3,4-Thiadiazol, das gegebenenfalls durch mindestens (i) eine lineare oder verzweigte (C1-C4)-Alkylgruppe oder (ii) eine (C3-C5)-Cycloalkylgruppe substituiert ist,
• einem Isoxazol, das gegebenenfalls durch mindestens eine lineare oder verzweigte (C1-C4)-Alkylgruppe substituiert ist,
• einem Pyrazol, das gegebenenfalls durch mindestens eine lineare oder verzweigte (C1-C4)-Alkylgruppe substituiert ist,
• einem Pyrazin,
• einem Isothiazol, das gegebenenfalls durch mindestens eine lineare oder verzweigte (C1-C4)-Alkylgruppe substituiert ist,
• einem Phenyl,
• einem Tetrahydrofuran
ausgewählt ist, mit der Maßgabe, dass dann, wenn **L** für ein Heteroaryl oder einen Heterocyclus steht, das bzw. der mindestens ein Stickstoffatom umfasst, letzteres gegebenenfalls substituiert sein kann.

5. Verbindung nach einem der vorhergehenden Ansprüche, **dadurch gekennzeichnet, dass R5** für ein Wasserstoffatom oder ein Methyl steht.

6. Verbindung nach einem der vorhergehenden Ansprüche, **dadurch gekennzeichnet, dass**:
• m gleich 1 oder 3 ist und/oder
• **R3** und **R4** unabhängig voneinander für gleiche oder verschiedene Gruppen stehen, wobei R3 und R4 aus:
∘ einem Wasserstoffatom und
∘ einem Methyl
ausgewählt sind.

7. Verbindung nach einem der vorhergehenden Ansprüche, **dadurch gekennzeichnet, dass Y, Z, V** und **W** unabhängig voneinander für:
• eine -CH-Gruppe;
• ein Kohlenstoffatom, das durch eine Gruppe R7 substituiert ist, wobei die Gruppe R7 für eine (C1-C4)-Alkylgruppe oder ein Fluoratom steht; oder
• ein Heteroatom wie ein Stickstoffatom, ein Schwefelatom oder ein Sauerstoffatom, vorteilhafterweise ein Stickstoffatom;
stehen.

8. Verbindung nach einem der vorhergehenden Ansprüche, **dadurch gekennzeichnet, dass R1** für ein Wasserstoffatom oder ein Methyl steht.

9. Verbindung nach einem der vorhergehenden Ansprüche, **dadurch gekennzeichnet, dass R2** für eine Gruppe -(CH₂)_{n'}-B steht, wobei:
o n' = 0, 1 oder 3; und/oder
o B für (i) eine (C3-C5)-Cycloalkylgruppe, (ii) eine (C1-C4)-Alkylgruppe oder (iii) eine (C1-C4)-Alkoxygruppe steht.

10. Verbindung nach einem der Ansprüche 1 und 6 bis 9, **dadurch gekennzeichnet, dass R5** und **R6** zusammen mit dem Stickstoffatom, an das sie gebunden sind, eine Heterocyclusgruppe bilden, die gegebenenfalls durch mindestens
• ein Heteroaryl, vorteilhafterweise ein Pyridin; oder
• eine (C1-C3)-Alkylgruppe, die ihrerseits durch einen Heterocyclus mit 5 oder 6 Atomen und mindestens einem aus Stickstoff und Sauerstoff ausgewählten Heteroatom substituiert sein kann, vorteilhafterweise handelt es sich um eine C1-Alkylgruppe, die ihrerseits durch einen Heterocyclus mit 5 Atomen, von denen eines ein Stickstoffatom ist, substituiert ist;
substituiert ist.

11. Verbindung nach einem der vorhergehenden Ansprüche, **dadurch gekennzeichnet, dass** sie in Basen- oder Säureadditionssalzform wie Salzsäure-oder Trifluoressigsäureadditionssalzform vorliegen.

12. Verbindung nach einem der vorhergehenden Ansprüche, **dadurch gekennzeichnet, dass** es sich um:
2-{4-[7-Amino-8-ethyl-6-(1H-imidazol-2-yl)-5-oxo-5,8-dihydro[1,8]naphthyridin-2-yl]phenyl}-N-pyridin-2-ylacetamid (Verbindung 1);
2-{6-[7-Amino-8-ethyl-6-(1H-imidazol-2-yl)-5-oxo-5,8-dihydro[1,8]naphthyridin-2-yl]pyridin-3-yl}-N-pyridin-2-ylacetamid (Verbindung 2);
2-{4-[7-Amino-8-ethyl-6-(1H-imidazol-2-yl)-5-oxo-5,8-dihydro[1,8]naphthyridin-2-yl]phenyl}-N-(4-cyclopropylmorpholin-3-ylmethyl)acetamid (Verbindung 3);
2-{4-[7-Amino-8-ethyl-6-(1H-imidazol-2-yl)-5-oxo-5,8-dihydro[1,8]naphthyridin-2-yl]phenyl}-N-(4-isopropylmorpholin-3-ylmethyl)acetamid (Verbindung 4) ;
2-Amino-1-ethyl-3-(1H-imidazol-2-yl)-7-{4-[2-oxo-2-((S)-2-pyrrolidin-1-ylmethyl-pyrrolidin-1-yl)ethyl]phenyl}-1H-[1,8]naphthyridin-4-on (Verbindung 5);
2-{4-[7-Amino-8-ethyl-6-(1H-imidazol-2-yl)-5-oxo-5,8-dihydro[1,8]naphthyridin-2-yl]phenyl}-N-(2-pyridin-4-ylethyl)acetamid (Verbindung 6);
2-{4-[7-Amino-8-ethyl-6-(1H-imidazol-2-yl)-5-oxo-5,8-dihydro[1,8]naphthyridin-2-yl]phenyl}-N-methyl-N-(2-pyridin-4-ylethyl)acetamid (Verbindung 7) ;
2-{4-[7-Amino-8-ethyl-6-(1H-imidazol-2-yl)-5-oxo-5,8-dihydro[1,8]naphthyridin-2-yl]phenyl}-N-(5-methylthiazol-2-yl)acetamid (Verbindung 8);
2-{4-[7-Amino-8-ethyl-6-(1H-imidazol-2-yl)-5-oxo-5,8-dihydro[1,8]naphthyridin-2-yl]-3-methyl-phenyl}-N-(1-ethyl-pyrrolidin-2-ylmethyl)acetamid (Verbindung 9);
2-{4-[7-Amino-8-ethyl-6-(1H-imidazol-2-yl)-5-oxo-5,8-dihydro[1,8]naphthyridin-2-yl]phenyl}-N-(6-methylpyridin-3-yl)acetamid (Verbindung 10);
2-{4-[7-Amino-8-ethyl-6-(1H-imidazol-2-yl)-5-oxo-5,8-dihydro[1,8]naphthyridin-2-yl]phenyl}-N-(1,3-dimethyl-1H-pyrazol-4-ylmethyl)acetamid (Verbindung 11);
2-{4-[7-Amino-8-ethyl-6-(1H-imidazol-2-yl)-5-oxo-5,8-dihydro[1,8]naphthyridin-2-yl]phenyl}-N-thiazol-2-ylmethylacetamid (Verbindung 12);
2-{4-[7-Amino-8-ethyl-6-(1H-imidazol-2-yl)-5-oxo-5,8-dihydro[1,8]naphthyridin-2-yl]phenyl}-N-(5-oxopyrrolidin-2-ylmethyl)acetamid (Verbindung 13);
2-{4-[7-Amino-8-ethyl-6-(1H-imidazol-2-yl)-5-oxo-5,8-dihydro[1,8]naphthyridin-2-yl]phenyl}-N-(5-methyl[1,3,4]thiadiazol-2-yl)acetamid (Verbindung 14);
2-{4-[7-Amino-8-ethyl-6-(1H-imidazol-2-yl)-5-oxo-5,8-dihydro[1,8]naphthyridin-2-yl]phenyl}-N-[1,3,4]thiadiazol-2-ylacetamid (Verbindung 15);
2-{4-[7-Amino-8-ethyl-6-(1H-imidazol-2-yl)-5-oxo-5,8-dihydro[1,8]naphthyridin-2-yl]phenyl}-N-(3-methylisoxazol-5-yl)acetamid (Verbindung 16);
2-{4-[7-Amino-8-ethyl-6-(1H-imidazol-2-yl)-5-oxo-5,8-dihydro[1,8]naphthyridin-2-yl]phenyl}-N-[2-(1-methylpyrrolidin-2-yl)ethyl]acetamid (Verbindung 17);
2-{4-[7-Amino-8-ethyl-6-(1H-imidazol-2-yl)-5-oxo-5,8-dihydro[1,8]naphthyridin-2-yl]phenyl}-N-(4-methylthiazol-2-yl)acetamid (Verbindung 18);
2-{4-[7-Amino-8-ethyl-6-(1H-imidazol-2-yl)-5-oxo-5,8-dihydro[1,8]naphthyridin-2-yl]phenyl}-N-pyridin-4-ylmethylacetamid (Verbindung 19);
2-{4-[7-Amino-8-ethyl-6-(1H-imidazol-2-yl)-5-oxo-5,8-dihydro[1,8]naphthyridin-2-yl]phenyl}-N-(5-cyclopropyl[1,3,4]thiadiazol-2-yl)acetamid (Verbindung 20);
2-{4-[7-Amino-8-ethyl-6-(1H-imidazol-2-yl)-5-oxo-5,8-dihydro[1,8]naphthyridin-2-yl]phenyl}-N-(2-pyridin-3-ylethyl)acetamid (Verbindung 21);
2-{4-[7-Amino-8-ethyl-6-(1H-imidazol-2-yl)-5-oxo-5,8-dihydro[1,8]naphthyridin-2-yl]phenyl}-N-(2,5-dimethyl-2H-pyrazol-3-ylmethyl)acetamid (Verbindung 22);
2-{4-[7-Amino-8-ethyl-6-(1H-imidazol-2-yl)-5-oxo-5,8-dihydro[1,8]naphthyridin-2-yl]phenyl}-N-(1-methyl-1H-imidazol-4-ylmethyl)acetamid (Verbindung 23) ;
2-{4-[7-Amino-8-ethyl-6-(1H-imidazol-2-yl)-5-oxo-5,8-dihydro[1,8]naphthyridin-2-yl]phenyl}-N-pyrazin-2-ylacetamid (Verbindung 24);
2-{4-[7-Amino-8-ethyl-6-(1H-imidazol-2-yl)-5-oxo-5,8-dihydro[1,8]naphthyridin-2-yl]phenyl}-N-(2-pyridin-2-ylethyl)acetamid (Verbindung 25);
2-{4-[7-Amino-8-ethyl-6-(1H-imidazol-2-yl)-5-oxo-5,8-dihydro[1,8]naphthyridin-2-yl]phenyl}-N-(5-chlorthiazol-2-yl)acetamid (Verbindung 26);
2-{4-[7-Amino-8-ethyl-6-(1H-imidazol-2-yl)-5-oxo-5,8-dihydro[1,8]naphthyridin-2-yl]phenyl}-N-(3,4-dimethylisoxazol-5-yl)acetamid (Verbindung 27);
2-{4-[7-Amino-8-ethyl-6-(1H-imidazol-2-yl)-5-oxo-5,8-dihydro[1,8]naphthyridin-2-yl]phenyl}-N-(2-methylpyridin-4-yl)acetamid (Verbindung 28);
2-{4-[7-Amino-8-ethyl-6-(1H-imidazol-2-yl)-5-oxo-5,8-dihydro[1,8]naphthyridin-2-yl]phenyl}-N-pyrazin-2-ylmethylacetamid (Verbindung 29);
2-{4-[7-Amino-8-ethyl-6-(1H-imidazol-2-yl)-5-oxo-5,8-dihydro[1,8]naphthyridin-2-yl]phenyl}-N-(5-ethyl[1,3,4]thiadiazol-2-yl)acetamid (Verbindung 30) ;
2-{4-[7-Amino-8-ethyl-6-(1H-imidazol-2-yl)-5-oxo-5,8-dihydro[1,8]naphthyridin-2-yl]phenyl}-N-(tetrahydrofuran-2-ylmethyl)acetamid (Verbindung 31) ;
2-{4-[7-Amino-8-ethyl-6-(1H-imidazol-2-yl)-5-oxo-5,8-dihydro[1,8]naphthyridin-2-yl]phenyl}-N-(3-methylpyridin-2-yl)acetamid (Verbindung 32);
2-{4-[7-Amino-8-ethyl-6-(1H-imidazol-2-yl)-5-oxo-5,8-dihydro[1,8]naphthyridin-2-yl]phenyl}-N-(4-methylpyridin-2-yl)acetamid (Verbindung 33);
2-{4-[7-Amino-8-ethyl-6-(1H-imidazol-2-yl)-5-oxo-5,8-dihydro[1,8]naphthyridin-2-yl]phenyl}-N-(4,6-dimethylpyridin-2-yl)acetamid (Verbindung 34);
2-{4-[7-Amino-8-ethyl-6-(1H-imidazol-2-yl)-5-oxo-5,8-dihydro[1,8]naphthyridin-2-yl]phenyl}-N-(6-methylpyridin-2-yl)acetamid (Verbindung 35);
2-{4-[7-Amino-8-ethyl-6-(1H-imidazol-2-yl)-5-oxo-5,8-dihydro[1,8]naphthyridin-2-yl]phenyl}-N-pyridin-3-ylmethylacetamid (Verbindung 36);
2-{4-[7-Amino-8-ethyl-6-(1H-imidazol-2-yl)-5-oxo-5,8-dihydro[1,8]naphthyridin-2-yl]phenyl}-N-(2-ethyl-2H-pyrazol-3-yl)acetamid (Verbindung 37);
2-{4-[7-Amino-8-ethyl-6-(1H-imidazol-2-yl)-5-oxo-5,8-dihydro[1,8]naphthyridin-2-yl]phenyl}-N-(3-methylisothiazol-5-yl)acetamid (Verbindung 38);
2-Amino-1-ethyl-3-(1H-imidazol-2-yl)-7-{4-[2-oxo-2-(2-pyridin-3-yl-pyrrolidin-1-yl)ethyl]phenyl}-1H-[1,8]naphthyridin-4-on (Verbindung 39);
2-{4-[7-Amino-8-ethyl-6-(1H-imidazol-2-yl)-5-oxo-5,8-dihydro[1,8]naphthyridin-2-yl]phenyl}-N-(1-benzylpyrrolidin-3-yl)acetamid (Verbindung 40);
2-{4-[7-Amino-8-ethyl-6-(1H-imidazol-2-yl)-5-oxo-5,8-dihydro[1,8]naphthyridin-2-yl]phenyl}-N-[(S)-1-(tetrahydrofuran-2-yl)methyl]acetamid (Verbindung 41) ;
2-{4-[7-Amino-8-ethyl-6-(1H-imidazol-2-yl)-5-oxo-5,8-dihydro[1,8]naphthyridin-2-yl]phenyl}-N-(4-methylpyridin-3-yl)acetamid (Verbindung 42);
2-{4-[7-Amino-8-ethyl-6-(1H-imidazol-2-yl)-5-oxo-5,8-dihydro[1,8]naphthyridin-2-yl]phenyl}-N-(4-ethylpyridin-2-yl)acetamid (Verbindung 43);
2-{4-[7-Amino-8-ethyl-6-(1H-imidazol-2-yl)-5-oxo-5,8-dihydro[1,8]naphthyridin-2-yl]phenyl}-N-ethyl-N-pyridin-4-ylmethylacetamid (Verbindung 44);
2-{4-[7-Amino-8-ethyl-6-(1H-imidazol-2-yl)-5-oxo-5,8-dihydro[1,8]naphthyridin-2-yl]phenyl}-N-(6-ethylpyridin-2-yl)acetamid (Verbindung 45);
2-{4-[7-Amino-8-ethyl-6-(1H-imidazol-2-yl)-5-oxo-5,8-dihydro[1,8]naphthyridin-2-yl]phenyl}-N-benzylacetamid (Verbindung 46);
2-{4-[7-Amino-8-ethyl-6-(1H-imidazol-2-yl)-5-oxo-5,8-dihydro[1,8]naphthyridin-2-yl]phenyl}-N-pyridin-4-ylmethylpropionamid (Verbindung 47);
4-[7-Amino-8-ethyl-6-(1H-imidazol-2-yl)-5-oxo-5,8-dihydro[1,8]naphthyridin-2-yl]-N-pyridin-4-ylmethylbenzamid (Verbindung 48);
4-{4-[7-Amino-8-ethyl-6-(1H-imidazol-2-yl)-5-oxo-5,8-dihydro[1,8]naphthyridin-2-yl]phenyl}-N-pyridin-4-ylmethylbutyramid (Verbindung 49);
2-{4-[7-Amino-8-ethyl-6-(4-methyl-1H-imidazol-2-yl)-5-oxo-5,8-dihydro[1,8]naphthyridin-2-yl]-phenyl}-N-pyridin-4-ylmethylacetamid (Verbindung 50) ;
2-{4-[7-Amino-8-CyClopropylmethyl-6-(1H-imidazol-2-yl)-5-oxo-5,8-dihydro[1,8]naphthyridin-2-yl]-phenyl}-N-pyridin-4-ylmethylacetamid (Verbindung 51) ;
2-{4-[7-Amino-8-cyclopentyl-6-(1H-imidazol-2-yl)-5-oxo-5,8-dihydro[1,8]naphthyridin-2-yl]phenyl}-N-pyridin-4-ylmethylacetamid (Verbindung 52);
2-{5-[7-Amino-8-ethyl-6-(1H-imidazol-2-yl)-5-oxo-5,8-dihydro[1,8]naphthyridin-2-yl]-pyridin-2-yl}-N-pyridin-4-ylmethylacetamid (Verbindung 53);
2-{4-[7-Amino-6-(1H-imidazol-2-yl)-8-(3-methoxy-propyl)-5-oxo-5,8-dihydro[1,8]naphthyridin-2-yl]-phenyl}-N-pyridin-4-ylmethylacetamid (Verbindung 54);
2-{4-[7-Amino-8-ethyl-6-(1H-imidazol-2-yl)-5-oxo-5,8-dihydro[1,8]naphthyridin-2-yl]phenyl}-N-(3-phenylpropyl)acetamid (Verbindung 55);
2-{4-[7-Amino-8-ethyl-6-(1H-imidazol-2-yl)-5-oxo-5,8-dihydro[1,8]naphthyridin-2-yl]-2-fluorphenyl}-N-(1-ethylpyrrolidin-2-ylmethyl)acetamid (Verbindung 56).
handelt.

13. Verfahren zur Herstellung einer Verbindung der Formel (I) nach einem der Ansprüche 1 bis 12, **dadurch gekennzeichnet, dass** man (i) eine Verbindung der folgenden Formel (VII): 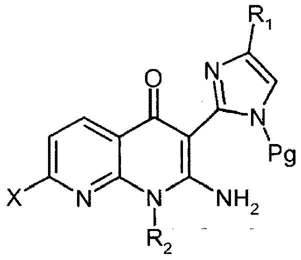 worin Pg für eine Schutzgruppe steht und X für ein Halogenatom steht,
gemäß einer Suzuki-Kupplungsreaktion mit (ii) einer Boronsäure oder einem Pinacolboronsäureester der Formel (IXa): 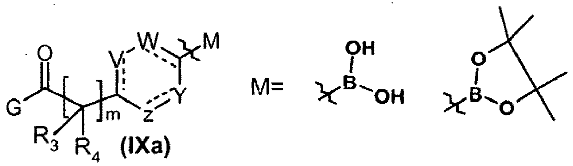 worin G für eine -NR5R6-Gruppe steht oder G für eine -OEt-Gruppe steht;
zu (iii) einer Verbindung der folgenden Formel (X) : 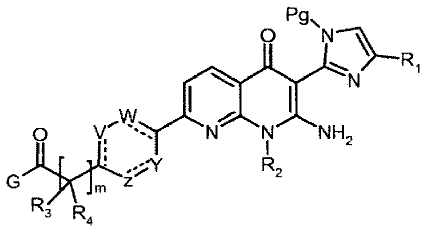 worin G für eine -NR5R6-Gruppe steht und Pg die oben angegebene Bedeutung besitzt;
bzw. (iv) einer Verbindung der folgenden Formel (XI) : 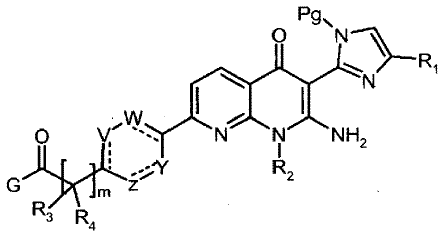 worin G für eine -OEt-Gruppe steht und Pg die oben angegebene Bedeutung besitzt;
umsetzt; mit der Maßgabe, dass m, R1, R2, R3, R4, R5, R6, V, W, Y und Z die in Anspruch 1 angegebene Bedeutung besitzen.

14. Verfahren zur Herstellung einer Verbindung der Formel (I) nach einem der Ansprüche 1 bis 12, **dadurch gekennzeichnet, dass** man (i) eine Verbindung der Formel (VIII): 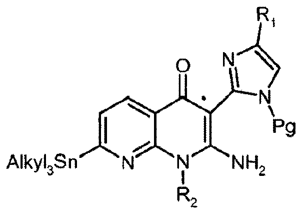 worin Pg für eine Schutzgruppe steht;
gemäß einer Stille-Kupplungsreaktion mit (ii) einem Aryl- oder Heteroarylhalogenid der folgenden Formel (IXb): 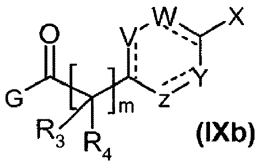 worin G für eine -OEt-Gruppe steht oder G für eine -NR5R6-Gruppe steht und X für ein Halogenatom steht;
zu (iii) einer Verbindung der folgenden Formel (X) : 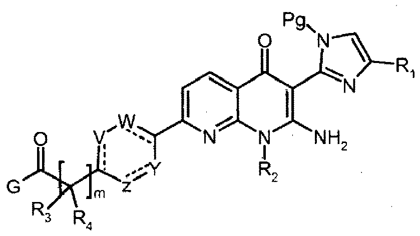 für die G für eine -NR5R6-Gruppe steht und Pg die oben angegebene Bedeutung besitzt;
oder (iv) einer Verbindung (XI) der folgenden Formel: 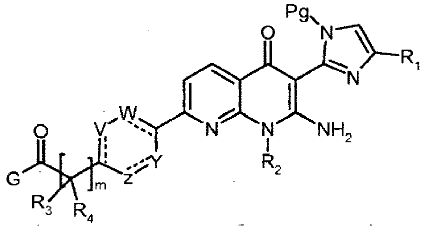 für die G für eine -OEt-Gruppe steht;
umsetzt; mit der Maßgabe, dass m, R1, R2, R3, R4, R5, R6, V, W, Y and Z die in Anspruch 1 angegebene Bedeutung besitzen.

15. Verfahren zur Herstellung einer Verbindung der Formel (I) nach einem der Ansprüche 13 oder 14, **dadurch gekennzeichnet, dass** man die Verbindung (XI), für die G für eine -OEt-Gruppe steht, anschließend einem Verseifungsschritt und dann einem Peptidkupplungsschritt mit dem Amin HNR5R6 unterwirft, was die Verbindung (XIII) der folgenden Formel ergibt: 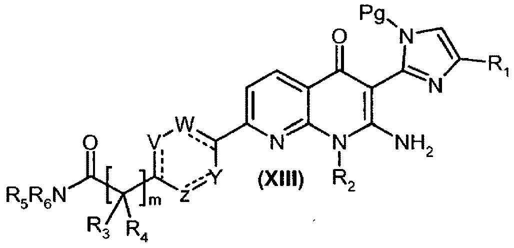 mit der Maßgabe, dass Pg für eine Schutzgruppe steht und dass m, R1, R2, R3, R4, R5, R6, V, W, Y and Z die in Anspruch 1 angegebene Bedeutung besitzen.

16. Medikament, **dadurch gekennzeichnet, dass** es eine Verbindung der Formel (I) nach einem der Ansprüche 1 bis 12 oder ein Additionssalz dieser Verbindung mit einer pharmazeutisch unbedenklichen Säure oder auch Solvat der Verbindung der Formel (I) umfasst.

17. Pharmazeutische Zusammensetzung, **dadurch gekennzeichnet, dass** sie eine Verbindung der Formel (I) nach einem der Ansprüche 1 bis 12 oder ein pharmazeutisch unbedenkliches Salz, ein Solvat dieser Verbindung sowie mindestens einen pharmazeutisch unbedenklichen Hilfsstoff umfasst.

18. Verwendung einer Verbindung der Formel (I) nach einem der Ansprüche 1 bis 12 zur Herstellung eines Medikaments zur Behandlung von Erkrankungen, die mit der Aktivität von Proteinkinasen verbunden sind, wobei die Verbindung der Formel (I) die Tyrosinkinaseaktivität von PDGF-R, insbesondere PDGF-R beta, in Baf3-tel/PDGF-Zellen inhibiert und/oder die Tyrosinkinaseaktivität von Flt-3 in MV4-11-Zellen inhibiert.

19. Verwendung einer Verbindung der Formel (I) nach einem der Ansprüche 1 bis 12 zur Herstellung eines Medikaments zur Behandlung von proliferativen Erkrankungen wie Krebserkrankungen mit flüssigen Tumoren, chronischen oder akuten Leukämien, lymphozytischen Lymphomen, Hodgkin-Krankheit, myeloproliferativen Syndromen und myelodysplastischen Syndromen.

20. Verwendung einer Verbindung der Formel (I) nach einem der Ansprüche 1 bis 12 zur Herstellung eines Medikaments zur Behandlung von proliferativen Erkrankungen wie Krebserkrankungen mit soliden Tumoren einschließlich Krebserkrankungen der Lunge (NSCLC), der Knochen, der Bauchspeicheldrüse, der Haut, Kaposi-Sarkom, intraokulären Melanomen, Krebserkrankungen, die mit Sexualorganen verbunden sind, einschließlich Krebserkrankungen der Brust, der Gebärmutter, des Gebärmutterhalses, der Eierstöcke, des Endometriums, der Vagina, der Vulva, der Harnröhre, des Penis, der Prostata, Eileiterkarzinomen, Krebserkrankungen vom Typ gastrointestinale Stromatumore (Abkürzung GIST) einschließlich Krebserkrankungen der Analregion, des Rektums, des Dünndarms, des Kolons, des Magens, der Speiseröhre, Krebserkrankungen der endokrinen Drüsen, der Schilddrüse, der Nebenschilddrüse oder der Nebenniere, Weichteilkarzinomen, Ewing-Sarkomen, Osteosarkomen, Dermatofibrosarkomen und anderen Fibrosarkomen, Krebserkrankungen der Blase oder der Nieren, Neoplasmen des Zentralnervensystems, Tumoren der Wirbelsäule und Desmoiden, Hirnstammgliomen und Glioblastomen, Hypophysenadenomen und Metastasen davon.

21. Verwendung einer Verbindung der Formel (I) nach einem der Ansprüche 1 bis 12 zur Herstellung eines Medikaments zur Behandlung von nichtmalignen proliferativen Erkrankungen wie Restenose, Atherosklerose, Thrombose, Herzinsuffizienz, Herzhypertrophie, Pulmonalarterienhypertonie, Fibrose, diabetischer Nephropathie, Glomerulonephritis, chronischer Pyelonephritis, Hämangiomen, Autoimmunkrankheiten wie Psoriasis, Sklerodermatitis, Immunsuppression, Pathologien des Auges wie postoperativer Fibrose und altersbedingter Makuladegeneration.

22. Verbindung der Formel (I) nach einem der Ansprüche 1 bis 12 zur Herstellung eines Medikaments zur Behandlung von proliferativen Erkrankungen wie Krebserkrankungen mit flüssigen Tumoren, chronischen oder akuten Leukämien, lymphozytischen Lymphomen, Hodgkin-Krankheit, myeloproliferativen Syndromen und myelodysplastischen Syndromen.

23. Verbindung der Formel (I) nach einem der Ansprüche 1 bis 12 zur Herstellung eines Medikaments zur Behandlung von proliferativen Erkrankungen wie Krebserkrankungen mit soliden Tumoren einschließlich Krebserkrankungen der Lunge (NSCLC), der Knochen, der Bauchspeicheldrüse, der Haut, Kaposi-Sarkom, intraokulären Melanomen, Krebserkrankungen, die mit Sexualorganen verbunden sind, einschließlich Krebserkrankungen der Brust, der Gebärmutter, des Gebärmutterhalses, der Eierstöcke, des Endometriums, der Vagina, der Vulva, der Harnröhre, des Penis, der Prostata, Eileiterkarzinomen, Krebserkrankungen vom Typ gastrointestinale Stromatumore (Abkürzung GIST) einschließlich Krebserkrankungen der Analregion, des Rektums, des Dünndarms, des Kolons, des Magens, der Speiseröhre, Krebserkrankungen der endokrinen Drüsen, der Schilddrüse, der Nebenschilddrüse oder der Nebenniere, Weichteilkarzinomen, Ewing-Sarkomen, Osteosarkomen, Dermatofibrosarkomen und anderen Fibrosarkomen, Krebserkrankungen der Blase oder der Nieren, Neoplasmen des Zentralnervensystems, Tumoren der Wirbelsäule und Desmoiden, Hirnstammgliomen und Glioblastomen, Hypophysenadenomen und Metastasen davon.

24. Verbindung der Formel (I) nach einem der Ansprüche 1 bis 12 zur Herstellung eines Medikaments zur Behandlung von nichtmalignen proliferativen Erkrankungen wie Restenose, Atherosklerose, Thrombose, Herzinsuffizienz, Herzhypertrophie, Pulmonalarterienhypertonie, Fibrose, diabetischer Nephropathie, Glomerulonephritis, chronischer Pyelonephritis, Hämangiomen, Autoimmunkrankheiten wie Psoriasis, Sklerodermatitis, Immunsuppression, Pathologien des Auges wie postoperativer Fibrose und altersbedingter Makuladegeneration.

25. Kombination von mindestens einer Verbindung der Formel (I) nach einem der Ansprüche 1 bis 12 mit mindestens einem Chemotherapiemittel.

## Claims

1. Compound corresponding to formula (I): 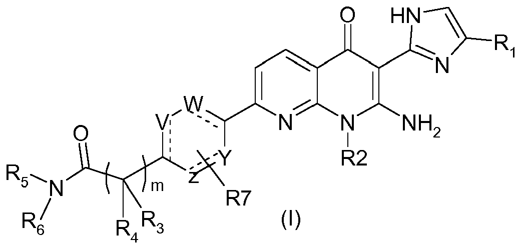 in which,
**R1** represents a hydrogen atom or a (C₁-C₄)alkyl group;
**R2** represents a group -(CH₂)_{n'}-B where:
• n'=0, 1, 2, 3 or 4; and
• B represents (i) a (C₃-C₅)cycloalkyl group or a (C₁-C₄)alkyl group, said group being optionally substituted with one or more fluorine atoms, or (ii) a (C₁-C₄)alkoxy group;
**Y, Z, V** and **W** represent, independently of one another:
• a -CH- group,
• a carbon atom optionally substituted with a group R7, said group R7 representing a (C₁-C₄)alkyl group or a halogen atom,
• a heteroatom such as a nitrogen atom, a sulphur atom or an oxygen atom, or
• no atom,
it being understood that the ring in which V, W, Y and Z are comprised is a ring comprising 5 or 6 ring members, it being understood that the dotted lines in said ring indicate that the resultant ring is an aromatic ring and it being understood that said ring comprises 0, 1 or 2 heteroatoms;
**R3** and **R4** represent, independently of one another, groups that may be identical or different, R3 and R4 being selected from:
• a hydrogen atom; and
• a linear (C₁-C₄)alkyl group;
or **R3** and **R4** form, together with the carbon to which they are bound, a (C₃-C₅)cycloalkyl group;
**m** is an integer equal to 1, 2, 3 or 4;
**R5** represents a hydrogen atom or a (C₁-C₄)alkyl group;
**R6** represents a group -(CH₂)ₙ-**L** in which:
• n= 0, 1, 2 or 3, and
• **L** is a group selected from the following groups:
o an aryl comprising 6 carbon atoms;
o a heteroaryl comprising between 5 and 6 ring members and comprising at least one heteroatom selected from nitrogen, oxygen and sulphur;
o a saturated heterocycle comprising 5, 6 or 7 ring members and comprising at least one heteroatom selected from nitrogen and oxygen, said heterocycle being optionally a lactam;
said aryl, heteroaryl or heterocyclic group being optionally substituted with at least one substituent selected from (i) linear or branched (C₁-C₄)alkyl groups, (ii) (C₃-C₅) cycloalkyl groups, (iii) halogen atoms, (iv) aryls and (v) benzyl;
it being understood that when **L** is a heteroaryl or a heterocycle, said heteroaryl or heterocycle comprising at least one nitrogen atom, the latter can optionally be substituted with said substituent;
or **R5** and **R6** form, together with the nitrogen atom to which they are bound, a heterocyclic group, optionally substituted with at least
• a heteroaryl, or
• a (C₁-C₃)alkyl group, which can itself be substituted with a heterocycle comprising 5 or 6 atoms and comprising at least one heteroatom selected from nitrogen and oxygen, it being understood that when it is a heterocycle comprising at least one nitrogen atom, the latter can optionally be substituted;
said compound of formula (I), its enantiomers and diastereoisomers, including mixtures thereof, being in the form of a base or a salt of acid addition and/or in the form of solvate.

2. Compound according to the preceding claim, **characterized in that R6** represents a group -(CH₂)ₙ-**L** in which:
• n= 0, 1, 2 or 3, and
• **L** is a group selected from the following groups:
o a heteroaryl comprising 5 ring members and comprising (i) 2 heteroatoms selected, independently of one another, from nitrogen, oxygen and sulphur, or (ii) 3 heteroatoms selected, independently of one another, from nitrogen and sulphur,
o a heteroaryl comprising 6 ring members and comprising 1 or 2 heteroatom(s),
o a heterocycle comprising 5 ring members and comprising a heteroatom selected from nitrogen and oxygen, said heterocycle being optionally a lactam, and
o a heterocycle comprising 6 ring members and comprising 2 heteroatoms selected from nitrogen and oxygen,
said heteroaryl group or heterocycle being optionally substituted with at least one substituent selected from (i) linear or branched (C₁-C₄)alkyl groups, (ii) (C₃-C₅)cycloalkyl groups, (iii) halogen atoms, (iv) aryls and (v) benzyl,
it being understood that when **L** is a heteroaryl or a heterocycle, said heteroaryl or heterocycle comprising at least one nitrogen atom, the latter can optionally be substituted with said substituent.

3. Compound according to any one of the preceding claims, **characterized in that L** is:
• a heteroaryl comprising 6 ring members selected from pyridine, pyrazine, pyridazine, pyrimidine;
• an aryl such as phenyl, or
• a heteroaryl comprising 5 ring members selected from thiazole, imidazole, pyrazole, isoxazole and 1,3,4-thiadiazole, or
• a saturated heterocycle comprising 5 ring members selected from pyrrolidine, tetrahydrofuran and 2-oxo-pyrrolidine or
• a saturated heterocycle comprising 6 ring members selected from morpholine, piperazine, piperidine,
said aryl, heteroaryl or heterocyclic group being optionally substituted with at least one substituent selected from (i) linear or branched (C₁-C₄)alkyl groups, (ii) (C₃-C₅)cycloalkyl groups and (iii) aryls,
it being understood that when **L** is a heteroaryl or a heterocycle, said heteroaryl or heterocycle comprising at least one nitrogen atom, the latter can optionally be substituted with said substituent.

4. Compound according to any one of the preceding claims, **characterized in that L** is selected from:
• pyridine, optionally substituted with at least one linear or branched (C₁-C₄)alkyl group,
• morpholine, optionally substituted with at least (i) a (C₃-C₅) cycloalkyl group or (ii) a linear or branched (C₁-C₄)alkyl group,
• a pyrrolidine, optionally substituted with at least (i) a linear or branched (C₁-C₄)alkyl group, or (ii) a benzyl,
• a thiazole, optionally substituted with at least (i) a linear or branched (C₁-C₄)alkyl group, or (ii) a chlorine atom,
• an imidazole, optionally substituted with at least one linear or branched (C₁-C₄)alkyl group,
• a 2-oxo-pyrrolidine,
• a 1,3,4-thiadiazole, optionally substituted with at least (i) a linear or branched (C₁-C₄)alkyl group, or (ii) a (C₃-C₅)cycloalkyl group,
• an isoxazole, optionally substituted with at least one linear or branched (C₁-C₄)alkyl group,
• a pyrazole, optionally substituted with at least one linear or branched (C₁-C₄)alkyl group,
• a pyrazine,
• an isothiazole, optionally substituted with at least one linear or branched (C₁-C₄)alkyl group,
• a phenyl,
• a tetrahydrofuran,
it being understood that when **L** is a heteroaryl or a heterocycle, said heteroaryl or heterocycle comprising at least one nitrogen atom, the latter can optionally be substituted.

5. Compound according to any one of the preceding claims, **characterized in that R5** represents a hydrogen atom or a methyl.

6. Compound according to any one of the preceding claims, **characterized in that**:
• m is equal to 1 or 3, and/or
• **R3** and **R4** represent, independently of one another, groups that may be identical or different, R3 and R4 being selected from:
∘ a hydrogen atom, and
∘ a methyl.

7. Compound according to any one of the preceding claims, **characterized in that Y, Z, V** and **W** represent, independently of one another:
• a group -CH-;
• a carbon atom substituted with a group R7, said group R7 representing a (C₁-C₄)alkyl group or a fluorine atom; or
• a heteroatom such as a nitrogen atom, a sulphur atom or an oxygen atom, advantageously a nitrogen atom.

8. Compound according to any one of the preceding claims, **characterized in that R1** represents a hydrogen atom or a methyl.

9. Compound according to any one of the preceding claims, **characterized in that R2** represents a group - (CH₂)_{n'}-B where:
o n'=0, 1 or 3; and/or
o B represents (i) a (C₃-C₅)cycloalkyl group, (ii) a (C₁-C₄)alkyl group or (iii) a (C₁-C₄)alkoxy group.

10. Compound according to any one of Claims 1 and 6 to 9, **characterized in that R5** and **R6** form, together with the nitrogen atom to which they are bound, a heterocyclic group, optionally substituted with at least
• a heteroaryl, advantageously a pyridine; or
• a (C₁-C₃)alkyl group, which can itself be substituted with a heterocycle comprising 5 or 6 atoms and comprising at least one heteroatom selected from nitrogen and oxygen, advantageously it is a C1alkyl group, itself substituted with a heterocycle comprising 5 atoms including a nitrogen atom.

11. Compound according to any one of the preceding claims, **characterized in that** it is in the form of a base or a salt of addition to an acid such as hydrochloric acid or trifluoroacetic acid.

12. Compound according to any one of the preceding claims, **characterized in that** it is:
2-{4-[7-Amino-8-ethyl-6-(1H-imidazol-2-yl)-5-oxo-5,8-dihydro-[1,8]naphthyridin-2-yl]-phenyl}-N-pyridin-2-yl-acetamide (compound 1);
2-{6-[7-Amino-8-ethyl-6-(1H-imidazol-2-yl)-5-oxo-5,8-dihydro-[1,8]naphthyridin-2-yl]-pyridin-3-yl}-N-pyridin-2-yl-acetamide (compound 2);
2-{4-[7-Amino-8-ethyl-6-(1H-imidazol-2-yl)-5-oxo-5,8-dihydro-[1,8]naphthyridin-2-yl]-phenyl}-N-(4-cyclopropyl-morpholin-3-ylmethyl)-acetamide (compound 3) ;
2-{4-[7-Amino-8-ethyl-6-(1H-imidazol-2-yl)-5-oxo-5,8-dihydro-[1,8]naphthyridin-2-yl]-phenyl}-N-(4-isopropyl-morpholin-3-ylmethyl)-acetamide (compound 4);
2-Amino-1-ethyl-3-(1H-imidazol-2-yl)-7-{4-[2-oxo-2-((S)-2-pyrrolidin-1-ylmethyl-pyrrolidin-1-yl)-ethyl]-phenyl}-1H-[1,8]naphthyridin-4-one (compound 5);
2-{4-[7-Amino-8-ethyl-6-(1H-imidazol-2-yl)-5-oxo-5,8-dihydro-[1,8]naphthyridin-2-yl]-phenyl}-N-(2-pyridin-4-yl-ethyl)-acetamide (compound 6);
2-{4-[7-Amino-8-ethyl-6-(1H-imidazol-2-yl)-5-oxo-5,8-dihydro-[1,8]naphthyridin-2-yl]-phenyl}-N-methyl-N-(2-pyridin-4-yl-ethyl)-acetamide (compound 7);
2-{4-[7-Amino-8-ethyl-6-(1H-imidazol-2-yl)-5-oxo-5,8-dihydro-[1,8]naphthyridin-2-yl]-phenyl}-N-(5-methyl-thiazol-2-yl)-acetamide (compound 8);
2-{4-[7-Amino-8-ethyl-6-(1H-imidazol-2-yl)-5-oxo-5,8-dihydro-[1,8]naphthyridin-2-yl]-3-methyl-phenyl}-N-(1-ethyl-pyrrolidin-2-ylmethyl)-acetamide (compound 9);
2-{4-[7-Amino-8-ethyl-6-(1H-imidazol-2-yl)-5-oxo-5,8-dihydro-[1,8]naphthyridin-2-yl]-phenyl}-N-(6-methyl-pyridin-3-yl)-acetamide (compound 10);
2-{4-[7-Amino-8-ethyl-6-(1H-imidazol-2-yl)-5-oxo-5,8-dihydro-[1,8]naphthyridin-2-yl]-phenyl}-N-(1,3-dimethyl-1H-pyrazol-4-ylmethyl)-acetamide (compound 11) ;
2-{4-[7-Amino-8-ethyl-6-(1H-imidazol-2-yl)-5-oxo-5,8-dihydro-[1,8]naphthyridin-2-yl]-phenyl}-N-thiazol-2-ylmethyl-acetamide (compound 12);
2-{4-[7-Amino-8-ethyl-6-(1H-imidazol-2-yl)-5-oxo-5,8-dihydro-[1,8]naphthyridin-2-yl]-phenyl}-N-(5-oxo-pyrrolidin-2-ylmethyl)-acetamide (compound 13);
2-{4-[7-Amino-8-ethyl-6-(1H-imidazol-2-yl)-5-oxo-5,8-dihydro-[1,8]naphthyridin-2-yl]-phenyl}-N-(5-methyl-[1,3,4]thiadiazol-2-yl)-acetamide (compound 14);
2-{4-[7-Amino-8-ethyl-6-(1H-imidazol-2-yl)-5-oxo-5,8-dihydro-[1,8]naphthyridin-2-yl]-phenyl}-N-[1,3,4]thiadiazol-2-yl-acetamide (compound 15);
2-{4-[7-Amino-8-ethyl-6-(1H-imidazol-2-yl)-5-oxo-5,8-dihydro-[1,8]naphthyridin-2-yl]-phenyl}-N-(3-methyl-isoxazol-5-yl)-acetamide (compound 16);
2-{4-[7-Amino-8-ethyl-6-(1H-imidazol-2-yl)-5-oxo-5,8-dihydro-[1,8]naphthyridin-2-yl]-phenyl}-N-[2-(1-methyl-pyrrolidin-2-yl)-ethyl]-acetamide (compound 17);
2-{4-[7-Amino-8-ethyl-6-(1H-imidazol-2-yl)-5-oxo-5,8-dihydro-[1,8]naphthyridin-2-yl]-phenyl}-N-(4-methyl-thiazol-2-yl)-acetamide (compound 18);
2-{4-[7-Amino-8-ethyl-6-(1H-imidazol-2-yl)-5-oxo-5,8-dihydro-[1,8]naphthyridin-2-yl]-phenyl}-N-pyridin-4-ylmethyl-acetamide (compound 19);
2-{4-[7-Amino-8-ethyl-6-(1H-imidazol-2-yl)-5-oxo-5,8-dihydro-[1,8]naphthyridin-2-yl]-phenyl}-N-(5-cyclopropyl-[1,3,4]thiadiazol-2-yl)-acetamide (compound 20) ;
2-{4-[7-Amino-8-ethyl-6-(1H-imidazol-2-yl)-5-oxo-5,8-dihydro-[1,8]naphthyridin-2-yl]-phenyl}-N-(2-pyridin-3-yl-ethyl)-acetamide (compound 21);
2-{4-[7-Amino-8-ethyl-6-(1H-imidazol-2-yl)-5-oxo-5,8-dihydro-[1,8]naphthyridin-2-yl]-phenyl}-N-(2,5-dimethyl-2H-pyrazol-3-ylmethyl)-acetamide (compound 22);
2-{4-[7-Amino-8-ethyl-6-(1H-imidazol-2-yl)-5-oxo-5,8-dihydro-[1,8]naphthyridin-2-yl]-phenyl}-N-(1-methyl-1H-imidazol-4-ylmethyl)-acetamide (compound 23);
2-{4-[7-Amino-8-ethyl-6-(1H-imidazol-2-yl)-5-oxo-5,8-dihydro-[1,8]naphthyridin-2-yl]-phenyl}-N-pyrazin-2-yl-acetamide (compound 24);
2-{4-[7-Amino-8-ethyl-6-(1H-imidazol-2-yl)-5-oxo-5,8-dihydro-[1,8]naphthyridin-2-yl]-phenyl}-N-(2-pyridin-2-yl-ethyl)-acetamide (compound 25);
2-{4-[7-Amino-8-ethyl-6-(1H-imidazol-2-yl)-5-oxo-5,8-dihydro-[1,8]naphthyridin-2-yl]-phenyl}-N-(5-chloro-thiazol-2-yl)-acetamide (compound 26);
2-{4-[7-Amino-8-ethyl-6-(1H-imidazol-2-yl)-5-oxo-5,8-dihydro-[1,8]naphthyridin-2-yl]-phenyl}-N-(3,4-dimethyl-isoxazol-5-yl)-acetamide (compound 27);
2-{4-[7-Amino-8-ethyl-6-(1H-imidazol-2-yl)-5-oxo-5,8-dihydro-[1,8]naphthyridin-2-yl]-phenyl}-N-(2-methyl-pyridin-4-yl)-acetamide (compound 28);
2-{4-[7-Amino-8-ethyl-6-(1H-imidazol-2-yl)-5-oxo-5,8-dihydro-[1,8]naphthyridin-2-yl]-phenyl}-N-pyrazin-2-ylmethyl-acetamide (compound 29);
2-{4-[7-Amino-8-ethyl-6-(1H-imidazol-2-yl)-5-oxo-5,8-dihydro-[1,8]naphthyridin-2-yl]-phenyl}-N-(5-ethyl-[1,3,4]thiadiazol-2-yl)-acetamide (compound 30);
2-{4-[7-Amino-8-ethyl-6-(1H-imidazol-2-yl)-5-oxo-5,8-dihydro-[1,8]naphthyridin-2-yl]-phenyl}-N-(tetrahydro-furan-2-ylmethyl)-acetamide (compound 31);
2-{4-[7-Amino-8-ethyl-6-(1H-imidazol-2-yl)-5-oxo-5,8-dihydro-[1,8]naphthyridin-2-yl]-phenyl}-N-(3-methyl-pyridin-2-yl)-acetamide (compound 32);
2-{4-[7-Amino-8-ethyl-6-(1H-imidazol-2-yl)-5-oxo-5,8-dihydro-[1,8]naphthyridin-2-yl]-phenyl}-N-(4-methyl-pyridin-2-yl)-acetamide (compound 33);
2-{4-[7-Amino-8-ethyl-6-(1H-imidazol-2-yl)-5-oxo-5,8-dihydro-[1,8]naphthyridin-2-yl]-phenyl}-N-(4,6-dimethyl-pyridin-2-yl)-acetamide (compound 34);
2-{4-[7-Amino-8-ethyl-6-(1H-imidazol-2-yl)-5-oxo-5,8-dihydro-[1,8]naphthyridin-2-yl]-phenyl}-N-(6-methyl-pyridin-2-yl)-acetamide (compound 35);
2-{4-[7-Amino-8-ethyl-6-(1H-imidazol-2-yl)-5-oxo-5,8-dihydro-[1,8]naphthyridin-2-yl]-phenyl}-N-pyridin-3-ylmethyl-acetamide (compound 36);
2-{4-[7-Amino-8-ethyl-6-(1H-imidazol-2-yl)-5-oxo-5,8-dihydro-[1,8]naphthyridin-2-yl]-phenyl}-N-(2-ethyl-2H-pyrazol-3-yl)-acetamide (compound 37);
2-{4-[7-Amino-8-ethyl-6-(1H-imidazol-2-yl)-5-oxo-5,8-dihydro-[1,8]naphthyridin-2-yl]-phenyl}-N-(3-methyl-isothiazol-5-yl)-acetamide (compound 38);
2-Amino-1-ethyl-3-(1H-imidazol-2-yl)-7-{4-[2-oxo-2-(2-pyridin-3-yl-pyrrolidin-1-yl)-ethyl]-phenyl}-1H-[1,8]naphthyridin-4-one (compound 39);
2-{4-[7-Amino-8-ethyl-6-(1H-imidazol-2-yl)-5-oxo-5,8-dihydro-[1,8]naphthyridin-2-yl]-phenyl}-N-(1-benzyl-pyrrolidin-3-yl)-acetamide (compound 40);
2-{4-[7-Amino-8-ethyl-6-(1H-imidazol-2-yl)-5-oxo-5,8-dihydro-[1,8]naphthyridin-2-yl]-phenyl}-N-[(S)-1-(tetrahydro-furan-2-yl)methyl]-acetamide (compound 41);
2-{4-[7-Amino-8-ethyl-6-(1H-imidazol-2-yl)-5-oxo-5,8-dihydro-[1,8]naphthyridin-2-yl]-phenyl}-N-(4-methyl-pyridin-3-yl)-acetamide (compound 42);
2-{4-[7-Amino-8-ethyl-6-(1H-imidazol-2-yl)-5-oxo-5,8-dihydro-[1,8]naphthyridin-2-yl]-phenyl}-N-(4-ethyl-pyridin-2-yl)-acetamide (compound 43);
2-{4-[7-Amino-8-ethyl-6-(1H-imidazol-2-yl)-5-oxo-5,8-dihydro-[1,8]naphthyridin-2-yl]-phenyl}-N-ethyl-N-pyridin-4-ylmethyl-acetamide (compound 44);
2-{4-[7-Amino-8-ethyl-6-(1H-imidazol-2-yl)-5-oxo-5,8-dihydro-[1,8]naphthyridin-2-yl]-phenyl}-N-(6-ethyl-pyridin-2-yl)-acetamide (compound 45);
2-{4-[7-Amino-8-ethyl-6-(1H-imidazol-2-yl)-5-oxo-5,8-dihydro-[1,8]naphthyridin-2-yl]-phenyl}-N-benzyl-acetamide (compound 46);
2-{4-[7-Amino-8-ethyl-6-(1H-imidazol-2-yl)-5-oxo-5,8-dihydro-[1,8]naphthyridin-2-yl]-phenyl}-N-pyridin-4-ylmethyl-propionamide (compound 47);
4-[7-Amino-8-ethyl-6-(1H-imidazol-2-yl)-5-oxo-5,8-dihydro-[1,8]naphthyridin-2-yl]-N-pyridin-4-ylmethyl-benzamide (compound 48);
4-{4-[7-Amino-8-ethyl-6-(1H-imidazol-2-yl)-5-oxo-5,8-dihydro-[1,8]naphthyridin-2-yl]-phenyl}-N-pyridin-4-ylmethyl-butyramide (compound 49);
2-{4-[7-Amino-8-ethyl-6-(4-methyl-1H-imidazol-2-yl)-5-oxo-5,8-dihydro-[1,8]naphthyridin-2-yl]-phenyl}-N-pyridin-4-ylmethyl-acetamide (compound 50);
2-{4-[7-Amino-8-cyclopropylmethyl-6-(1H-imidazol-2-yl)-5-oxo-5,8-dihydro-[1,8]naphthyridin-2-yl]-phenyl}-N-pyridin-4-ylmethyl-acetamide (compound 51);
2-{4-[7-Amino-8-cyclopentyl-6-(1H-imidazol-2-yl)-5-oxo-5,8-dihydro-[1,8]naphthyridin-2-yl]-phenyl}-N-pyridin-4-ylmethyl-acetamide (compound 52);
2-{5-[7-Amino-8-ethyl-6-(1H-imidazol-2-yl)-5-oxo-5,8-dihydro-[1,8]naphthyridin-2-yl]-pyridin-2-yl}-N-pyridin-4-ylmethyl-acetamide (compound 53);
2-{4-[7-Amino-6-(1H-imidazol-2-yl)-8-(3-methoxy-propyl)-5-oxo-5,8-dihydro-[1,8]naphthyridin-2-yl]-phenyl}-N-pyridin-4-ylmethyl-acetamide (compound 54);
2-{4-[7-Amino-8-ethyl-6-(1H-imidazol-2-yl)-5-oxo-5,8-dihydro-[1,8]naphthyridin-2-yl]-phenyl}-N-(3-phenyl-propyl)-acetamide (compound 55);
2-{4-[7-Amino-8-ethyl-6-(1H-imidazol-2-yl)-5-oxo-5,8-dihydro-[1,8]naphthyridin-2-yl]-2-fluoro-phenyl}-N-(1-ethyl-pyrrolidin-2-ylmethyl)-acetamide(compound 56).

13. Process for preparing a compound of formula (I) according to any one of Claims 1 to 12, **characterized in that** (i) a compound of the following formula (VII): 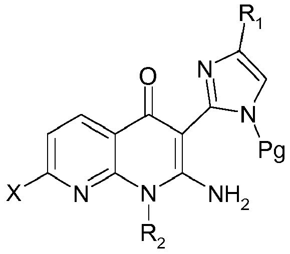 in which Pg represents a protecting group and X represents a halogen atom,
is reacted according to a Suzuki coupling reaction with (ii) a boronic acid or a boronic ester of pinacol of formula (IXa): 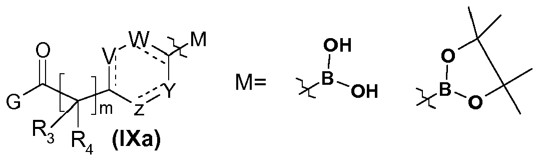 in which G represents a group -NR5R6 or G represents a group -OEt;
in order to obtain respectively (iii) a compound of the following formula (X): 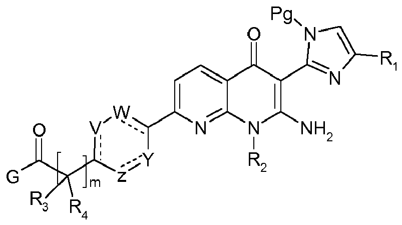 in which G represents a group -NR5R6 and Pg is as defined previously;
or (iv) a compound of the following formula (XI): 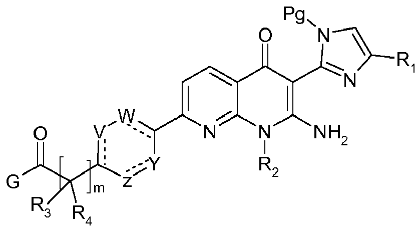 in which G represents a group -OEt and Pg is as defined previously;
it being understood that m, R1, R2, R3, R4, R5, R6, V, W, Y and Z are as defined in Claim 1.

14. Process for preparing a compound of formula (I) according to any one of Claims 1 to 12, **characterized in that** (i) a compound of formula (VIII): 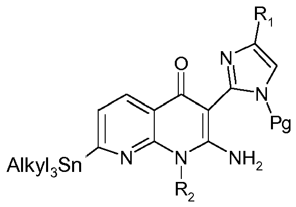 in which Pg represents a protecting group;
is reacted according to a Stille coupling reaction with (ii) an aryl or heteroaryl halide of the following formula (IXb): 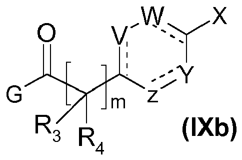 in which G represents a group -OEt or G represents a group -NR5R6 and X represents a halogen atom;
in order to obtain (iii) a compound of the following formula (X): 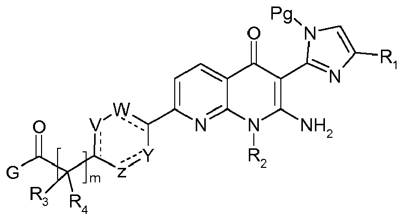 for which G represents a group -NR5R6 and Pg is as defined previously;
or (iv) a compound (XI) of the following formula: 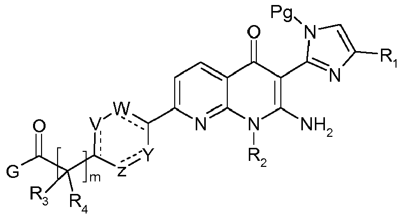 for which G represents a group -OEt,
it being understood that m, R1, R2, R3, R4, R5, R6, V, W, Y and Z are as defined in Claim 1.

15. Process for preparing a compound of formula (I) according to one of Claims 13 or 14, **characterized in that** the compound (XI) for which G represents a group - OEt then undergoes a stage of saponification and then a stage of peptide coupling with the amine HNR5R6, giving the compound (XIII) of the following formula: 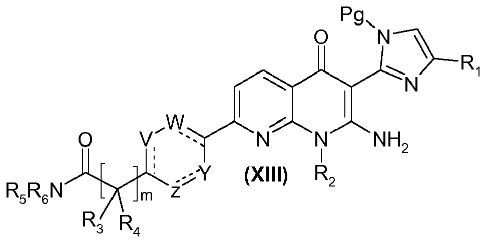 it being understood that Pg represents a protecting group and that m, R1, R2, R3, R4, R5, R6, V, W, Y and Z are as defined in Claim 1.

16. Medicinal product, **characterized in that** it comprises a compound of formula (I) according to any one of Claims 1 to 12, or a salt of addition of this compound to a pharmaceutically acceptable acid, or a solvate of the compound of formula (I).

17. Pharmaceutical composition, **characterized in that** it comprises a compound of formula (I) according to any one of Claims 1 to 12, or a pharmaceutically acceptable salt, a solvate of this compound, as well as at least one pharmaceutically acceptable excipient.

18. Use of a compound of formula (I) according to any one of Claims 1 to 12 for preparing a medicinal product intended for treating diseases linked to the activity of protein kinases, said compound of formula (I) inhibiting the tyrosine kinase activity of PDGF-R, in particular PDGF-R beta, in Baf3 tel/PDGF cells and/or inhibiting the tyrosine kinase activity of Flt-3 in MV4-11 cells.

19. Use of a compound of formula (I) according to any one of Claims 1 to 12 for preparing a medicinal product intended for the treatment of proliferative diseases such as cancers with liquid tumours, chronic or acute leukaemias, lymphocytic lymphomas, Hodgkin's disease, myeloproliferative syndromes and myelodysplastic syndromes.

20. Use of a compound of formula (I) according to any one of Claims 1 to 12 for preparing a medicinal product intended for the treatment of proliferative diseases such as cancers with solid tumours comprising lung cancers (NSCLC), cancers of bone, pancreas, skin, Kaposi syndrome, intraocular melanomas, cancers associated with sexual organs comprising cancer of the breast, uterus, cervix, ovary, endometrium, vagina, vulva, urethra, penis, prostate, carcinomas of the fallopian tubes, cancers of the gastrointestinal stromal tumour type (abbreviation: GIST) comprising cancers of the anal region, rectum, small intestine, colon, stomach, oesophagus, cancers of endocrine glands, thyroid, parathyroid or adrenal glands, soft tissue sarcomas, Ewing sarcomas, osteosarcomas, dermatofibrosarcomas and other fibrosarcomas, cancers of the bladder or kidney, neoplasms of the central nervous system, tumours of the vertebral column and desmoids, gliomas of the brainstem and glioblastomas, pituitary adenomas and metastases thereof.

21. Use of a compound of formula (I) according to any one of Claims 1 to 12 for preparing a medicinal product intended for the treatment of non-malignant proliferative diseases such as restenosis, atherosclerosis, thrombosis, heart failure, cardiac hypertrophy, pulmonary arterial hypertension, fibrosis, diabetic nephropathy, glomerulonephritis, chronic pyelonephritis, haemangiomas, auto-immune diseases such as psoriasis, sclerodermatitis, immunosuppression, pathologies of the eye such as post-operative fibrosis and age-related macular degeneration.

22. Compound of formula (I) according to any one of Claims 1 to 12 for preparing a medicinal product intended for the treatment of proliferative diseases such as cancers with liquid tumours, chronic or acute leukaemias, lymphocytic lymphomas, Hodgkin's disease, myeloproliferative syndromes and myelodysplastic syndromes.

23. Compound of formula (I) according to any one of Claims 1 to 12 for preparing a medicinal product intended for the treatment of proliferative diseases such as cancers with solid tumours comprising lung cancers (NSCLC), cancers of bone, pancreas, skin, Kaposi syndrome, intraocular melanomas, cancers associated with sexual organs comprising cancer of the breast, uterus, cervix, ovary, endometrium, vagina, vulva, urethra, penis, prostate, carcinomas of the fallopian tubes, cancers of the gastrointestinal stromal tumour type (abbreviation: GIST) comprising cancers of the anal region, rectum, small intestine, colon, stomach, oesophagus, cancers of endocrine glands, thyroid, parathyroid or adrenal glands, soft tissue sarcomas, Ewing sarcomas, osteosarcomas, dermatofibrosarcomas and other fibrosarcomas, cancers of the bladder or kidney, neoplasms of the central nervous system, tumours of the vertebral column and desmoids, gliomas of the brainstem and glioblastomas, pituitary adenomas and metastases thereof.

24. Compound of formula (I) according to any one of Claims 1 to 12 for preparing a medicinal product intended for the treatment of non-malignant proliferative diseases such as restenosis, atherosclerosis, thrombosis, heart failure, cardiac hypertrophy, pulmonary arterial hypertension, fibrosis, diabetic nephropathy, glomerulonephritis, chronic pyelonephritis, haemangiomas, auto-immune diseases such as psoriasis, sclerodermatitis, immunosuppression, pathologies associated with the eye such as post-operative fibrosis or age-related macular degeneration.

25. Combination of at least one compound of formula (I) according to any one of Claims 1 to 12 with at least one chemotherapy agent.
